# EUROPEAN PATENT APPLICATION

(11) **EP 4 473 980 A2**
(43) Date of publication of application: **11.12.2024**
(21) Application number: 24206722.1
(22) Date of filing: 27.09.2019
(51) Int. Cl.: A61K 51/04

(54) **LABELED INHIBITORS OF PROSTATE SPECIFIC MEMBRANE ANTIGEN (PSMA), THEIR USE AS IMAGING AGENTS AND PHARMACEUTICAL AGENTS FOR THE TREATMENT OF PSMA-EXPRESSING CANCERS**

(30) Priority: 28.09.2018 EP 18197704
(62) Divisional of application: 19773468.4
(71) Applicant: Universität Heidelberg, 69117 Heidelberg (DE); Deutsches Krebsforschungszentrum, 69120 Heidelberg (DE)
(72) Inventor: Haberkorn, Uwe, 68723 Schwetzingen (DE); Dos Santos, José Carlos, 69118 Heidelberg (DE); Mier, Walter, 64625 Bensheim (DE); Kratochwil, Clemens, 69493 Hirschberg (DE); Bauder-Wuest, Ulrike, 69198 Schriesheim (DE); Kopka, Klaus, 69221 Dossenheim (DE); Schäfer, Martin, 69239 Neckarsteinach (DE)
(74) Representative: Altmann Stößel Dick Patentanwälte PartG mbB

(57) **Abstract**

The present invention relates to a compound of formula (1) 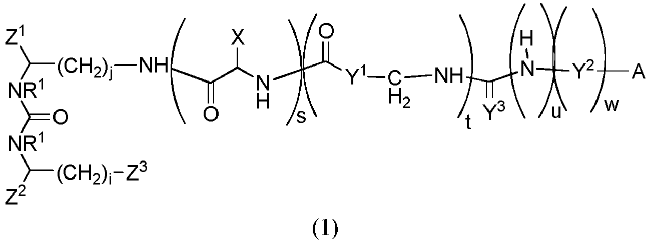 or a pharmaceutically acceptable salt or solvate thereof, wherein Y³ is O or S, wherein s, t, u and w are, independently of each other, 0 or 1, wherein i is an integer of from 1 to 3, wherein j is an integer of from 3 to 5, and wherein Z¹, Z² and Z³ are independently of each other, selected from the group consisting of CO₂H, -SO₂H, -SO₃H, -OSO₃H, and -OPO₃H_{2,,} R¹ is -CH₃ or H, preferably H, X is selected from the group consisting of alkylaryl, aryl, alkylheteroaryl and heteroaryl, Y¹ and Y² are independently, of each other, selected from the group consisting of aryl, alkylaryl, cycloalkyl, heterocycloalkyl, heteroaryl and alkylheteroaryl, and wherein A is a chelator residue having a structure selected from the group consisting of (Ia), (Ib) and (Ic) 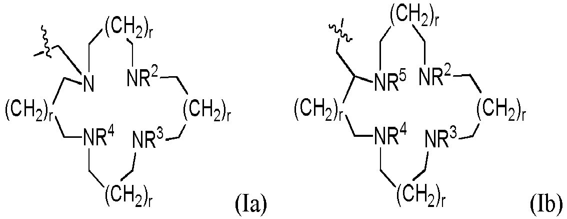 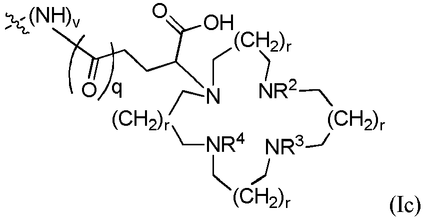 wherein R², R³, R⁴ and R⁵ are, independently of each other, selected from the group consisting of H, - CH₂-COOH and -CH₂-C(=O)-NH₂ or wherein R² and R⁴ form a -(CH₂)ₙ- bridge with n being an integer of from 1 to 3, wherein n is preferably 2, and wherein r, v and q, are independently of each other, 0 or 1, with the proviso that in case u and w are 0, q and v are 0, and (A) wherein u and w are 1, or (B) wherein u is 0 and w is 1, and wherein A is selected from (Ia) or (Ib), or (C) wherein A is not

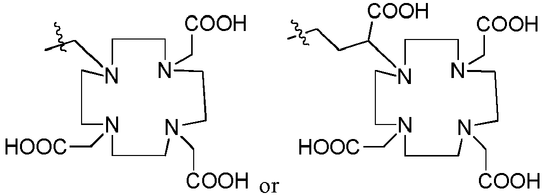

Further, the present invention relates to a complex comprising
(a) a radionuclide, and
(b) the compound, as described above or below, or a salt, solvate, metabolite or prodrug thereof.

Further, the present invention relates to a pharmaceutical composition comprising a compound, as described above or below, or a complex, as described above or below. Further, the present invention relates to a compound, as described above or below, or a complex, as described above or below, or a pharmaceutical composition, as described above or below, for use in treating, ameliorating or preventing PSMA-expressing cancer and/or metastases thereof, in particular prostate cancer and/or metastases thereof. Further, the present invention relates to a compound, as described above or below, or a complex, as described above or below, or a pharmaceutical composition, as described above or below, for use in diagnostics. In addition, the present invention relates to a compound, as described above or below, or a complex, as described above or below, or a pharmaceutical composition, as described above or below, for use in the diagnosis of cancer, in particular of PSMA-expressing cancer and/or metastases thereof, in particular prostate cancer and/or metastases thereof.

## Description

### Field of the invention

The present invention generally relates to the field of radiopharmaceuticals and their use in nuclear medicine as tracers, imaging agents and for the treatment of various disease states of PSMA-expressing cancers, especially prostate cancer, and metastases thereof.

### Related art

Prostate cancer (PCa) is the leading cancer in the US and European population. At least 1-2 million men in the western hemisphere suffer from prostate cancer and it is estimated that the disease will strike one in six men between the ages of 55 and 85. There are more than 300,000 new cases of prostate cancer diagnosed each year in USA. The mortality from the disease is second only to lung cancer. Currently, imaging methods with high resolution of the anatomy, such as computed tomography (CT), magnetic resonance (MR) imaging and ultrasound, predominate for clinical imaging of prostate cancer. An estimated annual $ 2 billion is currently spent worldwide on surgical, radiation, drug therapy and minimally invasive treatments. However, there is presently no effective therapy for relapsing, metastatic, androgen-independent prostate cancer.

A variety of experimental low molecular weight PCa imaging agents are currently being pursued clinically, including radiolabeled choline analogs [¹⁸F]fluorodihydrotestosterone ([¹⁸F]FDHT), anti-1-amino-3-[¹⁸F]fluorocycIobutyl-1-carboxylic acid (anti[¹⁸F]F-FACBC, [¹¹C]acetate and 1-(2-deoxy-2-[¹⁸F]fluoro-E-arabinofuranosyl)-5-methyluracil (-[¹⁸F]FMAU)(Scher, B.; et al. Eur J Nucl Med Mol Imaging 2007, 34, 45-53; Rinnab, L; et al. BJU Int 2007, 100, 786,793; Reske, S.N.; et al. J Nucl Med 2006, 47, 1249-1254; Zophel, K.; Kotzerke, J. Eur J Nucl Med Mol Imaging 2004, 31 , 756-759; Vees, H.; et al. BJU Int 2007, 99, 1415-1420; Larson, S. M.; et al. J Nucl Med 2004, 45, 366-373; Schuster, D.M.; et al. J Nucl Med 2007, 48, 56-63; Tehrani, O.S.; et al. J Nucl Med 2007, 48, 1436-1441). Each operates by a different mechanism and has certain advantages, e.g., low urinary excretion for [¹¹C]choline, and disadvantages, such as the short physical half-life of positron-emitting radionuclides.

It is well known that tumors may express unique proteins associated with their malignant phenotype or may over-express normal constituent proteins in greater number than normal cells. The expression of distinct proteins on the surface of tumor cells offers the opportunity to diagnose and characterize disease by probing the phenotypic identity and biochemical composition and activity of the tumor. Radioactive molecules that selectively bind to specific tumor cell surface proteins provide an attractive route for imaging and treating tumors under non-invasive conditions. A promising new series of low molecular weight imaging agents targets the prostate-specific membrane antigen (PSMA) (Mease R.C. et al. Clin Cancer Res. 2008, 14, 3036-3043; Foss, C.A.; et al. Clin Cancer Res 2005, 11 , 4022-4028; Pomper, M.G.; et al. Mol Imaging 2002, 1 , 96-101 ; Zhou, J.; et al. Nat Rev Drug Discov 2005, 4, 015-1026; WO 2013/022797).

PSMA is a trans-membrane, 750 amino acid type II glycoprotein that has abundant and restricted expression on the surface of PCa, particularly in androgen-independent, advanced and metastatic disease (Schulke, N.; et al. Proc Natl Acad Sci U S A 2003, 100, 12590-12595). The latter is important since almost all PCa become androgen independent over the time. PSMA possesses the criteria of a promising target for therapy (Schulke, N.; et al. Proc. Natl. Acad. Sci. USA 2003, 100, 12590-12595). The PSMA gene is located on the short arm of chromosome 11 and functions both as a folate hydrolase and neuropeptidase. It has neuropeptidase function that is equivalent to glutamate carboxypeptidase II (GCPII), which is referred to as the "brain PSMA", and may modulate glutamatergic transmission by cleaving /V-acetylaspartylglutamate (NAAG) to N-acetylaspartate (NAA) and glutamate (Nan, F.; et al. J Med Chem 2000, 43, 772-774). There are up to 10⁶ PSMA molecules per cancer cell, further suggesting it as an ideal target for imaging and therapy with radionuclide-based techniques (Tasch, J.; et al. Crit Rev Immunol 2001 , 21 , 249-261).

The radio-immunoconjugate of the anti-PSMA monoclonal antibody (mAb) 7E11, known as the PROSTASCINT^{®} scan, is currently being used to diagnose prostate cancer metastasis and recurrence. However, this agent tends to produce images that are challenging to interpret (Lange, P.H. PROSTASCINT scan for staging prostate cancer. Urology 2001 , 57, 402-406; Haseman, M.K.; et al. Cancer Biother Radiopharm 2000, 15, 131-140; Rosenthal, S.A.; et al. Tech Urol 2001 , 7, 27-37). More recently, monoclonal antibodies have been developed that bind to the extracellular domain of PSMA and have been radiolabeled and shown to accumulate in PSMA-positive prostate tumor models in animals. However, diagnosis and tumor detection using monoclonal antibodies has been limited by the low permeability of the monoclonal antibody in solid tumors.

The selective targeting of cancer cells with radiopharmaceuticals, either for imaging or therapeutic purposes is challenging. A variety of radionuclides are known to be useful for radio-imaging or cancer radiotherapy, including ¹¹¹In, ⁹⁰Y, ⁶⁸Ga, ¹⁷⁷Lu, ^{99m}Tc, ¹²³I and ¹³¹I. Recently it has been shown that some compounds containing a glutamate-urea-glutamate (GUG) or a glutamate-urea-lysine (GUL) recognition element linked to a radionuclide-ligand conjugate exhibit high affinity for PSMA. In WO 2015/055318 new imaging agents with improved tumor targeting properties and pharmacokinetics were described. These compounds comprise a motif specifically binding to cell membranes of cancerous cells, wherein said motif comprises a prostate-specific membrane antigen (PSMA), that is the above mentioned glutamate-urea-lysine motif. The preferred molecules described in WO 2015/055318 further comprise a linker which binds via an amide bond to a carboxylic acid group of DOTA as chelator. Some of these compounds have been shown to be promising agents for the specific targeting of prostate tumors. The compounds were labeled with ¹⁷⁷Lu (for therapy purposes) or ⁶⁸Ga (for diagnostic purposes) and allow for visualization and targeting of prostate cancer for radiotherapy purposes.

However, there is still the need for alternative or improved ligands that interact with PSMA and carry appropriate radionuclides, such as ¹⁷⁷Lu or ⁶⁸Ga, for the detection, treatment and management of PSMA-expressing cancers, in particular prostate cancer.

Further, it is to be noted that none of the compounds described in WO 2015/055318 are described to form stable complexes with ⁶⁴Cu and ⁶⁷Cu. However, the pair ⁶⁴Cu/⁶⁷Cu has favorable properties, ⁶⁴Cu is ideally suited for long term PET imaging and it allows to determine the dosimetry of ⁶⁷Cu labeled radiotherapeutics. The therapeutic nuclide ⁶⁷Cu is cyclotron produced and suited to GMP (good manufacturing practices) production. Its half-life allows for optimized repeated dosing, only a few days of hospitalization and reduced costs of waste management. Due to its characteristics t_{1/2} = 12.7 h, β⁺ 17.4%, Eₘₐₓ = 0.656 MeV, β⁻ 39%, Eₘₐₓ = 0.573 MeV), ⁶⁴Cu is available in high quantities in comparison to ⁶⁸Ga (t_{½} = 67.71 min, 88.9% β⁺) (Wadas TJ, Wong EH, Weisman GR, Anderson CJ. Copper chelation chemistry and its role in copper radiopharmaceuticals. Curr Pharm Des. 2007;13:3-16.). The adequate half-life of ⁶⁴Cu (12.7 h) in contrast to ⁶⁸Ga (67.71 min) favours imaging at later time points and subsequent with increased tumor delineation (Lewis MR, Wang M, Axworthy DB, et al. In vivo evaluation of pretargeted 64Cu for tumor imaging and therapy. J Nucl Med. 2003;44:1284-1292. 21. De Silva RA, Jain S, Lears KA, et al. Copper-64 radiolabeling and biological evaluation of bifunctional chelators for radiopharmaceutical development. Nucl Med Biol. 2012;39:1099-1104.). A further major advantage of this radioisotope is that it may be used for diagnostics as well as for therapy. Thus, there is the need for PSMA ligands labeled with copper radionuclides.

Further, it is to be noted that in bone metastatic prostate cancer a survival benefit was observed after alpha-radiation therapy with the bone-seeker ²²³RaCl₂, which could not be demonstrated for its beta-emitting analogue ⁸⁹SrCl₂ (Rubini G, Nicoletti A, Rubini D, Asabella AN. Radiometabolic treatment of bone-metastasizing cancer: from 186Re to 223Ra. Cancer Biother Radiopharm. 2014; 29:1-11]). It already had been demonstrated for patients with neuroendocrine tumors, that alpha-emitting ²¹³Bi-DOTATOC can overcome resistance against beta-emitting ⁹⁰Y/¹⁷⁷Lu-DOTATOC [Kratochwil C, Giesel FL, Bruchertseifer F, et al. 213Bi-DOTATOC receptor-targeted alpha-radionuclide therapy induces remission in neuroendocrine tumors refractory to beta radiation: a first-in-human experience. Eur J Nucl Med Mol Imaging. 2014 Nov; 41(11):2106-19]. Consecutively, the interest in alpha-emitter based radionuclide therapy increases. Despite being interesting for research purposes, the short physical half-lives of ²¹³Bi (tvz= 0.8 h), ²¹²Bi (t_{1/2} = 1.0 h), ¹⁴⁹Tb (t_{1/2}=4.1 h) and ²¹¹At (t_{1/2}= 7.2 h) are challenging for a possible clinical application. On the other hand, long half-life alpha-emitters such as ²²⁷Th (t_{1/2}= 18.7 d), which might be necessary to cope with the slow pharmacokinetics of full-length antibodies, may accumulate in the environment and cause issues regarding waste disposal if applied in large scale, e.g. for treatment of epidemiologic important tumors. Intense radiation and considerably shorter path length are the main properties of alpha emitters. However, there are only few alpha emitters with an appropriate half-life suitable for clinical routine such as ²¹²Pb.

Thus, there is still the need for PSMA ligands which form suitable complexes with alpha-emitting radionuclides.

Thus, the overall object of the present invention is to develop improved ligands that interact with PSMA and carry appropriate radionuclides which provide advantageous options for the detection, treatment and management of PSMA-expressing cancers, in particular prostate cancer.

### Summary of the invention

The solution of said object is achieved by providing the embodiments characterized in the claims. The inventors found new compounds which are useful and advantageous radiopharmaceuticals and can be used in nuclear medicine as tracers, imaging agents and for the treatment of various disease states of PSMA-expressing cancers, in particular prostate cancer. These compounds are described in more detail below:

In particular, the present invention relates to a compound of formula (1) 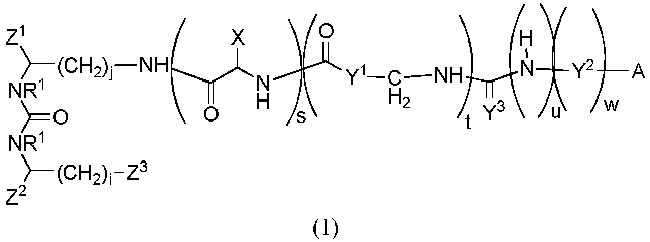 or a pharmaceutically acceptable salt or solvate thereof, wherein Y³ is O or S, wherein s, t, u and w are, independently of each other, 0 or 1, wherein i is an integer of from 1 to 3, wherein j is an integer of from 3 to 5, and wherein Z¹, Z² and Z³ are independently of each other, selected from the group consisting of -CO₂H, -SO₂H, -SO₃H, -OSO₃H, and -OPO₃H₂, R¹ is -CH₃ or H, preferably H, X is selected from the group consisting of, optionally substituted, alkylaryl, aryl, alkylheteroaryl and heteroaryl, Y¹ and Y² are, independently of each other, selected from the group consisting of, optionally substituted, aryl, alkylaryl, cycloalkyl, heterocycloalkyl, heteroaryl and alkylheteroaryl, and wherein A is a chelator residue having a structure selected from the group consisting of (Ia), (Ib) and (Ic) 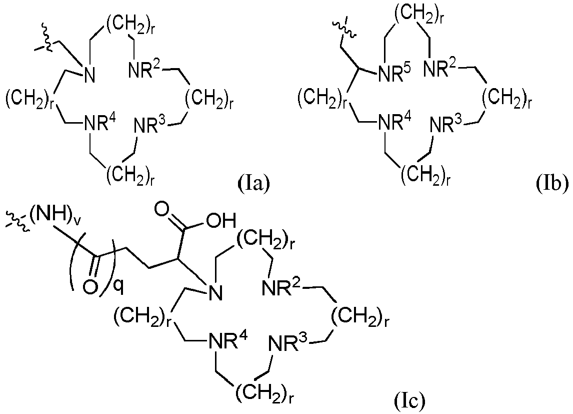 wherein R², R³, R⁴ and R⁵ are, independently of each other, selected from the group consisting of H, -CH₂-COOH and -CH₂-C(=O)-NH₂ or wherein R² and R⁴ form a -(CH₂)ₙ- bridge with n being an integer of from 1 to 3, wherein n is preferably 2, and wherein r, v and q, are independently of each other, 0 or 1, with the proviso that in case u and w are 0, q and v are 0, and wherein
(A) u and ware 1, or
(B) u is 0 and w is 1, and wherein A is selected from (Ia) or (Ib), or
(C) A is not

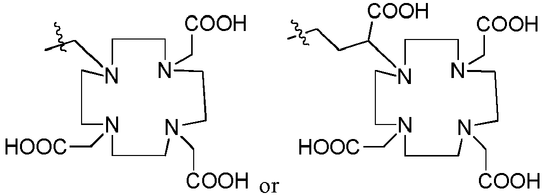

Further, the present invention relates to a complex comprising
(a) a radionuclide, and
(b) the compound, as described above or below, or a salt, solvate, metabolite or prodrug thereof.

Further, the present invention relates to a pharmaceutical composition comprising a compound, as described above or below, or a complex, as described above or below. Further, the present invention relates to a compound, as described above or below, or a complex, as described above or below, or a pharmaceutical composition, as described above or below, for use in treating, ameliorating or preventing PSMA expressing cancers and/or metastases thereof, especially prostate cancer and/or metastases thereof. Further, the present invention relates to a compound, as described above or below, or a complex, as described above or below, or a pharmaceutical composition, as described above or below, for use in diagnostics. In addition, the present invention relates to a compound, as described above or below, or a complex, as described above or below, or a pharmaceutical composition, as described above or below, for use in the diagnosis of cancer, in particular of PSMA expressing tumors and/or metastases thereof.

The term "PSMA expressing cancers and/or metastases thereof as used within the meaning of the present invention relates to any cancer whose cancerous cells express Prostate Specific Membrane Antigen (PSMA) and the respective metastases thereof. Preferably, cancers (or cancer cells) that may be treated according to the invention are selected among prostate cancer, conventional renal cell cancers, cancers of the transitional cells of the bladder, testicular-embryonal cancers, neuroendocrine cancers, colon cancers, brain tumors and breast cancers. In particularly preferred aspects of the invention, said PSMA expressing cancer is prostate cancer or breast cancer, in particular prostate cancer.

As described above, the compound or the compound comprised in the complex of the present invention has the following structure:

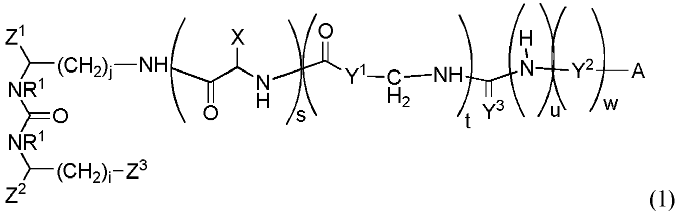

It has to be understood the compound or the compound comprised in the complex may be in the form of an anion, or a salt of the compound of formula (1).

The invention thus also relates to salt, in particular a pharmaceutically acceptable salt, of the compound of general formula (1) or the compound comprised in the complex. The invention also relates to solvates of these compounds, including the salts as well as the active metabolites thereof and, where appropriate, the tautomers thereof including prodrug formulations.

A "pharmaceutically acceptable salt" is a pharmaceutically acceptable, organic or inorganic acid or base salt of a compound of the invention. Representative pharmaceutically acceptable salts include, e.g., alkali metal salts, alkali earth metal salts, ammonium salts, water-soluble and water-insoluble salts, such as the acetate, carbonate, chloride, gluconate, glutamate, lactate, laurate, malate or tartrate. The term "prodrug" refers to a precursor of a drug that is a compound which upon administration to a patient must undergo chemical conversion by metabolic processes before becoming an active pharmacological agent. Illustrative prodrugs of compounds in accordance with formula (1) are esters and amides, preferably alkyl esters of fatty acid esters. Prodrug formulations here comprise all substances which are formed by simple transformation including hydrolysis, oxidation or reduction either enzymatically, metabolically or in any other way. A suitable prodrug contains e.g. a substance of general formula (1) bound via an enzymatically cleavable linker (e.g. carbamate, phosphate, N-glycoside or a disulfide group) to a dissolution-improving substance (e.g. tetraethylene glycol, saccharides, formic acids or glucuronic acid, etc.). Such a prodrug of a compound according to the invention can be applied to a patient, and this prodrug can be transformed into a substance of general formula (1) so as to obtain the desired pharmacological effect.

Some compounds of formula (1) may be encompassed in form of stereoisomeric mixtures, such as racemic mixtures and/or mixtures of cis/trans isomers, or as single enantiomers, diastereomers and/or a specific cis/trans isomer, including all possible mixtures thereof.

According to the invention all chiral C-atoms shall have D- and/or L-configuration; also combinations within one compound shall be possible, i.e. some of the chiral C-atoms may be D- and others may be L-configuration. Most preferably, amino acid residues present in the compound have L-configuration.

The obtained compounds can be optionally separated by known methods (e.g. Allinger, N.L. und Elliel E.L. in "Topics in Stereochemistry" Vol. 6, Wiley Interscience, 1971) in their enantiomers and/or diasteromers. One possible method of enantiomeric separation is the use of chromatography.

### The urea backbone:

Compound (1) comprises an urea building block (1A). In this urea building block (1A) of compound (1) 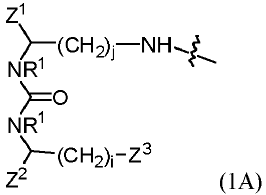 Z¹, Z² and Z³ are , independently of each other, selected from the group consisting of CO₂H, - SO₂H, -SO₃H, -OSO₃H, and -OPO₃H_{2,,} more preferably, at least one of, more preferably all of, Z¹, Z² and Z³ are -CO₂H.

It is to be understood that the building block (1A) may be present in any stereoisomeric form, preferably, however, (1A) has the structure (1Aa):

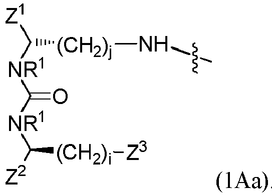

Thus, preferably, the compound of the invention and the compound comprised in the complex of the invention, has the structure

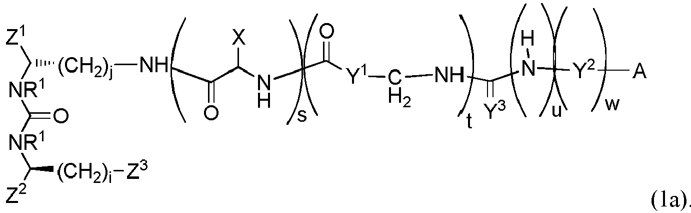

Integer i and integer j are as described above.

Preferably i is 2. Thus, the present invention also relates to a compound of formula (1), preferably (1a), and to the compound comprised in the complex of the invention, wherein i is 2.

Preferably, j is 4. Thus, the present invention also relates to a compound of formula (1), preferably (1a), and to the compound comprised in the complex of the invention, wherein j is 4.

R¹ is preferably H.

The urea building block (1Aa), thus most preferably has the structure (1Aa_1)

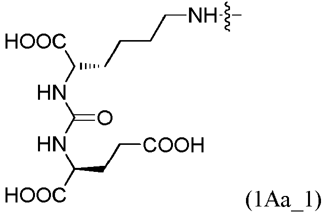

### Residue X and building block (1B):

If s is 1, the compound of formula (1) comprises the building block (1B)

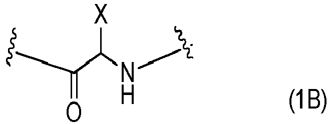

As described, above, in this building block, X preferably comprises a residue selected from the group consisting of naphtyl, phenyl, biphenyl, indolyl and benzothiazolyl. Preferably, X is a naphtyl, alkyl-naphtyl, phenyl, benzyl, biphenyl, alkyl-biphenyl, indolyl, alkyl-indolyl, benzothiazolyl or alkyl-benzothiazolyl group.

Within the meaning of the present invention, the terms naphtyl, phenyl, biphenyl, indolyl and benzothiazolyl, encompass groups which are further substituted by one or more suitable substituents. The term "substituted" as used in this context of the present invention preferably refers to groups being substituted in any position by one or more substituents, preferably by 1, 2, 3, 4, 5 or 6 substituents, more preferably by 1, 2, or 3 substituents. If two or more substituents are present, each substituent may be the same or may be different from the at least one other substituent. Preferably, the groups are unsubstituted.

Within the meaning of the present invention, the term "alkyl" relates to non-branched alkyl residues and branched alkyl residues. The term also encompasses alkyl groups which are further substituted by one or more suitable substituents. The term "substituted alkyl" as used in this context of the present invention preferably refers to alkyl groups being substituted in any position by one or more substituents, preferably by 1, 2, 3, 4, 5 or 6 substituents, more preferably by 1, 2, or 3 substituents.

More preferably, the residue X is selected from the group consisting of 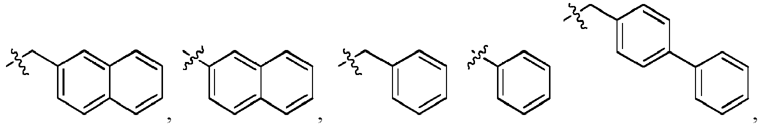 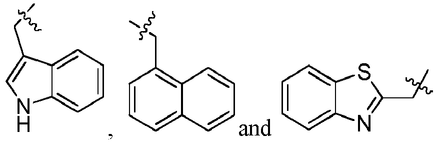 , wherein these groups may be suitably substituted. Perferably, these groups are not substituted.

Most preferably, X, if present, is

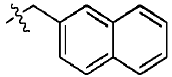

The building block (1B), if present, thus preferably has the following structure:

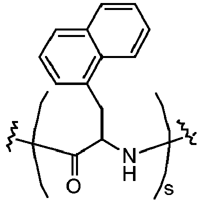

### The group Y¹ and building block (1C):

If t is 1, the compound of formula (1) comprises the building block (1C)

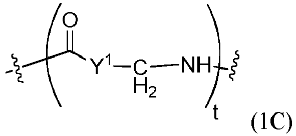

As described, Y' is selected from the group consisting of aryl, alkylaryl, cycloalkyl, heterocycloalkyl, heteroaryl and alkylheteroaryl.

The term "aryl", as used in the context of the present invention, means optionally substituted aryl groups, i.e. in particular optionally substituted, 5- and 6-membered aromatic rings, and substituted or unsubstituted polycyclic aromatic groups (aryl groups), for example tricyclic or bicyclic aryl groups. Optionally substituted phenyl groups or naphthyl groups may be mentioned as examples. Polycyclic aromatic groups can also contain non-aromatic rings.

The term "heteroaryl", as used in the context of the present invention, means means optionally substituted hetroaryl groups, i.e. in particular optionally substituted, 5- and 6-membered aromatic rings, and substituted or unsubstituted polycyclic aromatic groups, for example tricyclic or bicyclic aryl groups, containing one or more, for example 1 to 4, such as 1, 2, 3, or 4, heteroatoms in the ring system. If more than one heteroatom is present in the ring system, the at least two heteroatoms that are present can be identical or different. Suitable heteroaryl groups are known to the skilled person. The following, optionally substituted, heteroaryl residues may be mentioned, as non limiting examples: benzodioxolyl, pyrrolyl, furanyl, thiophenyl, thiazolyl, isothiaozolyl, imidazolyl, triazolyl, tetrazolyl, pyrazolyl, oxazolyl, isoxazolyl, pyridinyl, pyrazinyl, pyridazinyl, benzoxazolyl, benzodioxazolyl, benzothiazolyl, benzoimidazolyl, benzothiophenyl, methylenedioxyphenylyl, napthridinyl, quinolinyl, isoqunilyinyl, indolyl, benzofuranyl, purinyl, benzofuranyl, deazapurinyl, pyridazinyl and indolizinyl.

The term "alkylaryl" or "alkylheteroaryl" as used within the meaning of the present invention, refers to a group wherein the aryl group or heteroaryl is linked via an alkyl group to the respective rest of the building block. In case of X that is e.g. to the C-backbone, thus "alkylaryl" in this case refers to the group -Alkyl-Aryl and "alkylheteroaryl" refers to the group -Alkyl-Heteroalkyl. In case of Y1, ) the aryl group or heteroaryl is linked via an alkyl group to the carbonylgroup, i.e. thus "alkylaryl" in this case refers to the group -Alkyl-Aryl- and "alkylheteroaryl to the group -Alkyl-Heteroalkyl-. In case of Y2, the aryl group or heteroaryl is linked via an alkyl group to the NH group, .e. thus "alkylaryl" in this case refers to the group -Alkyl-Aryl- and "alkylheteroaryl to the group -Alkyl-Heteroalkyl-.

The term "cycloalkyl" means, in the context of the invention, optionally substituted, cyclic alkyl residues, wherein they can be monocyclic or polycyclic groups. Optionally substituted cyclohexyl may be mentioned as a preferred example of a cycloalkyl residue.

The term "heterocycloalkyl", as used in the context of the present invention, means optionally substituted, cyclic alkyl residues, which have at least one heteroatom, such as O, N or S in the ring, wherein they can be monocyclic or polycyclic groups.

The terms "substituted cycloalkyl residue" or "cycloheteroalkyl", as used in the context of the present invention, mean cycloalkyl residues or cycloheteroalkyl residues, in which at least one H has been replaced with a suitable substituent.

Preferably, Y' is a cycloalkyl or heterocycloalkyl group, more preferably a cycloalkyl group, more preferably

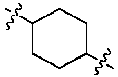

The building block (1C), if present, thus preferably has the following structure;

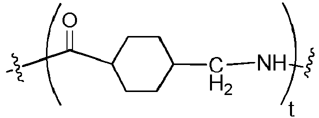

It is to be understood that this structure includes any possible stereoisomers, such as cis/trans isomers. Preferably, the group 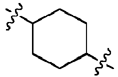 is present as the trans isomer. The building block (1C), if present, thus preferably has the following structure;

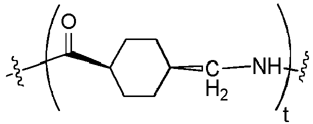

### The group Y³

As described above, Y³ is preferably O or S.

### The radionucleotide

Depending on whether the compounds of the invention are to be used as radio-imaging agents or radio-pharmaceuticals different radionuclides are complexed to the chelator.

Illustrative radionuclides include, for example, ⁸⁹Zr, ⁴⁴Sc, ¹¹¹In, ⁹⁰Y, ⁶⁶Ga, ⁶⁷Ga, ⁶⁸Ga, ¹⁷⁷Lu, ^{99m}Tc, ⁶⁰Cu, ⁶¹Cu, ⁶²Cu, ⁶⁴Cu, ⁶⁶Cu, ⁶⁷Cu, ¹⁴⁹Tb, ¹⁵²Tb, ¹⁵³Sm, ¹⁵⁵Tb, ¹⁶¹Tb, ¹⁵³Gd, ¹⁵⁵Gd, ¹⁵⁷Gd, ²¹³Bi, ²²⁵Ac, ²³⁰U, ²²³Ra, ¹⁶⁵Er, radionuclides of Fe (such as ⁵²Fe and ⁵⁹Fe) and radionuclides of Pb (such as ²⁰³Pb and ²¹²Pb, ²¹¹ Pb, ²¹³ Pb, ²¹⁴Pb, ²⁰⁹Pb, ¹⁹⁸Pb, ¹⁹⁷Pb).

Preferably, the radionuclide is selected from the group consisting of ¹¹¹In, ⁹⁰Y, ⁶⁸Ga, ¹⁷⁷Lu, ¹⁵³Gd, ¹⁵⁵Gd, ²¹³Bi, ²²⁵Ac, ⁶⁰Cu, ⁶¹Cu, ⁶²Cu, ⁶⁴Cu, ⁶⁶Cu, ⁶⁷Cu, radionuclides of Fe (such as ⁵²Fe and ⁵⁹Fe) and radionuclides of Pb (such as ²⁰³Pb and ²¹²Pb, ²¹¹ Pb, ²¹³ Pb, ²¹⁴Pb, ²⁰⁹Pb, ¹⁹⁸Pb, ¹⁹⁷Pb).

The radionuclides of Pb are more preferably ²⁰³Pb and ²¹²Pb.

The radionuclides of Cu are more preferably ⁶⁴Cu and ⁶⁷Cu.

The complexes of the compounds according of the invention may contain one or more radionuclides, preferably one radionuclide. These radionuclides are preferably suitable for use as radio-imaging agents or as therapeutics for the treatment of proliferating cells, for example, PSMA expressing cancer cells, in particular PSMA-expressing prostate cancer cells. According to the present invention they are called "metal complexes" or "radiopharmaceuticals".

Preferred imaging methods are positron emission tomography (PET) or single photon emission computed tomography (SPECT).

### Embodiment (A)

According to a preferred embodiment of the invention, u and w are 1. According to this embodiment, the compound of the invention thus has the following structure.

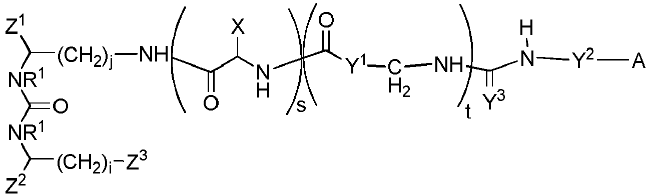

In this case, Y³ is most preferably S. Thus, the compound of the invention has more preferably the following structure.

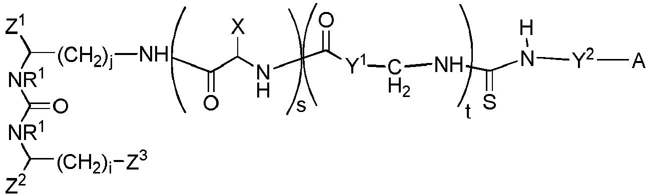

As described above, Y² is selected from the group consisting of aryl, alkylaryl, cycloalkyl, heterocycloalkyl, heteroaryl and alkylheteroaryl. More preferably, Y² is an aryl group, more preferably, Y² comprises an, optionally substituted, phenyl ring, and even more preferably Y² is 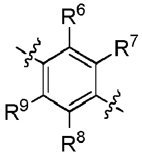 wherein R⁶, R⁷, R⁸ and R⁹ are, independently of each other, H or alkyl, alkenyl, alkinyl, alkyloxy, alkanoyloxy, aryl, heteroaryl, halogen, hydroxyl, mercapto, nitrile, amine, or in each case optionally substituted alkyl, alkenyl, alkinyl, alkyloxy, alkylthio, alkanoyloxy, cycloalkyl, benzyloxy or aryl, most preferably R⁶, R⁷, R⁸ and R⁹ are, independently of each other, alkyl or H, more preferably R⁶, R⁷, R⁸ and R⁹ are H.

Thus, the compound of the invention has preferably the following structure.

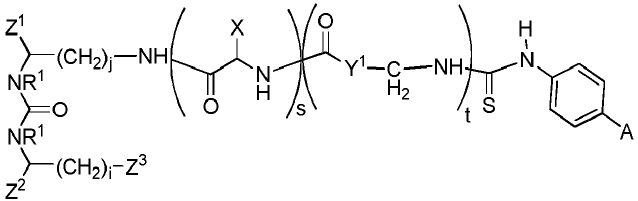

It has been surprisingly found that with such compounds, the interactions with PSMA may be optimized. With the compounds according to this invention improved tumor-to-non-target tissue accumulation and tissue values can be reached and an improved distribution pattern in the non-target tissues can be obtained.

Moreover, the chelators applied in accordance with this invention allow the stable conjugation of radionuclides that can not be used to target PSMA expressing tumors with the actually known tracers.

As described above, in general, the chelator is selected from the group consisting of (Ia), (Ib) and (Ic).

In case of embodiment A, described above and below, integer r is preferably 0. More preferably, A is a chelator selected from the group consisting of 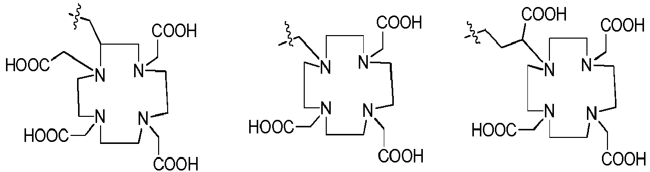 and

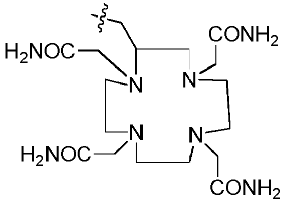

Thus, the present invention also relates to a compound, as described above and below, as well as to a complex comprising said compound, the compound having the following structure. 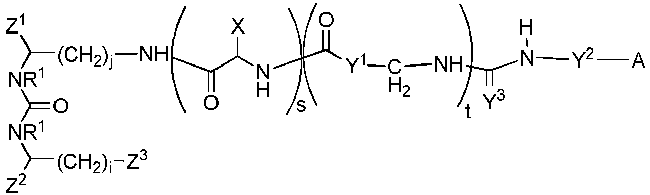 more preferably having the following structure: 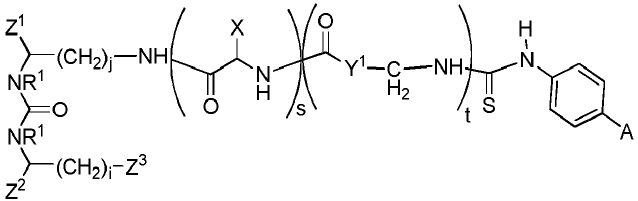 wherein A is a chelator selected from the group consisting of 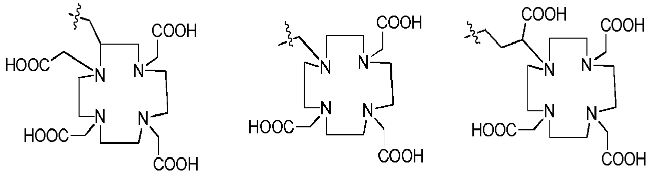 and

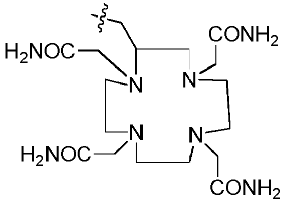

Preferred compounds according to this embodiment have a structure selected from the group consisting of CA007, CA008*, CA009*, CA029* and CA030* (see Table 1A), wherein the compounds CA007, CA008* and CA009* are even more preferred. It is to be understood that if a stereocenter in the respective shown structure in Table 1A is not specified, this shall mean that all respective stereoisomers shall be encompassed, in isolated form as well in form of a mixture of the respective stereoisomers. Thus, the compounds CA008*, CA009*, CA029* and CA030* may be present as single stereoisomers or as mixture of stereoisomers. More preferably, according to this embodiment, the compound have a structure selected from the group consisting of CA007, CA008, CA009, CA029 and CA030 (see Table 1B), wherein the compounds CA007, CA008 and CA009 are particularly preferred.

It has been surprisingly shown that these compounds show a high binding affinity for PSMA and are effectively internalized.

### Embodiment (B)

According to another preferred embodiment of the invention, u is 0 and w is 1. According to this embodiment, the compound of the invention thus has the following structure.

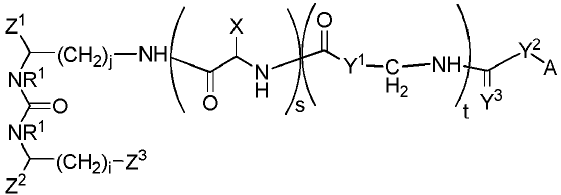

According to this embodiment, A has the structure (Ia) or the structure (Ib).

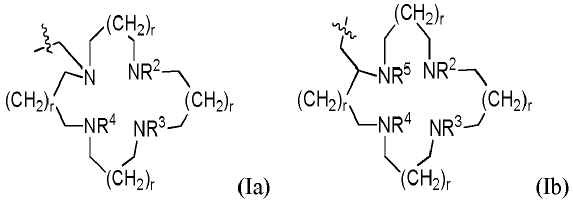

As described above, Y² is selected from the group consisting of aryl, alkylaryl, cycloalkyl, heterocycloalkyl, heteroaryl and alkylheteroaryl. More preferably, Y² is an aryl or heteroaryl group, more preferably, Y² comprises an, optionally substituted, phenyl ring, and even more preferably Y² is 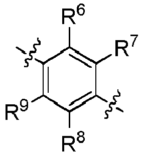 wherein R⁶, R⁷, R⁸ and R⁹ are, independently of each other H or alkyl, most preferably H.

Thus, the present invention also relates to a compound, as described above and below, as well as to a complex comprising said compound, the compound having the following structure: 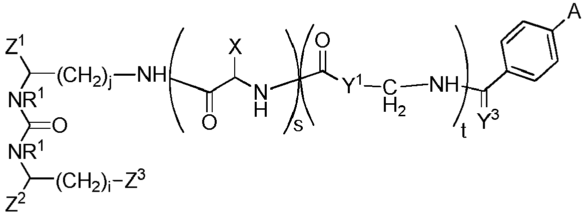 wherein A has the structure (Ia) or the structure (Ib). More preferably, in this case, Y³ is O. As preferred groups A in this context, the following groups are mentioned:

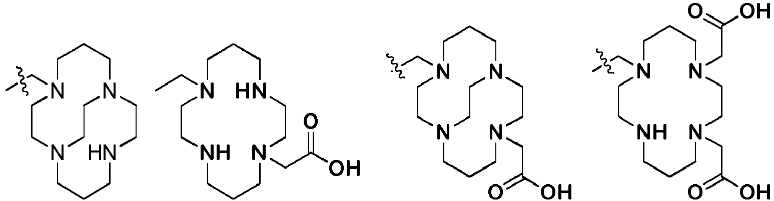

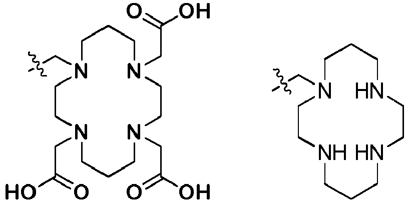

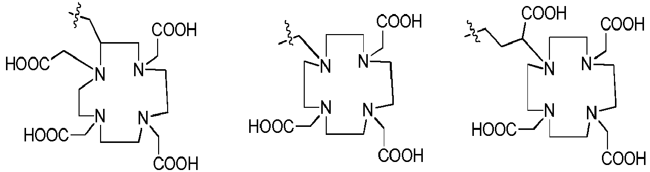

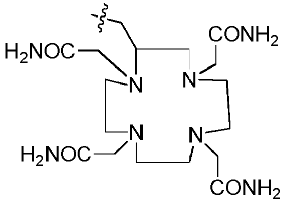

More preferably, A is selected from the group consisting of 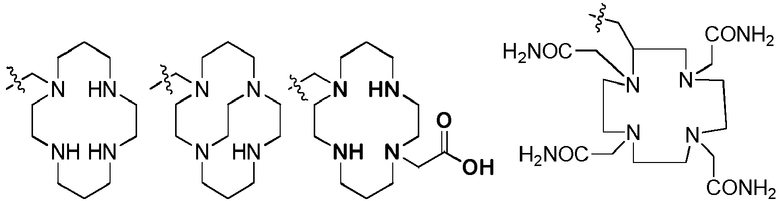 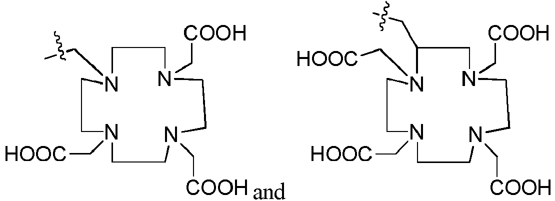 and most preferably A is

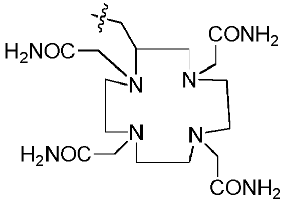

Preferred compounds according to this embodiment have a structure selected from the group consisting of CA001, CA002*, CA003*, CA005*, CA006*, CA007, CA008*, CA009*, CA023*, CA025*, CA026*, CA027, CA028* and CA029* (The structures of these compounds are depicted in Table 1A).

More preferably, the compound has a structure selected from the group consisting of CA007, CA008* and CA009* (The structures of these compounds are depicted in Table 1A). It is to be understood that if a stereocenter in the respective shown structure in Table 1A is not specified, this shall mean that all respective stereoisomers shall be encompassed, in isolated form as well in form of a mixture of the respective stereoisomers. Thus, the compounds CA002*, CA003*, CA005*, CA006*, CA008*, CA009*, CA023*, CA025*, CA026*, CA027, CA028* and CA029* may be present as single stereoisomers or as mixture of stereoisomers. More preferably, according to this embodiment, the compound have a structure selected from the group consisting of CA001, CA002, CA003, CA005, CA006, CA007, CA008, CA009, CA023, CA025, CA026, CA027, CA028 and CA029 (The structures of these compounds are depicted in Table 1B), with compounds CA007, CA008 and CA009 being particularly preferred.

It has been surprisingly shown that these compounds show a high binding affinity for PSMA and are effectively internalized.

### Embodiment (C)

According to another preferred embodiment of the invention A is not

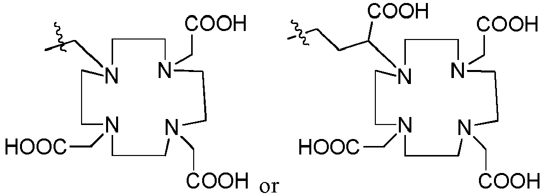

Preferably, according to this embodiment, A is selected from the group consisting of

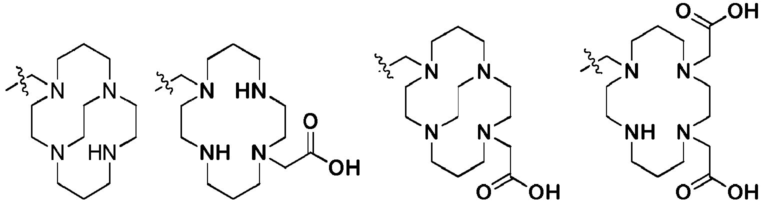

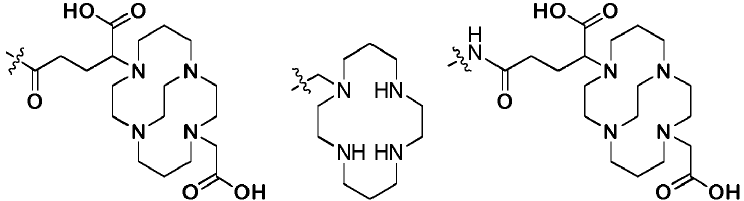

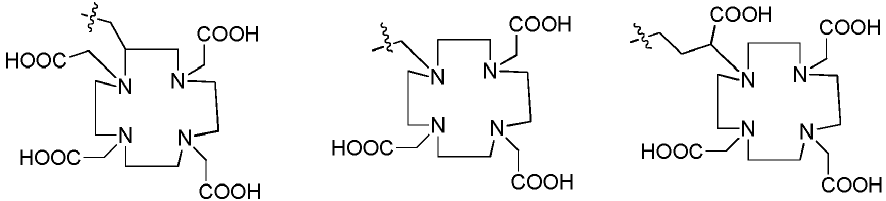

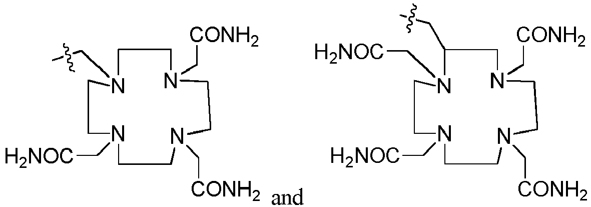

It has surprisingly been found, that the compounds of the invention comprising these chelator building blocks form stable complexes with radionuclides of lead and/or copper and have advantageous tumor targeting properties. The novel compounds provide the possibility to fine tune the pharmacokinetic profile in accordance with the respective radionuclide applied. Furthermore, the compounds allow the stable labelling with specific radionuclides.

### Copper binding compounds:

In case, the compounds are to be used as copper binding PSMA ligands, as mentioned above, A is preferably selected from the group consisting of .

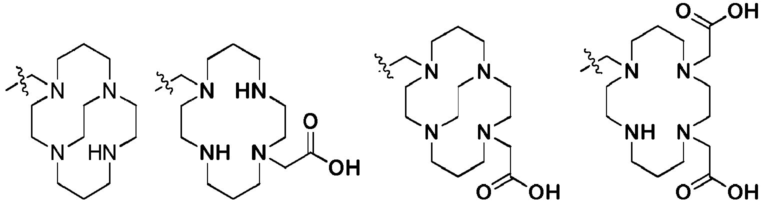

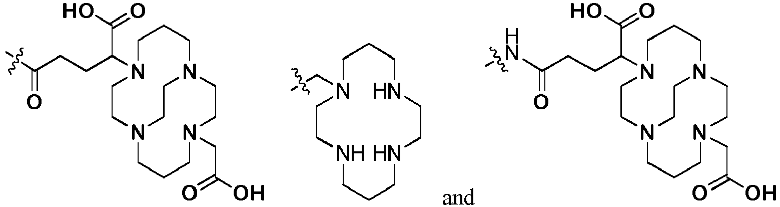

It has surprisingly been found that with these compounds stable and effective complexes with copper radionuclides can be formed. Thus, the present invention also relates to a compound, as described above or below, or a complex, as described above or below, wherein A is 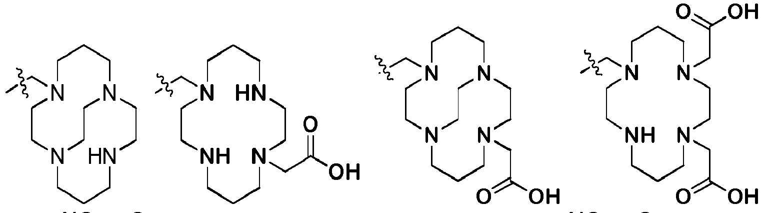 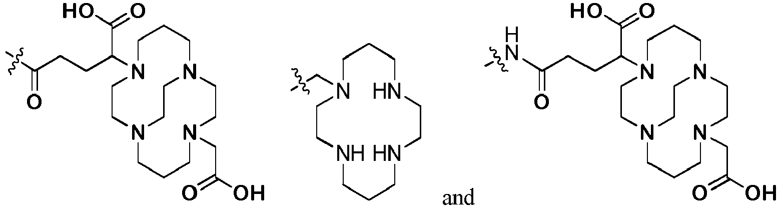 and wherein the radionuclide is a radionuclide of copper, more preferably ⁶⁴Cu and/or ⁶⁷Cu.

As example, the following copper binding compounds should be mentioned CA001, CA002*, CA003*, CA005*, CA006*, CA022*, CA023*, CA024*, CA025* and CA026* (the structures of these compounds are depicted in Table 1A).1). More preferred examples are CA001, CA002, CA003, CA005, CA006, CA022, CA023, CA024, CA025 and CA026 (see Table 1B). It is to be understood that if a stereocenter in the respective shown structure in Table 1A is not specified, this shall mean that all respective stereoisomers shall be encompassed, in isolated form as well in form of a mixture of the respective stereoisomers. Thus, the compounds CA002*, CA003*, CA005*, CA006*, CA022*, CA023*, CA024*, CA025* and CA026*may be present as single stereoisomers or as mixture of stereoisomers.

More preferably, in case the compounds are to be used with copper as radionuclide, the compounds are selected from the group consisting of CA003*, CA006*, CA022* and CA023* (the respective chemical structures are depicted in Table 1A).), preferably of the group CA003, CA006, CA022 and CA023 (see Table 1B)

More preferably, the compound is CA003* (see Table 1A).), mots preferably CA003 (see Table 1B)

It has surprisingly been found that with these compounds two yet unmet needs of PSMA targeting can be fulfilled a) a highly specific enrichment in the tumor is achieved with favourable biodistribution characteristics - in particular a significantly improved kidney clearance and b) the possibility to use isotopes of copper and lead two metals of which preferred radioisotopes exist.

### Lead binding compounds:

In case, the compounds are to be used, e.g., with lead as radionuclide, as mentioned above, A is preferably selected from the group consisting of

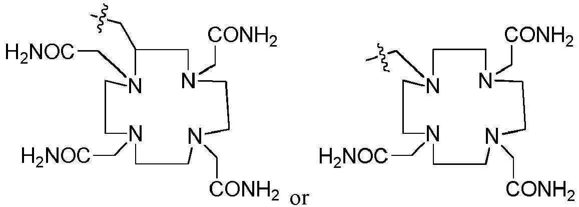

It has surprisingly been found that with such compounds favourable complexes with lead may be formed which show advantageous PSMA targeting properties.

Thus, the present invention also relates to a compound, as described above or below, or a complex, as described above or below, wherein A is 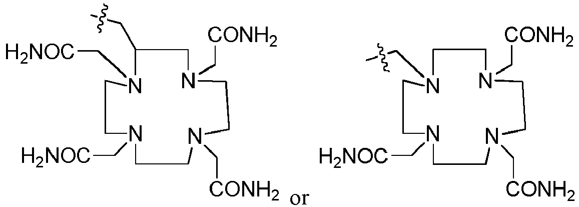 and wherein the radionuclide is a radionuclide of lead, more preferably ²⁰³Pb or ²¹²Pb.

As example, the following lead binding compounds the following compounds should be mentioned CA007, CA008, CA009, CA010, CA011 and CA012 (the structures of these compounds are depicted in Table 1A), preferably CA007, CA008*, CA009*, CA010*, CA011* and CA012*. More preferably, the compound is CA009* or CA012*, most preferably CA009 or CA012. Thus, the present invention relates to a compound or a complex, as described above, wherein the compound has the structure CA009* or CA012 (Table A1) more preferably CA009 or CA012 (see Table 1B.), and wherein the radionuclide is preferably a radionuclide of lead, more preferably ²⁰³Pb or ²¹²Pb.

It has surprisingly been found that these compounds presented a high stability in human serum for 48 h. Further, the compounds revealed a high affinity to inhibit PSMA.

Moreover, the ²⁰³Pb-labeled compounds showed high rates of specific internalization in the PSMA positive cell line.

### Pharmaceutical composition

As described above, the present invention also relates to a pharmaceutical composition comprising a compound, as described above or below, or a complex, as described above or below. It is to be understood that the pharmaceutical compositions comprise therapeutically effective amounts of the compound and/or the complex, respectively. The composition may further comprise at least one organic or inorganic solid or liquid and/or at least one pharmaceutically acceptable carrier.

The phrase "pharmaceutically acceptable" is employed herein to refer to those compounds, material, compositions, and/or dosage forms which are, within the scope of sound medical judgment, suitable for use in contact with the tissues of a patient without excessive toxicity, irritation, allergic response, or other problem or complication, commensurate with a reasonable benefit/risk ratio.

A "patient" includes an animal, such as a human, monkey, cow, horse, cat or dog. The animal can be a mammal such as a non-primate and a primate (e.g., monkey and human). In one embodiment, a patient is a human being.

In general, the compound of formula (1) or the pharmaceutical composition thereof, may be administered orally or via a parenteral route, usually injection or infusion.

A "parenteral administration route" means modes of administration other than enteral and topical administration, usually by injection, and includes, without limitation, intravenous, intramusclular, intraarterial, intrathecal, intracapsular, intraorbital, intracardiac, intradermal, intraperitoneal, transtracheal, subcutaneous, subcuticular, intraarticluare, subcapsular, subarachnoid, intraspinal and intrasternal injection and infusion.

The dosage (referring to the amount of carrier molecule) of the compounds according to the invention is determined by the physician on the basis of the patient-specific parameters, such as age, weight, sex, severity of the disease, etc. The dosage depends on the mode of application: in general, the compounds used for molecular imaging purposes are applied in tracer amounts - i.e. by using a total dose of 1 to 100 nmol per patient the preferred dose being 5 to 20 nmol per patient. For therapeutic application (endoradiotherapy) higher doses are required to achieve the radiation quantity absorbed dose (gray) numbers required to cause a therapeutic effect. For therapeutic applications the doses preferably range from 0.1 nmol/kg to 10 nmol/kg body weight, preferably from 0.2 to 5 nmol/kg body weight and most preferably from 0.5 to 2 nmol/kg body weight. Corresponding to the kind of administration, the medicament is suitably formulated, e.g. in the form of solutions or suspensions, simple tablets or dragees, hard or soft gelatine capsules, suppositories, ovules, preparations for injection, which are prepared according to common galenic methods.

The compounds according to the invention can be formulated, where appropriate, together with further active substances and with excipients and carriers common in pharmaceutical compositions, e.g. - depending on the preparation to be produced - talcum, gum arabic, lactose, starch, magnesium stearate, cocoa butter, aqueous and non-aqueous carriers, fatty bodies of animal or vegetable origin, paraffin derivatives, glycols (in particular polyethylene glycol), various plasticizers, dispersants or emulsifiers, pharmaceutically compatible gases (e.g. air, oxygen, carbon dioxide, etc.), preservatives.

In order to produce liquid preparations, additives, such as sodium chloride solution, ethanol, sorbitol, glycerine, olive oil, almond oil, propylene glycol or ethylene glycol, can be used.

When solutions for infusion or injection are used, they are preferably aqueous solutions or suspensions, it being possible to produce them prior to use, e.g. from lyophilized preparations which contain the active substance as such or together with a carrier, such as mannitol, lactose, glucose, albumin and the like. The readymade solutions are sterilized and, where appropriate, mixed with excipients, e.g. with preservatives, stabilizers, emulsifiers, solubilizers, buffers and/or salts for regulating the osmotic pressure. The sterilization can be obtained by sterile filtration using filters having a small pore size according to which the composition can be lyophilized, where appropriate. Small amounts of antibiotics can also be added to ensure the maintenance of sterility. The phrases effective amount" or "therapeutically-effective amount" as used herein means that amount of a compound, material, or composition comprising a compound of the invention, or other active ingredient which is effective for producing some desired therapeutic effect in at least a subpopulation of cells in a patient at a reasonable benefit/risk ratio applicable to any medical treatment. A therapeutically effective amount with respect to a compound of the invention means that amount of therapeutic agent alone, or in combination with other therapies, that provides a therapeutic benefit in the treatment of prevention of a disease. Used in connection with a compound of the invention, the term can encompass an amount that improves overall therapy, reduces or avoids symptoms or causes of disease, or enhances the therapeutic efficacy of or synergies with another therapeutic agent.

Further, the present invention also relates to a compound, as described above or below, or a complex, as described above or below, or a pharmaceutical composition, as described for use in treating, ameliorating or preventing a cell proliferative disease or disorder, in particular prostate cancer and/or metastases thereof.

Further, the present invention also relates to a compound, as described above or below, or a complex, as described above or below, or a pharmaceutical composition, for use in diagnostics.

Further, the present invention also relates to as described above or below, or a complex, as described above or below, or a pharmaceutical composition, for use in the diagnosis of cancer, in particular of prostate cancer and/or metastases thereof.

As used herein, the terms "treating" or "treatment" is intended to encompass also diagnosis, prophylaxis, prevention, therapy and cure.

The terms, "prevent", "preventing," and "prevention" refer to the prevention of the onset, recurrence, or spread of the disease in a patient resulting from the administration of a prophylactic or therapeutic agent.

Preferably, as described above or below, or a complex, as described above or below, or a pharmaceutical composition, are used for in vivo imaging and radiotherapy. Suitable pharmaceutical compositions may contain a radio imaging agent, or a radiotherapeutic agent that has a radionuclide either as an element, i.e. radioactive iodine, or a radioactive metal chelate complex of the compound of formula (la) and/or (lb) in an amount sufficient for imaging, together with a pharmaceutically acceptable radiological vehicle. The radiological vehicle should be suitable for injection or aspiration, such as human serum albumin; aqueous buffer solutions, e.g., tris(hydromethyl)-aminomethane (and its salts), phosphate, citrate, bicarbonate, etc; sterile water physiological saline; and balanced ionic solutions containing chloride and or dicarbonate salts or normal blood plasma cautions such as calcium potassium, sodium and magnesium.

The concentration of the imaging agent or the therapeutic agent in the radiological vehicle should be sufficient to provide satisfactory imaging. For example, when using an aqueous solution, the dosage ranges from 0.1 to 300 millicuries this broad range is caused by the fact that alpha emitting isotopes exert very strong cytotoxic effects and are therefore applied in low doses such as 0.135 mCi ²²⁵Ac per therapy cycle in case of actinium labeled PSMA-617. In case of beta-emitting radioisotopes such as ¹⁷⁷Lu doses of up to 216 mCi are typically applied in one therapy cycle. These doses can be determined by the skilled person. The actual dose administered to a patient for imaging or therapeutic purposes, however, is determined by the physician administering treatment. The imaging agent or therapeutic agent should be administered so as to remain in the patient for about 1 hour to 10 days, although both longer and shorter time periods are acceptable. Therefore, convenient ampoules containing 1 to 10 mL of aqueous solution may be prepared.

Imaging may be carried out in the normal manner, for example by injecting a sufficient amount of the imaging composition to provide adequate imaging and then scanning with a suitable imaging or scanning machine, such as a tomograph or gamma camera. In certain embodiments, a method of imaging a region in a patient includes the steps of: (i) administering to a patient a diagnostically effective amount of a compound complexed with a radionuclide; exposing a region of the patient to the scanning device; and (ii) obtaining an image of the region of the patient. In certain embodiments of the region imaged is the head or thorax. In other embodiments, the compounds and complexes of formula l(a) and/or (lb) target the PSMA protein.

Thus, in some embodiments, a method of imaging tissue such as spleen tissue, kidney tissue, or PSMA-expressing tumor tissue is provided including contacting the tissue with a complex synthesized by contacting a radionuclide and a formula (la) and/or formula (lb) compound.

The amount of the compound of the present invention, or a formulation comprising a complex of the compound, or its salt, solvate, stereoisomer, or tautomer that is administered to a patient depends on several physiological factors. These factors are known by the physician, including the nature of imaging to be carried out, tissue to be targeted for imaging or therapy and the body weight and medical history of the patient to be imaged or treated using a radiopharmaceutical.

Accordingly in another aspect, the invention provides a method for treating a patient by administering to a patient a therapeutically effective amount of a complex, as described above, to treat a patient suffering from a cell proliferative disease or disorder. Specifically, the cell proliferative disease or disorder to be treated or imaged using a compound, pharmaceutical composition or radiopharmaceutical in accordance with this invention is a cancer, for example, prostate cancer and/or prostate cancer metastasis in e.g. lung, liver, kidney, bones, brain, spinal cord, bladder, etc.

The synthesis of the compounds of the present invention is described in detail in the example section

Summarizing the findings of the present invention, the following embodiments are particularly preferred:
1. A compound of formula (1) 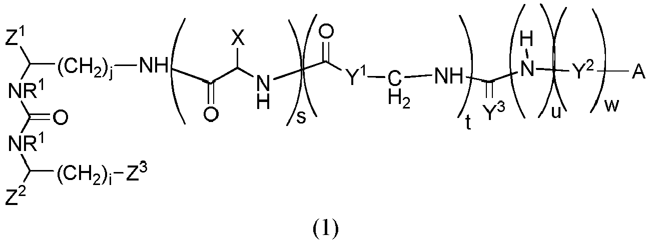
   or a pharmaceutically acceptable salt or solvate thereof,
   wherein Y³ is O or S,
   wherein s, t, u and w are, independently of each other, 0 or 1,
   wherein i is an integer of from 1 to 3,
   wherein j is an integer of from 3 to 5,
   and wherein Z¹, Z² and Z³ are independently of each other, selected from the group consisting of CO₂H, -SO₂H, -SO₃H, -OSO₃H, and -OPO₃H₂,
   R¹ is -CH₃ or H, preferably H
   X is selected from the group consisting of, optionally substituted, alkylaryl (-akyl-aryl), aryl, alkylheteroaryl (-akly-heteroaryl) and heteroaryl,
   Y¹ and Y² are independently, of each other, selected from the group consisting of, optionally substituted, aryl, alkylaryl (-alkyl-aryl), cycloalkyl, heterocycloalkyl, heteroaryl and alkylheteroaryl (-akly-heteroaryl),
   and wherein A is a chelator residue having a structure selected from the group consisting of (Ia), (Ib) and (Ic) 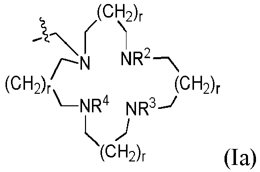 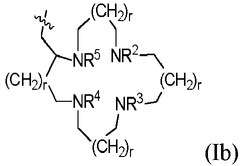 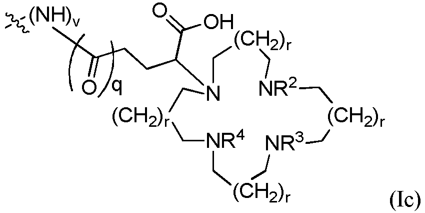
   wherein R², R³, R⁴ and R⁵ are, independently of each other, selected from the group consisting of H, -CH₂-COOH and -CH₂-C(=O)-NH₂ or wherein R² and R⁴ form a -(CH₂)ₙ-bridge with n being an integer of from 1 to 3, wherein n is preferably 2,
   and wherein r, v and q, are independently of each other, 0 or 1,
   with the proviso that in case u and w are 0, q and v are 0, and
      (A) wherein u and w are 1, or
      (B) wherein u is 0 and w is 1, and wherein A is selected from (Ia) or (Ib), or
      (C) A is not 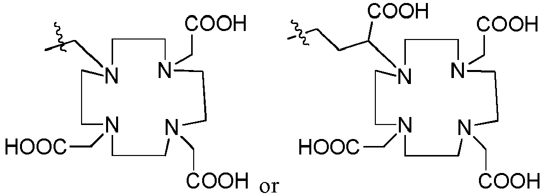
2. The compound of embodiment 1, wherein X preferably comprises a residue selected from the group consisting of, optionally substituted, naphtyl, phenyl, biphenyl, indolyl and benzothiazolyl, more preferably, wherein X is selected from the group consisting of 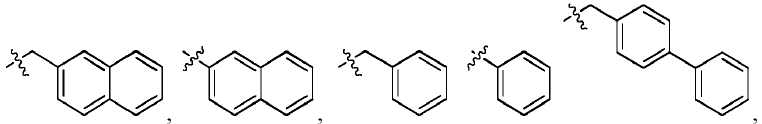 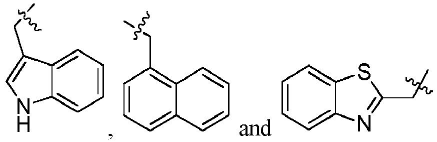
3. The compound of embodiment 1, wherein X is 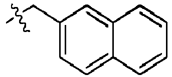
4. The compound of any one of embodiments 1 to 3, wherein Z¹, Z² and Z³ are -CO₂H.
5. The compound of any one of embodiments 1 to 4, wherein R¹ is H.
6. The compound of any one of embodiments 1 to 5, wherein Y' is 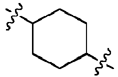
7. The compound of any one of embodiments 1 to 6, wherein i is 2 and j is 4, and wherein the compound has preferably the structure (1a) 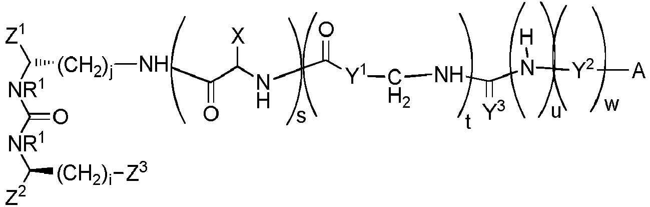
8. The compound of any one of embodiments 1 to 7, wherein u and w are 1.
9. The compound of embodiment 8, wherein Y³ is S.
10. The compound of embodiment 8 or 9, wherein Y² is 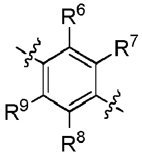 and wherein R⁶, R⁷, R⁸ and R⁹ are, independently of each other H or alkyl, preferably H.
11. The compound of any one of embodiments 8 to 10, wherein r is preferably 0.
12. The compound of any one of embodiments 8 to 11, wherein A is a chelator selected from the group consisting of 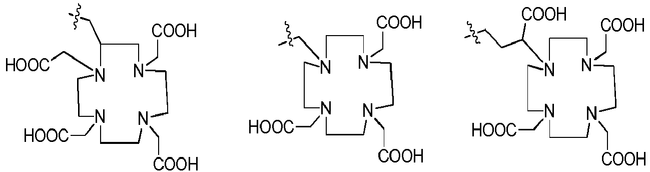 and 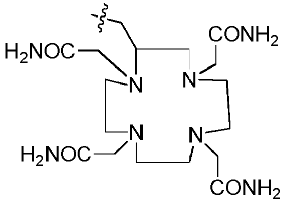
13. The compound of any one of embodiments 8 to 12, having a structure selected from the group consisting of CA007, CA008*, CA009*, CA029* and CA030* (see Table 1A), preferably a structure selected from the group consisting of CA007, CA008, CA009, CA029 and CA030 (see Table 1B.).
14. The compound of any one of claims 8 to 12, having a structure selected from the group consisting of CA007, CA008* and CA009*(see Table 1A), preferably a structure selected from the group consisting of CA007, CA008 and CA009 (see Table 1B).
15. The compound of any one of embodiments 1 to 7, wherein u is 0 and w is 1, and wherein A is selected from (Ia) or (Ib).
16. The compound embodiment 15, wherein Y² is an, optionally substituted, aryl or heteroaryl group.
17. The compound of embodiment 16 or 17, wherein Y² is 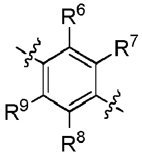 and wherein R⁶, R⁷, R⁸ and R⁹ are, independently of each other H or alkyl, preferably H.
18. The compound of any one of embodiment 15 to 17, wherein A is selected from the group consisting of 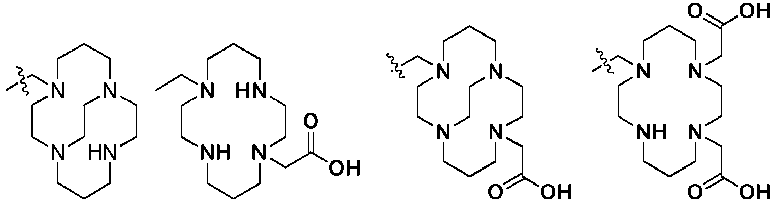 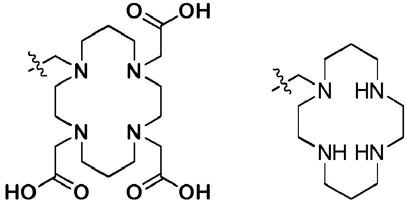 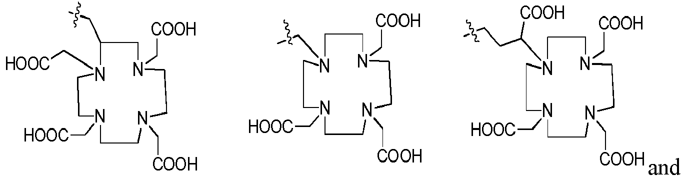 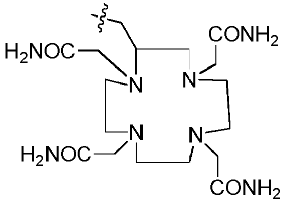
19. The compound of any one of embodiments 15 to 18, wherein A is selected from the group consisting of 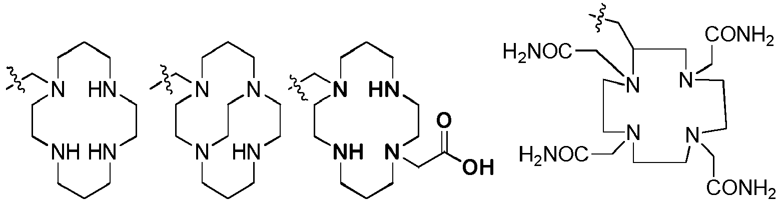 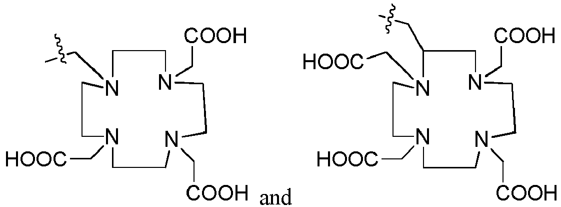
20. The compound of any one of embodiments 15 to 19, wherein A is 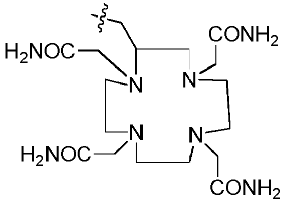
21. The compound of any one of embodiments 15 to 20, having a structure selected from the group consisting of CA001, CA002*, CA003*, CA007, CA008*, CA009*, CA022*, CA023*, CA025*, CA026*, CA027, CA028* and CA029* (see Table 1A), preferably a structure selected from the group consisting of CA001, CA002, CA003, CA007, CA008, CA009, CA022, CA023, CA025, CA026, CA027, CA028 and CA029 (see Table 1B).
22. The compound of any one of embodiments 15 to 21, having a structure selected from the group consisting of CA007, CA008* and CA009*, preferably a structure selected from the group consisting of CA007, CA008 and CA009 (Table 1B).
23. The compound of any one embodiments 1 to 7, wherein A is not 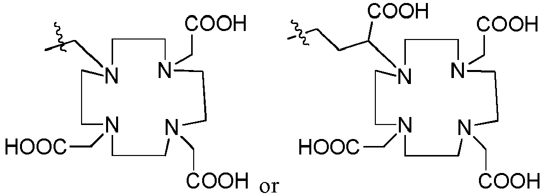
24. The compound of embodiment 23, wherein A is selected from the group consisting of 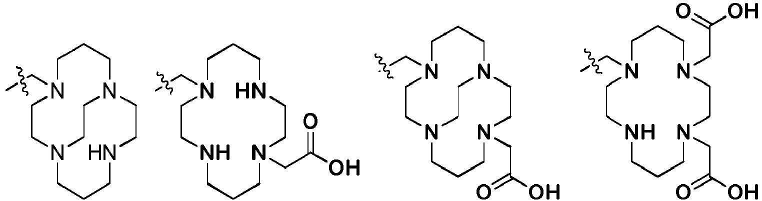 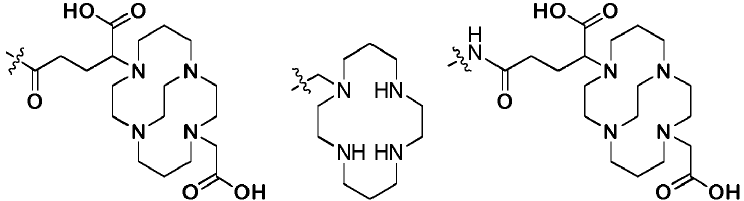 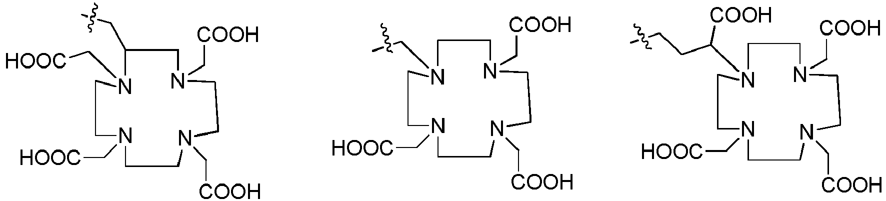 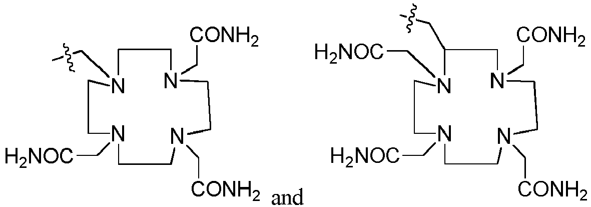
25. Complex comprising
   (a) a radionuclide, and
   (b) the compound of any one claims 1 to 24 or a salt thereof.
26. The complex of embodiment 25, wherein, the radionuclide is selected from the group consisting ⁸⁹Zr, ⁴⁴Sc, ¹¹¹In, ⁹⁰Y, ⁶⁶Ga, ⁶⁷Ga, ⁶⁸Ga, ¹⁷⁷Lu, ^{99m}Tc, ⁶⁰Cu, ⁶¹Cu, ⁶²Cu, ⁶⁴Cu, ⁶⁶Cu, ⁶⁷Cu, ¹⁴⁹Tb, ¹⁵²Tb, ¹⁵⁵Tb, ¹⁶¹Tb, ¹⁵³Sm, ¹⁵³Gd, ¹⁵⁵Gd, ¹⁵⁷Gd, ²¹³Bi, ²²⁵Ac, ²³⁰U, ²²³Ra, ¹⁶⁵Er, radionuclides of Fe (such as ⁵²Fe and ⁵⁹Fe) and radionuclides of Pb (such as ²⁰³Pb and ²¹²Pb, ²¹¹ Pb, ²¹³ Pb, ²¹⁴Pb, ²⁰⁹Pb, ¹⁹⁸Pb, ¹⁹⁷Pb).
27. The complex of embodiment 25, wherein the radionuclide is a radioactive nuclide of lead and A is 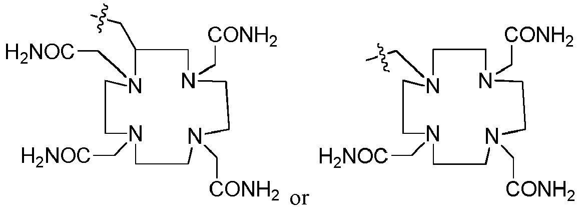
28. The complex of embodiment 27, wherein (b) is a compound having a structure selected from the group consisting of CA007, CA008, CA009, CA010 and CA011 or salts thereof.
29. The complex of embodiment 25, wherein the radionuclide is a radioactive nuclide of copper and A is 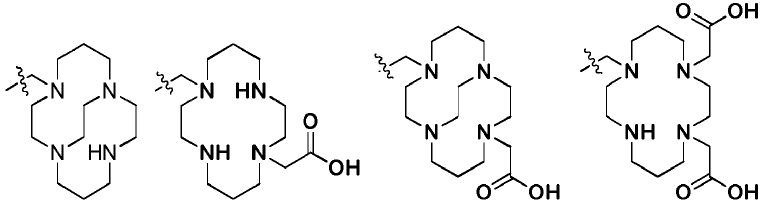 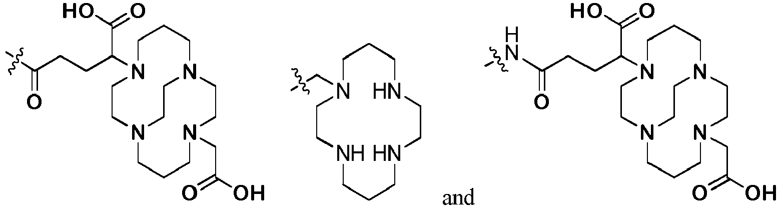
30. A pharmaceutical composition comprising a compound of any of embodiment 1 to 24 or a complex of any one of claims 25 to 29.
31. A compound of any one of embodiments 1 to 24 or a complex of any one of embodiments 25 to 29 or a pharmaceutical composition of embodiment 30 for use in treating, ameliorating or preventing PSMA-expressing cancer and/or metastases thereof, in particular prostate cancer and/or metastases thereof.
32. Compound of any one of embodiments 1 to 24 or a complex of any one of embodiments 25 to 29 or a pharmaceutical composition of embodiment 30 for use in diagnostics.
33. Compound of any one of embodiments e 1 to 24 or a complex of any one of embodiments 25 to 29 or a pharmaceutical composition of embodiment 30 for use in the diagnosis of cancer, such as PSMA-expressing cancer and/or metastases thereof, in particular prostate cancer and/or metastases thereof.

Further, the following embodiments 1a to 16a are particularly preferred:
1a. A compound of formula (1) 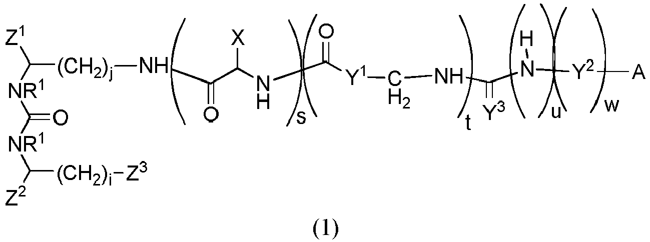
   or a pharmaceutically acceptable salt or solvate thereof,
   wherein Y³ is O or S,
   wherein s, t, u and w are, independently of each other, 0 or 1,
   wherein i is an integer of from 1 to 3,
   wherein j is an integer of from 3 to 5,
   and wherein Z¹, Z² and Z³ are independently of each other, selected from the group consisting of - CO₂H, -SO₂H, -SO₃H, -OSO₃H, and -OPO₃H₂,
   R¹ is -CH₃ or H, preferably H
   X is selected from the group consisting of, optionally substituted, alkylaryl (-alkyl-aryl), aryl, alkylheteroaryl (-alkyl-heteroaryl) and heteroaryl,
   Y' and Y² are independently, of each other, selected from the group consisting of, optionally substituted, aryl, alkylaryl (-alkyl-aryl-), cycloalkyl, heterocycloalkyl, heteroaryl and alkylheteroaryl (-alkyl-heteroaryl),
   and wherein A is a chelator residue having a structure selected from the group consisting of (Ia), (Ib) and (Ic) 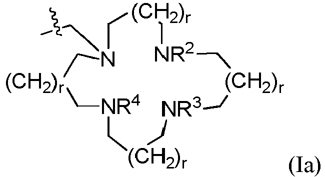 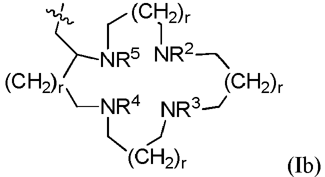 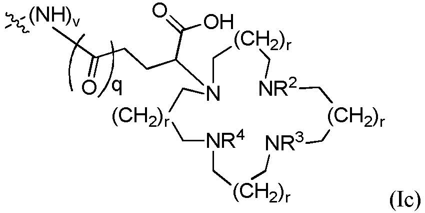
   wherein R², R³, R⁴ and R⁵ are, independently of each other, selected from the group consisting of H, -CH₂-COOH and -CH₂-C(=O)-NH₂ or wherein R² and R⁴ form a -(CH₂)ₙ-bridge with n being an integer of from 1 to 3, wherein n is preferably 2,
   and wherein r, v and q, are independently of each other, 0 or 1,
   with the proviso that in case u and w are 0, q and v are 0, and
      (A) wherein u and w are 1, or
      (B) wherein u is 0 and w is 1, and wherein A is selected from (Ia) or (Ib), or
      (C) wherein A is not 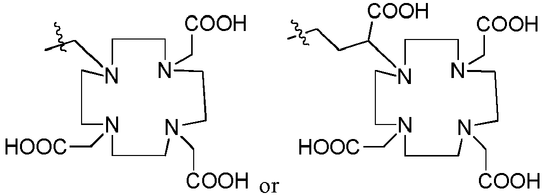
2a The compound of embodiment 1a, wherein X comprises a residue selected from the group consisting of, optionally substituted, phenyl, biphenyl, indolyl and benzothiazolyl, more preferably, wherein X is selected from the group consisting of 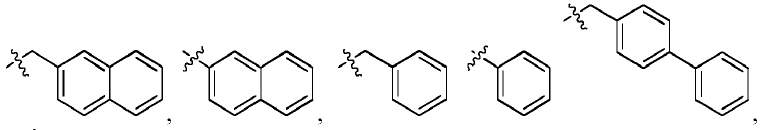 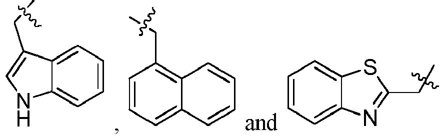 preferably wherein X is 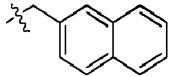
3a The compound of embodiment 2a or 3a, wherein Z¹, Z² and Z³ are -CO₂H and R¹ is H.
4a The compound of any one of embodiments 1a to 3a, wherein Y' is 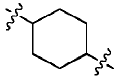
5a The compound of any one of embodiments 1a to 4a, wherein i is 2 and j is 4, and wherein the compound has preferably the structure (1a) 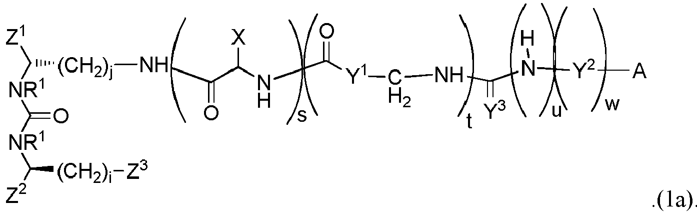
6a The compound of any one of embodiments 1a to 5a, wherein u and w are 1, and wherein
   Y³ is S, and wherein Y² is 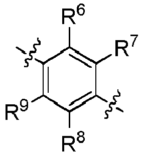
   and wherein R⁶, R⁷, R⁸ and R⁹ are, independently of each other H or alkyl, preferably H.
7a The compound of embodiment 6a, wherein A is a chelator selected from the group consisting of 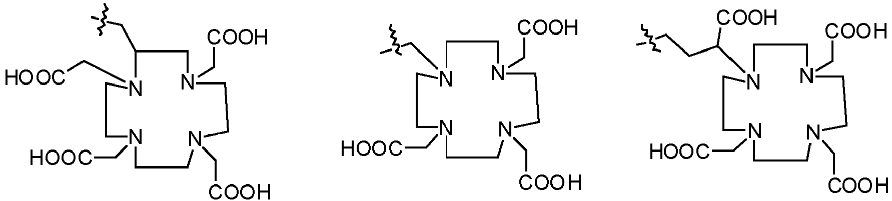 and 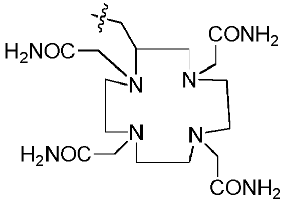
8a The compound of any one of embodiments 1a to 5a, wherein u is 0 and w is 1, and wherein A is selected from (Ia) or (Ib).
9a The compound embodiment 8a, wherein Y² is 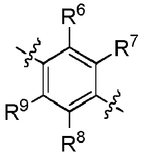 and wherein R⁶, R⁷, R⁸ and R⁹ are, independently of each other H or alkyl, preferably H.
10a The compound of embodiments 8a or 9a, wherein A is selected from the group consisting of 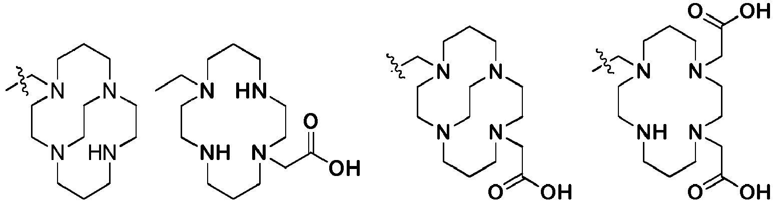 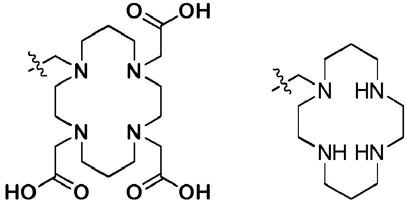 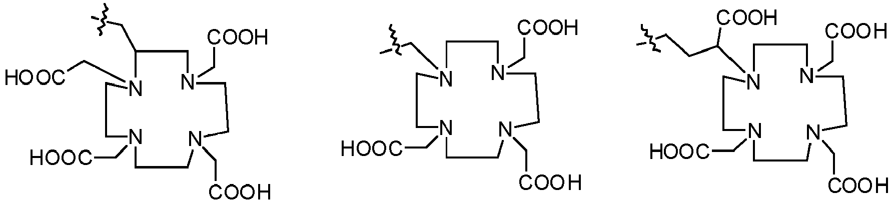 and. 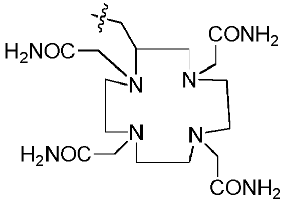
11a Complex comprising
   (a) a radionuclide, and
   (b) the compound of any one embodiments 1a to 10a or a salt thereof.
12a The complex of embodiment 11a, wherein, the radionuclide is selected from the group consisting of ⁸⁹Zr, ⁴⁴Sc, ¹¹¹In, ⁹⁰Y, ⁶⁶Ga, ⁶⁷Ga, ⁶⁸Ga, ¹⁷⁷Lu, ^{99m}Tc, ⁶⁰Cu, ⁶¹Cu, ⁶²Cu, ⁶⁴Cu, ⁶⁶Cu, ⁶⁷Cu, ¹⁴⁹Tb, ¹⁵²Tb, ¹⁵⁵Tb, ¹⁶¹Tb, ¹⁵³Sm, ¹⁵³Gd, ¹⁵⁵Gd, ¹⁵⁷Gd, ²¹³Bi, ²²⁵Ac, ²³⁰U, ²²³Ra, ¹⁶⁵Er, radionuclides of Fe and radionuclides of Pb.
13a The complex of embodiment 11a, wherein the radionuclide is a radioactive nuclide of lead (Pb) and A is 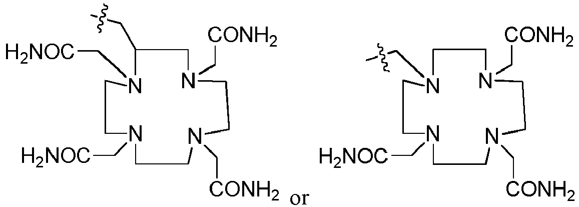
14a The complex of embodiment 11a, wherein the radionuclide is a radioactive nuclide of copper and A is 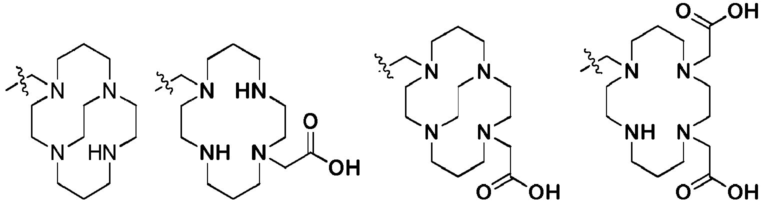 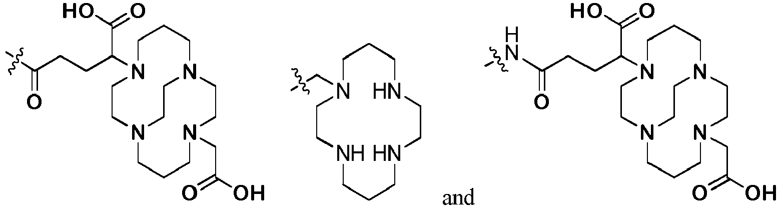
15a A pharmaceutical composition comprising a compound of any one of embodiments1a to 10a or a complex of any one of embodiments 11a to 14a.
16a A compound of any one of embodiments 1a to 10a or a complex of any one of embodiments 11a to 14a or a pharmaceutical composition of embodiment 15a for use in treating, ameliorating or preventing PSMA-expressing cancer and/or metastases thereof, in particular prostate cancer and/or metastases thereof, or for use in diagnostics.

All references cited throughout this specification are herewith incorporated by reference with respect to the specifically mentioned disclosure content as well as in their entireties.

### FIGURES

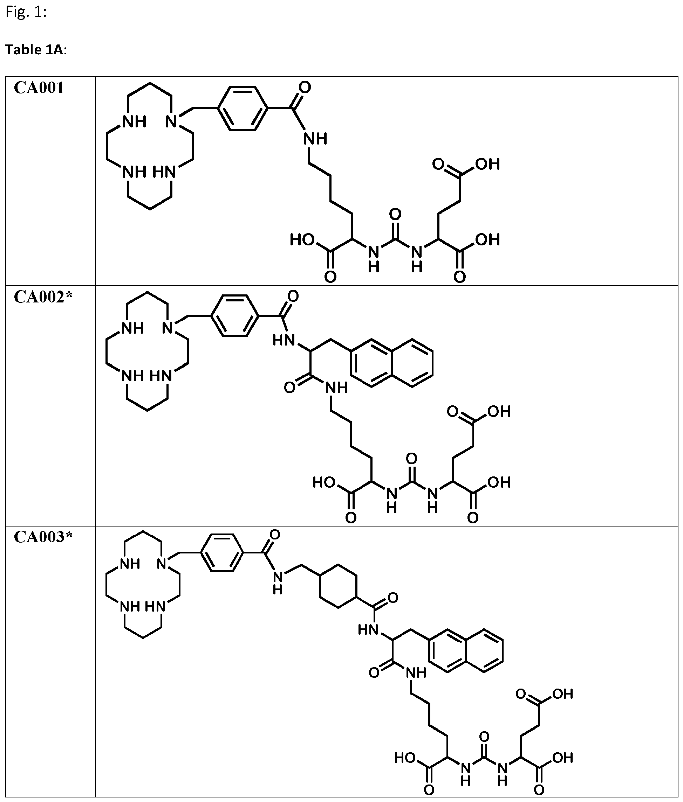

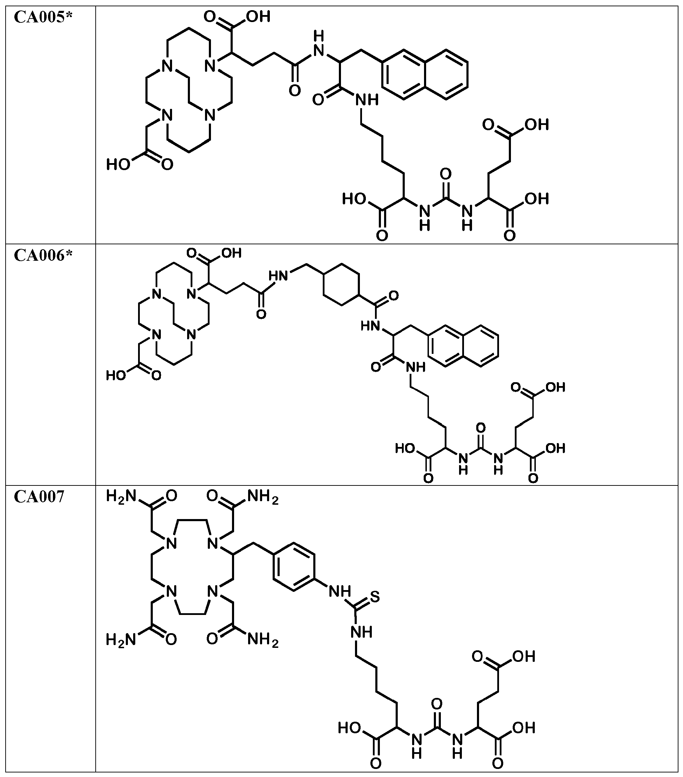

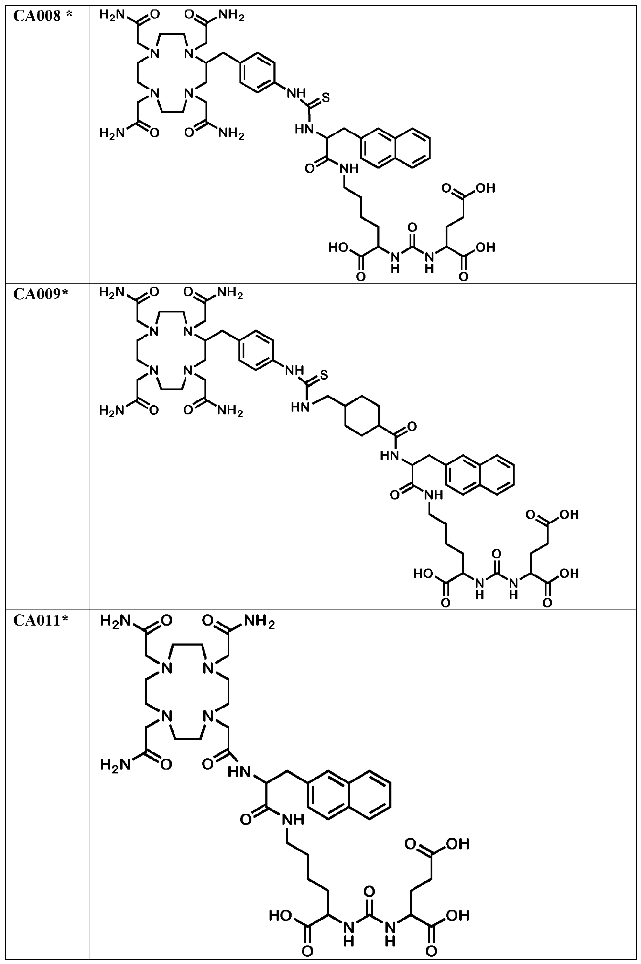

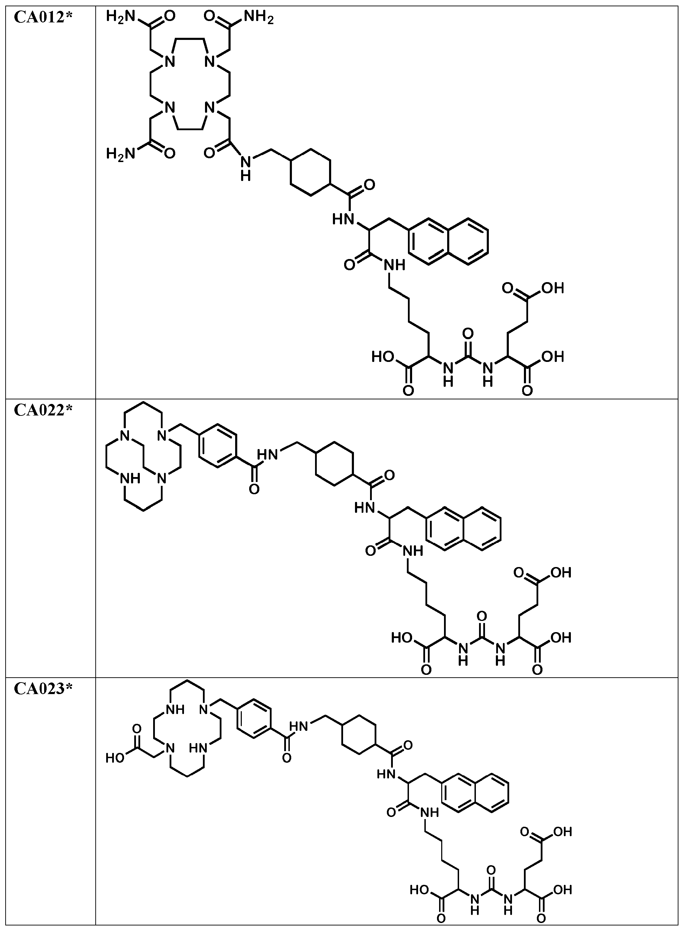

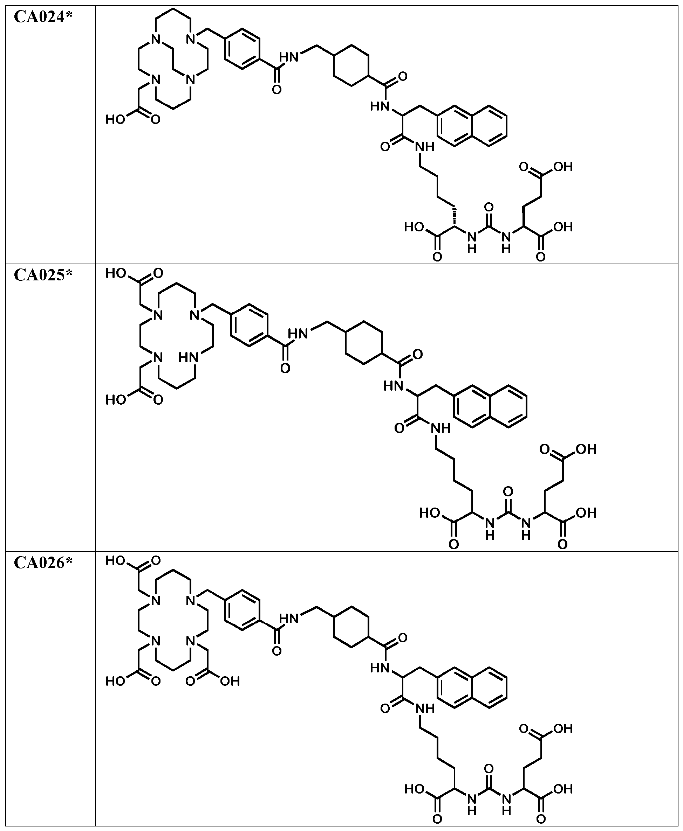

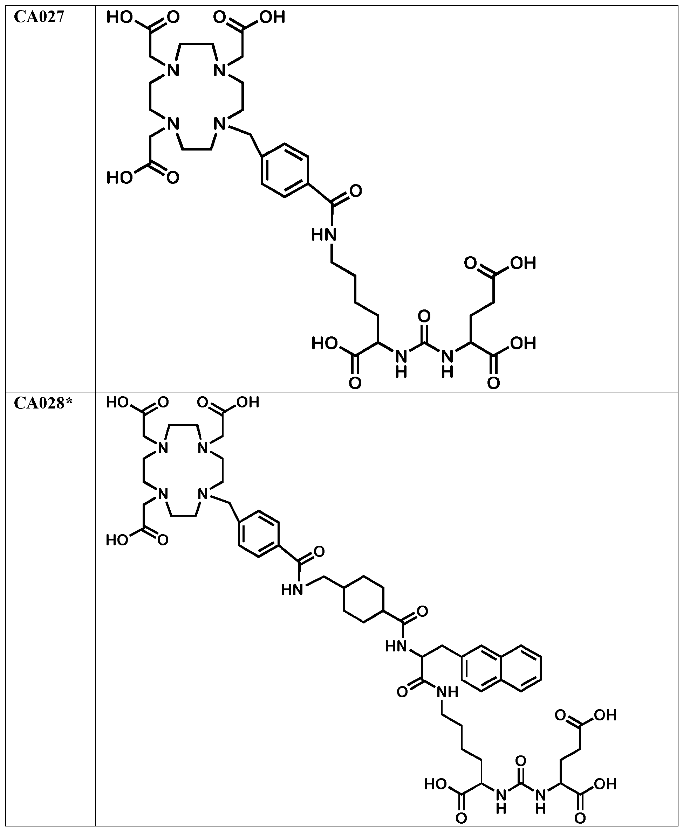

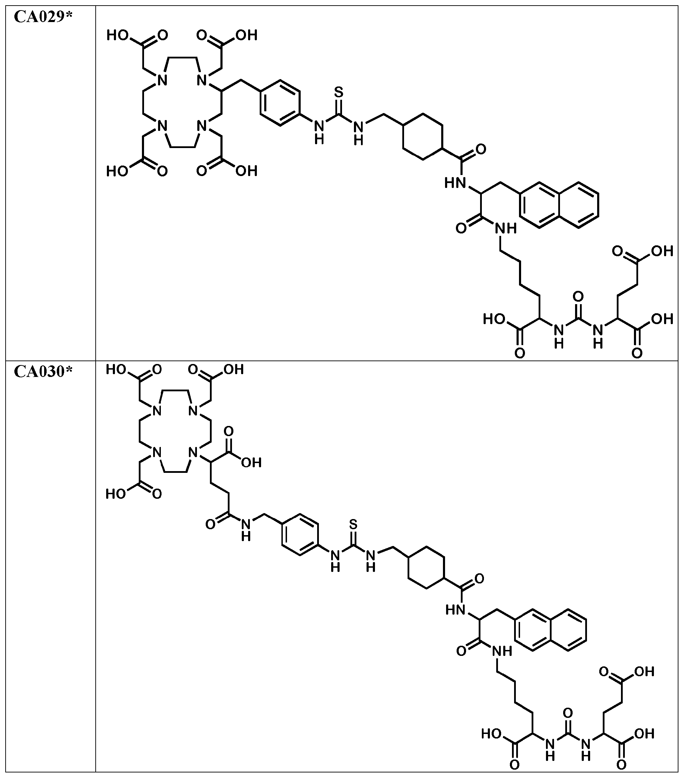

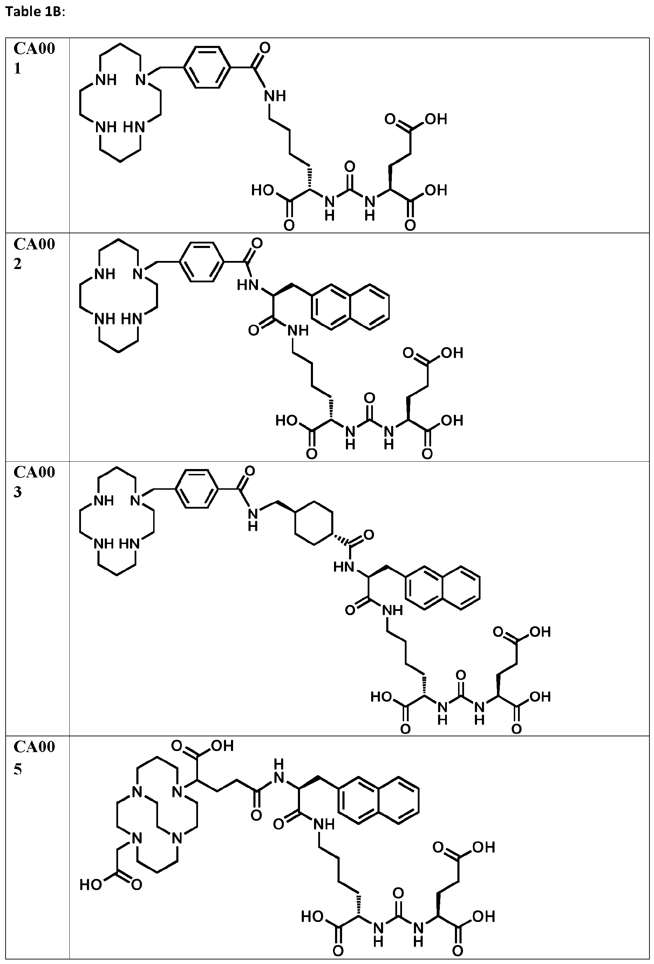

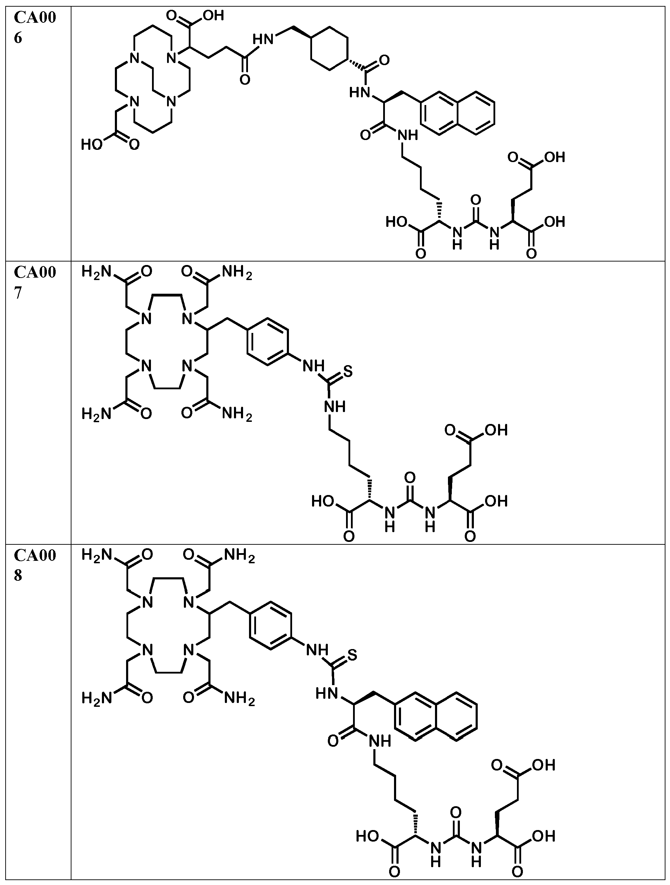

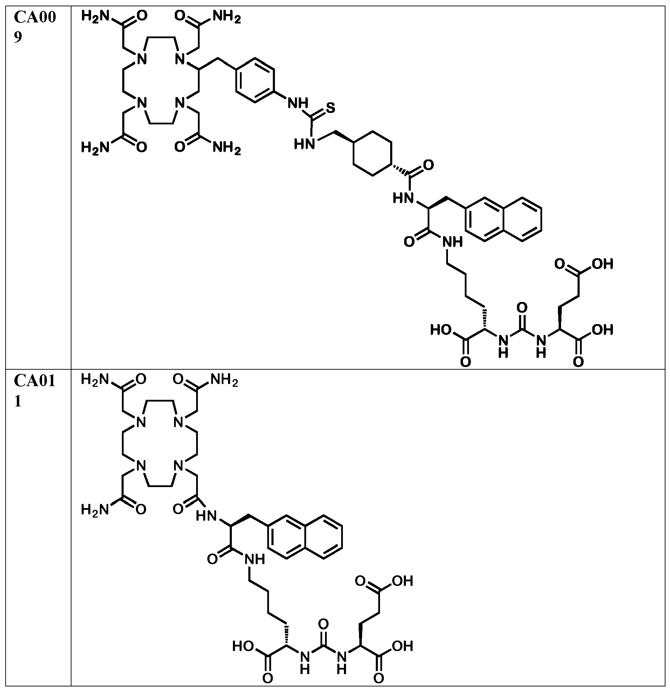

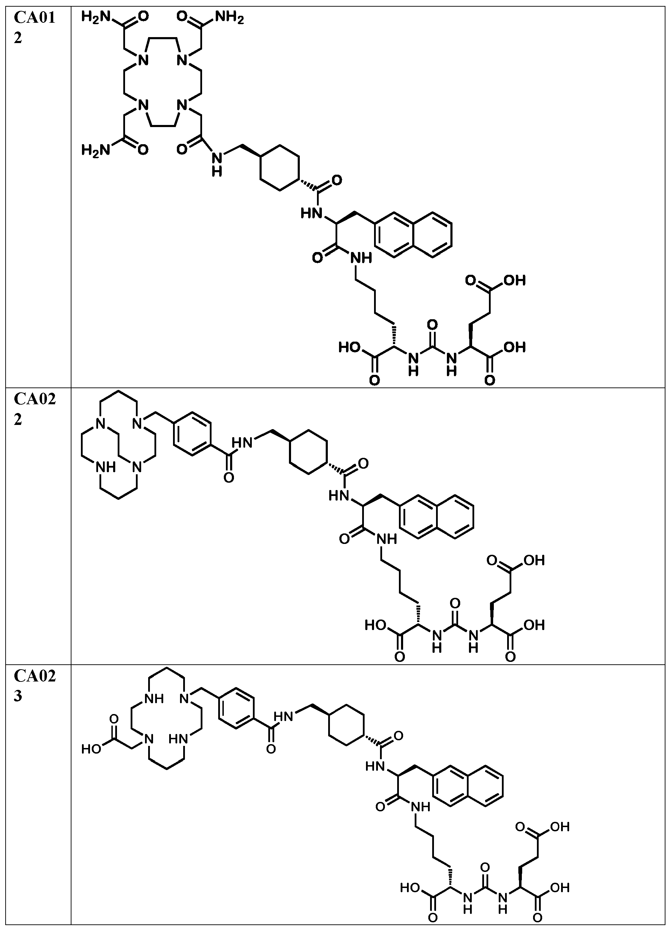

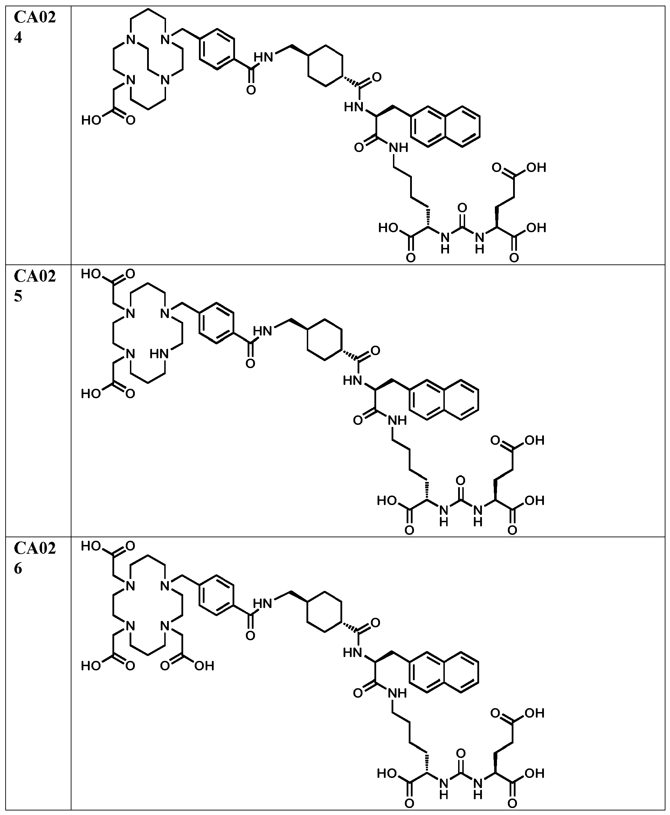

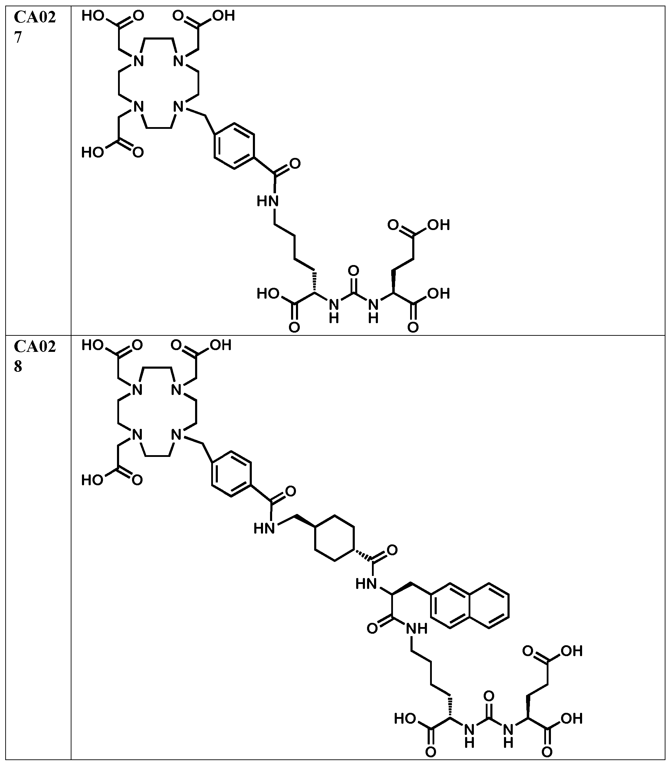

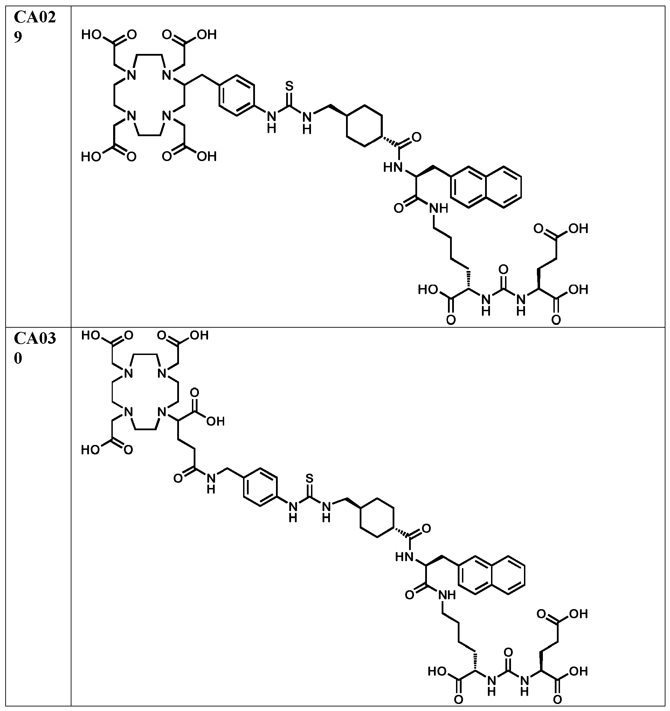

**Figure 1****:** Table 1A: Overview over preferred compounds. It is to be understood that if a stereocenter in the respective shown structure is not specified, this shall mean that all respective stereoisomers shall be encompassed, in isolated form as well in form of a mixture of the respective stereoisomers, Table 1B: Overview over very preferred compounds.
**Figure 2****:** Reaction scheme for the synthesis of the PSMA-chelator conjugates (a) triphosgene, DIPEA, CH₂Cl₂, 0 °C; (b) H-Lys(Alloc)-2CT-resin, CH₂Cl₂ (c) Pd[P(C₆H₅)₃]₄, morpholine, CH₂Cl₂; (d) Fmoc-2-Nal-OH, HBTU, DIPEA, DMF; (e) 20% piperidine, DMF; (f) trans-4-(Fmoc-aminomethyl)-cyclohexanecarboxylic acid, HBTU, DIPEA, DMF; (g) 20% piperidine, DMF; (h) chelator, HBTU (if necessary), DIPEA, DMF; (i) 95% TFA, 2.5% H₂O, 2.5% TIPS.
**Figure 3****:** PSMA inhibition potencies and specific internalization values of the novel PSMA ligands labeled with the nuclide specified in the table. Competitive cell binding was performed using the PSMA-positive C4-2 cell line. The new ligands showed good inhibition potencies with K*ᵢ* in the low nanomolar range and partially higher internalization values (specific lysate) than PSMA-617.

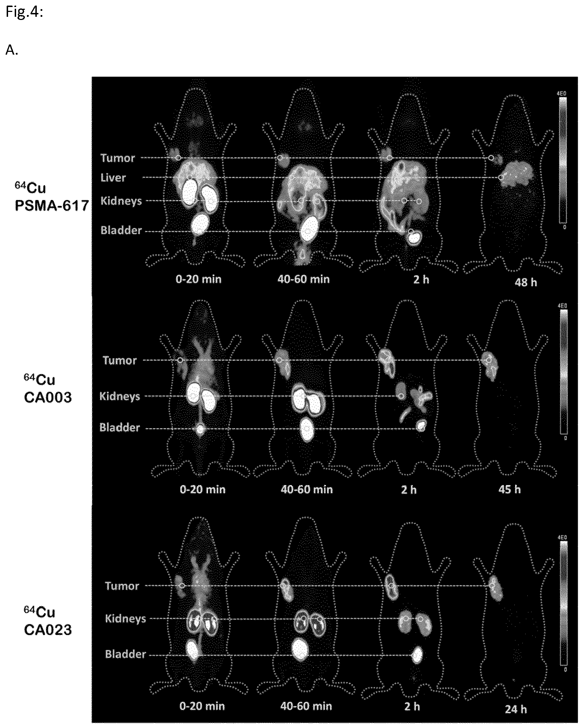

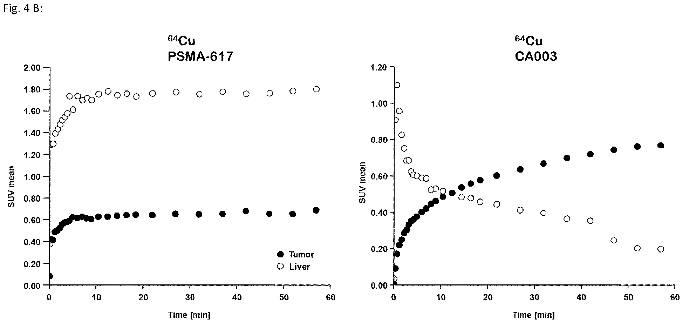

**Figure 4****:** A.PET scan and time-activity curves of C4-2 tumour bearing mice showing biodistribution of ⁶⁴Cu-PSMA-617, ⁶⁴Cu-CA003 and ⁶⁴Cu-CA023. ⁶⁴Cu-PSMA-617 shows high accumulation in the tumor and kidneys but also in the liver. A. Visualization of biodistribution of ⁶⁴Cu-PSMA-617, ⁶⁴Cu-CA003 and ⁶⁴Cu-CA023 at different time points (0-20 min, 40-60 min, 2 h, 48 h). **B.** Time-activity curve of dynamic PET scan (0-60 min) of C4-2 tumour bearing mice showing biodistribution of⁶⁴Cu-PSMA-617 and ⁶⁴Cu-CA003. Standard-uptake-values (SUV) for tumor and liver show higher uptake of ⁶⁴Cu-PSMA-617 in the liver than in the tumor, contrary to ⁶⁴Cu-CA003.

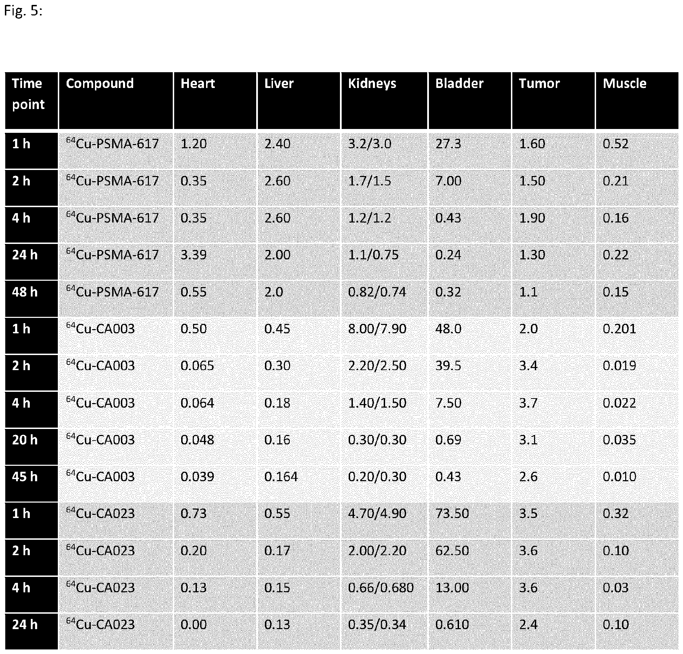

**Figure 5****:** Maximum standard-uptake values (mSUV) of ⁶⁴Cu-PSMA-617, ⁶⁴Cu-CA003 and ⁶⁴Cu-CA023 in PET images of C4-2 tumour bearing mice at different time points (0-48 h). ⁶⁴Cu-CA003 shows high accumulation in the tumor and kidneys, ⁶⁴Cu-CA023shows high accumulation in the tumor and fast clearance from the kidneys.
**Figure 6A****:** Accumulation of ⁶⁴Cu-CA003 in dissected organs of interest in C4-2 tumour bearing mice confirms tumor specificity and fast clearance from the kidneys.
**Figure 6B****:** Accumulation of ⁶⁴Cu-CA023 in dissected organs of interest in C4-2 tumour bearing mice confirms tumor specificity and fast clearance from the kidneys
**Figure 6C****:** Organ distribution of 0.025 nmol of ⁶⁴Cu-CA003 at the time points: 10 min, 1 h, 4 h, 24 h and 72 h after injection. Values are expressed as % ID/g of tissue ± standard deviation; n = 3 for all tissues.
**Figure 6D****:** In a blockade experiment (B), the radiotracer ⁶⁴Cu-CA003 (0.030 nmol) was injected at the same time as 2 mg of PSMA-617 per kilogram of body weight. Values are expressed as % ID/g of tissue ± standard deviation; n = 3 for all tissues.
**Figure 7**: ⁶⁴Cu-CA003 (200 MBq, 0.5 nmol) PET/CT maximum intensity projections of a patient at 2 h (A) and 20 h post injection (B). The red arrows point to selected right shoulder soft-tissue infiltration from scapula origin, lung, bone and lymph-node metastases increased in contrast over time. The hepatobiliary clearance causes hot-spots inside the intestine; cross-sectional slices (C) are mandatory to avoid false-positive readings.
**Figure 8****:** Planar scintigraphic imaging of different ²⁰³Pb-labeled compounds A) at 1 h after tail vein injection and (B) time course of the distribution of ²⁰³Pb-CA012 in BALB/c nu/nu C4-2 tumor bearing mice. The radiolabelled derivatives of CA009 and CA012 show high uptake of the tracer in the tumor tissue. The kinetics of the uptake determined for ²⁰³Pb-CA012 reveals a long retention of the radiotracer in the tumor tissue. The selectivity of uptake of ²⁰³Pb-CA012 is enhanced when compared to of ²⁰³Pb-CA009. This is the result of the rapid clearance of the non-target organs.
**Figure 9****:** Organ distribution of ²⁰³Pb-PSMA-CA012 in tumor bearing mice 0.025 nmol of ²⁰³Pb-PSMA-CA012. This quantification confirms the results of the imaging experiment. The high ratio of the tumor to kidney uptake is achieved due to the high excretion valued observed for the kidneys.
**Figure 10****:** Geometric mean images of ²⁰³Pb-CA012 planar scans over time (A) in comparison to a treatment scan with ¹⁷⁷Lu-PSMA-617 (B); both acquired with a Medium Energy collimator.
**Figure 11****:** Safety Dosimetry estimate of diagnostic ²⁰³Pb-CA012 (left column) and therapeutic ²¹²Pb-CA012 (right column) based on the male-adult phantom in OLINDA (ULI = upper large intestine, LLI = lower large intestine)
**Figure 12****:** Dosimetry of ²¹²Pb-CA012 ("TCMC"-PSMA-617) for salivary glands, randomly chosen tumor lesions (sphere model) and the presumably dose-limiting organs in comparison ²¹³Bi-PSMA-617 and ²²⁵Ac-PSMA-617.
**Figure 13A****:** Maximum intensity projection of a ⁶⁸Ga-PSMA-CA028 PET scan of a patient with multiple lymph node prostate cancer metastases at 1 h and 3 h post injection. Cross-sectional slices demonstrate lymph-node metastases (indicated by red arrows) axillar and hilar (C); also delineable on the correlated CT which serves as a standard of reference.
**Figure 13B****.** Maximum intensity projections of PSMA-PET performed 1 h (A) and 3 h (B) post injection of 295 MBq / 20 nmol ⁶⁸Ga-CA030. Arrows point to the position of the cross-sectional slices demonstrating bone-metastases in multiple regions of the axial skeleton (C-E). In CT (F), no typical osteoblastic reactions allowed tumor-delineation by morphological information alone.
**Figure 14****:** Organ distribution expressed as % ID/g of tissue ± SD (n = 3) of ⁶⁸Ga-PSMA-CA028 at 1 h, 2 h and 4 h after injection.
**Figure 15****:** Comparison of the whole-body small-animal PET imaging of selected ⁶⁸Ga-PSMA ligands 2 h after injection (A) and time course of ⁶⁸Ga-CA028 (B) and the time course of⁶⁸Ga-CA030 (C), in BALB/c nu/nu mice bearing a C4-2 tumor xenograft.
**Figure 16****:** Serum stability of ¹⁷⁷Lu-CA028, ¹⁷⁷Lu-CA029, ¹⁷⁷Lu-CA030 in comparison with ¹⁷⁷Lu-PSMA-617 at 37 °C over 72 h (mean ± SD, n = 3) as determined by radio-ITLC.
**Figure 17****:** Serum stability of ⁶⁴Cu-CA003, ⁶⁴Cu-CA005 and ⁶⁴Cu-PSMA-617 at 37 °C over 72 h (mean ± SD, n = 4) as determined by radio-ITLC.
**Figure 18****:** Serum stability of ⁶⁴Cu-CA003, ⁶⁴Cu-CA005 and ⁶⁴Cu-PSMA-617 at 37 °C over 72 h (mean ± SD, n = 4) as determined by activity measurement.
**Figure19****:** *In vivo* metabolite analysis of ⁶⁴Cu-CA003 in a BALB/c nude mouse (no tumor) at 10 min *p.i.* Radio-HPLC chromatograms of extracts from the kidney, the blood and the liver show that the activity elutes at the retention time of the intact tracer. This proves the integrity of the copper complex within the main distribution period.
**Figure 20****:** Radio-HPLC chromatograms of extracts of ⁶⁴Cu-CA003 in liver in comparison with of ⁶⁴Cu-chloride in the liver in a BALB/c nude mouse (no tumor) at 10 min *p.i.*
**Figure 21A****:** Whole-body small-animal PET scans as maximum-intensity projections of BALB/c nu/nu mice bearing C4-2 tumor xenografts. PET imaging of ⁶⁴Cu-PSMA-617 (10 MBq, 0.2 nmol), ⁶⁴Cu-PSMA-CA003 (10 MBq, 0.2 nmol),
**Figure 21B****:** ⁶⁴Cu-PSMA-CA003 (5 MBq, 0.030 nmol) co-injected with an excess amount of non-labeled PSMA-617 (2 mg per kilogram of body weight) and ⁶⁴Cu-chloride (10 MBq). The color bar gives a link between the SUV and the color scale of the PET image with 0 = minimum and 4 = maximum.
**Figure 22****:** Comparison of the whole body small animal PET scans as maximum-intensity projections of BALB/c nu/nu mice bearing C4-2 tumor xenografts. PET imaging of the four newly PSMA ligands radiolabeled with ⁶⁸Ga (20 MBq; 0.2 nmol) 2 h post injection (A) , the time course of ⁶⁸Ga-CA028 (B) and the time course of ⁶⁸Ga-CA030 (C). The color bar gives a link between the SUV and the color scale of the PET image with 0 = minimum and 4E0 = maximum.
**Figure 23****:** Blood time-activity curve for ⁶⁸Ga PSMA-CA027 (0.6 nmol, 5 MBq) and ⁶⁸Ga PSMA-CA028 (0.6 nmol, 6 MBq), including bi-exponential curve fit.

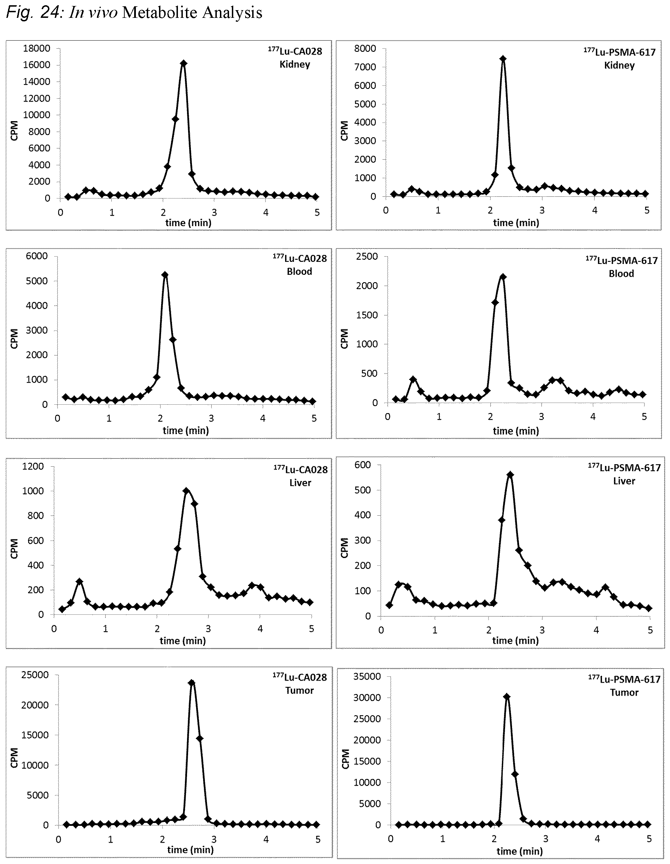

**Figure 24****.** *In vivo* metabolite analysis of ¹⁷⁷Lu-CA028 in comparison with ¹⁷⁷Lu-PSMA-617 (10 MBq, 0.2 nmol in approximately 100 µl of 0.9% saline) in a BALB/c nude mouse (tumor) at 1 h*p.i..* Radio-HPLC chromatograms of extracts from the kidney, the blood, the liver and tumor show that the activity elutes at the retention time of the intact tracer. This proves the integrity of the complex within the main distribution period.
**Figure 25****.** Organ distribution of 0.05 nmol of ⁶⁸Ga-CA028 expressed as % ID/g of tissue ± SD (n = 3) at 20 min, 1 h, 2 h and 4 h after injection.
**Figure 26****.** Radio-HPLC chromatograms of the novel compounds labeled with ⁶⁴Cu.
**Figure 27****:** Time-activity curves of the novel PSMA ligands labeled with ⁶⁸Ga. (A) Time activity curves for kidney and (B) time activity curves for the tumor up to 1 h post injection. Data are mean standardized uptake values (SUV mean).
**Figure 28****.** (A) PET image of 9 MBq (0.30 nmol) ⁶⁴Cu-CA003 10 min post injection in a female Swiss mouse. The maximum intensity projection (MIP) illustrates circulation in the blood and renal uptake. (B) PET image of a female Swiss mouse at 10 min p.i. of 10 MBq ⁶⁴Cu 10 min post injection. The maximum intensity projection (MIP) illustrates a strong uptake in the liver and the kidneys.

The following examples shall merely illustrate the invention. Whatsoever, they shall not be construed as limiting the scope of the invention.

### Examples:

### MATERIALS AND METHODS

Solvents and chemicals were purchased from Merck (Darmstadt, Germany) and Sigma-Aldrich (Munich, Germany) and used without further purification. The *in vitro* experiments were conducted in triplicate and at least three independent sets of data were obtained for each experiment performed. The PET imaging of the prostate cancer patient was consented by the University Hospital Heidelberg following the German laws in vigor and granted the Helsinki Declaration (permit S321/2012).

### Synthesis of the chelator moieties

The chelator moieties were synthesized in high yields and characterized by LC-MS. The synthesis of the chelator 4-[(1,4,8,11-tetraazacyclotetradec-1-yl)-methyl] benzoic acid, a bifunctional macrocyclic cyclam analogue, was described by Studer and Kaden (Studer M, and Kadan, T.A. One-step synthesis of mono-N-substituted azamacrocycles with a carboxylic group in the side-chain and their complexes with Cu2+ and Ni2+. Helvetica. 1986; 69:2081-2086), while 4-carboxymethyl-11-(1,3-dicarboxypropyl)-1,4,8,11-tetraazabicyclo[6.6.2]hexadecane-pentanedioic acid, a cross-bridged chelator, was reported by Boswell *et al.* (Boswell CA, Regino CA, Baidoo KE, et al. Synthesis of a cross-bridged cyclam derivative for peptide conjugation and 64Cu radiolabeling. Bioconjug Chem. 2008;19:1476-1484).

### I. General procedure: Synthesis of novel PSMA Ligands

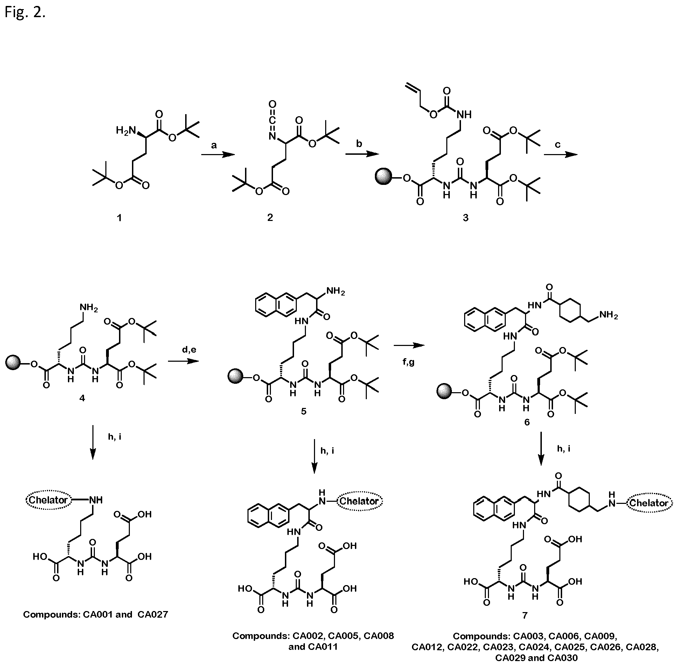

The PSMA-binding motif was prepared by solid-phase synthesis on a 2-chlorotrityl resin (2CT-resin), as previously described by Eder *et al.* (Eder M, Schäfer M, Bauder-Wiist U, et al. 68Ga-complex lipophilicity and the targeting property of a urea-based PSMA inhibitor for PET imaging. Bioconjug Chem. 2012;23:688-697) and Benešová *et al.* (Benesova M, Schäfer M, Bauder-Wüst U, et al. Preclinical Evaluation of a Tailor-Made DOTA-Conjugated PSMA Inhibitor with Optimized Linker Moiety for Imaging and Endoradiotherapy of Prostate Cancer. J Nucl Med. 2015;56:914-920) see Fig. 2. For this purpose Fmoc-Lys(Alloc)-OH was immobilized on an equimolar amount of 2-chlorotrityl resin. Afterwards, the isocyanate **(2)** of the glutamyl moiety by was generated using triphosgene. The ε-allyloxycarbonyl-protected lysine immobilized on 2-chloro-tritylresin was added and reacted for 16 h with careful agitation resulting in compound **3.** The resin was filtered off and the allyloxycarbonyl-protecting group was cleaved to obtain **(4).** In order to obtain compounds CA001 and CA027, the respective chelator was coupled to this intermediate. Subsequently, the PSMA coupled to the chelator was cleaved from the resin. Alternatively, the coupling of Fmoc-2-naphthylalanine was proceeded to obtain **(5).** In order to obtain compounds CA002, CA005, CA008 and CA011 the respective chelator was coupled to this intermediate. Subsequently, the PSMA coupled to the chelator was cleaved from the resin. Alternatively, *trans*-4-(Fmoc-aminomethyl) cyclohexanecarboxylic acid was coupled to obtain **(6),** the compound to which the respective chelator was coupled to obtain compounds CA003, CA006, CA009, CA012, CA022, CA023, CA024, CA025, CA026, CA028, CA029, and CA030. Subsequently, the PSMA coupled to the chelator was cleaved from the resin. The structures were confirmed by HPLC and MS-LC. The substances were isolated by preparative HPLC using water-acetonitrile gradients containing trifluoroacetic acid. For this, the compounds were purified using a gradient of 20 - 50% of acetonitrile in water over 15 min. The purified compounds were analyzed by analytical HPLC (0-100%) acetonitrile in water containing trifluoroacetic acid over 5 min, Monolith RP HPLC column 100 × 3 mm and LC/MS. The product fractions were pooled and lyophilized.

### II. Ligands for Imaging and Therapy with Copper Isotopes

### Specification for (CA001)

The product was obtained by incubating the resin (compound 4) with 1.5 equivalents of CTPA-NHS-ester (4-[(1,4,8,11-tetraazacyclotetradec-1-yl)-methyl] benzoic acid) and 10 equivalents of DIPEA in 500 µl of DMF. The compound was purified and the final product was analyzed by HPLC as described above (see section I.). HPLC-retention time: 1.68 min; ESI-MS (m/z): [M+H]⁺ (calculated C₃₀H₅₀N₇O₈): 636.37 (636.36) 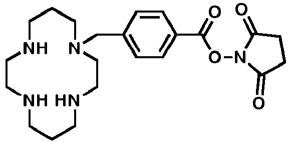 Chemical structure of the chelator CTPA-NHS-ester, the compound used in the synthesis of CA001, CA002 and CA003.

### Specification for CA002

The product was obtained by incubating the resin (compound **5**) with 1.5 equivalents of CTPA-NHS-ester (4-[(1,4,8,11-tetraazacyclotetradec-1-yl)-methyl] benzoic acid) and 10 equivalents of diisopropylamine (DIPEA) in 500 µl of DMF. The compound was purified and the final product was analyzed by HPLC as described above (see section I.). HPLC-retention time: 2.39 min; ESI-MS (m/z): [M+H]⁺ (calculated C₄₃H₆₁N₈O₉): 833.42 (833.45)

### Specification for CA003

The product was obtained by incubating the resin (compound **6**) with 1.5 equivalents of CTPA-NHS-ester (4-[(1,4,8,11-tetraazacyclotetradec-1-yl)-methyl] benzoic acid) and 10 equivalents of DIPEA in 500 µl of dimethylformamide (DMF). The compound was purified and the final product was analyzed by HPLC as described above (see section I.). HPLC-retention time: 2.50 min; ESI-MS (m/z): [M+H]⁺ (calculated C₅₁H₇₄N₉O₁₀): 972.52 (972.55)

### Specification for CA005

The product was obtained by incubating the resin (compound **5**) with 1.5 equivalents of Cross bridged-TE2A chelator, 0.98 × n_{chelator} HBTU and 10 equivalents of DIPEA in 500 µl of DMF. The compound was purified and the final product was analyzed by HPLC as described above (see section I.). HPLC-retention time: 2.38 min; ESI-MS (m/z): [M+H]⁺ (calculated C₄₄H₆₄N₈O₁₃): 913.45 (913.47) 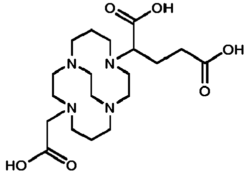 Chemical structure of the chelator chelator 8-carboxymethyl-cross bridged-TE2A, the compound used in the synthesis of CA005, and CA006.

### Specification for CA006

The product was obtained by incubating the resin (compound 6) with 1.5 equivalents of Cross bridged-TE2A chelator, 0.98 × n_{chelator} HBTU and 10 equivalents of DIPEA in 500 µl of DMF. The compound was purified and the final product was analyzed by HPLC as described above (see section I.). HPLC-retention time: 2.55 min; ESI-MS (m/z): [M+H]⁺ (calculated C₅₂H₇₈N₉O₁₄): 1052.62

### (1052.56)

### Specification for CA022

The product was obtained by incubating the resin (compound 6) with 1.5 equivalents of cross bridged-CTPA chelator and 10 equivalents of DIPEA in 500 µl of DMF. The compound was purified and the final product was analyzed by HPLC as described above (see section I.). HPLC-retention time: 2.72 min; ESI-MS (m/z): [M+H]⁺ (calculated C₅₃H₇₆N₉O₁₀): 998.56 (998.57) 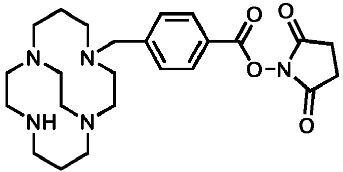 Chemical structure of the chelator Cross-bridged-CTPA, the compound used in the synthesis of CA022

### Specification CA023

The product was obtained by incubating the resin (compound 6) with 1.5 equivalents of 8-carboxymethyl-CTPA chelator and 10 equivalents of DIPEA in 500 µl of DMF. The compound was purified and the final product was analyzed by HPLC as described above (see section I.). HPLC-retention time: 2.54 min; ESI-MS (m/z): [M+H]⁺ (calculated C₅₃H₇₆N₉O₁₂): 1030.55 (1030.56) 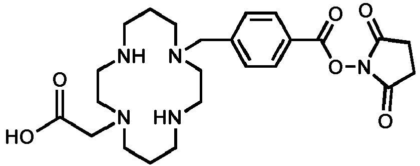 Chemical structure of the chelator 8-carboxymethyl-CTPA, the compound used in the synthesis of CA023.

### Specification for CA024

The product was obtained by incubating the resin (compound 6) with 1.5 equivalents of 8-carboxymethyl-cross bridged-CTPA chelator and 10 equivalents of DIPEA in 500 µl of DMF. The compound was purified and the final product was analyzed by HPLC as described above (see section I.). HPLC-retention time: 2.60 min; ESI-MS (m/z): [M+H]⁺ (calculated C₅₅H₇₈N₉O₁₂): 1056.56 (1056.57) 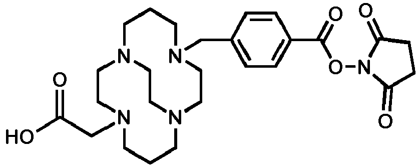 Chemical structure of the chelator 8-carboxymethyl-cross bridged-CTPA, the compound used in the synthesis of CA024.

### Specification for CA025

The product was obtained by incubating the resin (compound 6) with 1.5 equivalents of 8,11-*bis*(carboxymethyl)-CTPA chelator [CPTA = 4-[(1,4,8,11-tetraazacyclotetradec-1-yl)methyl]benzoic acid] and 10 equivalents of DIPEA in 500 µl of DMF. The compound was purified and the final product was analyzed by HPLC as described above (see section I.). HPLC-retention time: 2.60 min; ESI-MS (m/z): [M+H]⁺ (calculated C₅₅H₇₈N₉O₁₄): 1088.55 (1088.56) 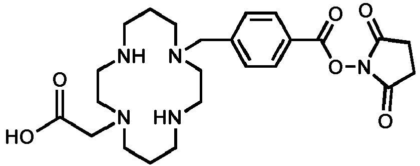 Chemical structure of the chelator 8,11- bis(carboxymethyl)-CTP A, the compound used in the synthesis of CA025.

### Specification for CA026

The product was obtained by incubating the resin (compound 6) with 1.5 equivalents of 8,11-*bis*(carboxymethyl)-CTPA chelator and 10 equivalents of DIPEA in 500 µl of DMF. The compound was purified and the final product was analyzed by HPLC as described above (see section I.). HPLC-retention time: 2.53 min; ESI-MS (m/z): [M+H]⁺ (calculated C₅₇H₈₀N₉O₁₆): 1146.56 (1146.57) 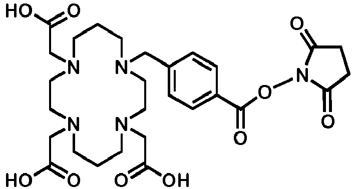 Chemical structure of the chelator 4, 8,1 1-*tris*(carboxymethyl)-CTPA, the compound used in the synthesis of CA026.

### III. PSMA Ligands for Alpha Therapy with Lead-Isotopes (²⁰³Pb/²¹²Pb)

### Specification for CA007

The product was obtained by incubating the resin (compound 5) with 1.5 equivalents ofp-SCN-Bn-TCMC chelator [TCMC = 1,4,7,10-tetraaza-1,4,7,10-tetra(2-carbamoylmethyl)cyclododecane] and 10 equivalents of DIPEA in 500 µl of DMF. The compound was purified and the final product was analyzed by HPLC as described above (see section I.). HPLC-retention time: 2.41 min; ESI-MS (m/z): [M+H]⁺ (calculated C₄₉H₇₀N₁₃O₁₂S): 1064.49 (1064.50) 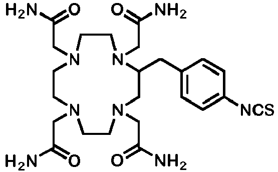 Chemical structure of the chelator *p*-SCN-Bn-TCMC, the compound used in the synthesis of CA007, and CA008, CA009.

### Specification for CA009

The product was obtained by incubating the resin (compound 6) with 1.5 equivalents ofp-SCN-Bn-TCMC chelator and 10 equivalents of DIPEA in 500 µl of DMF. The compound was purified and the final product was analyzed by HPLC as described above (see section I.). ESI-MS (m/z): [M+H]⁺ (calculated C₅₇H₈₃N₁₄O₁₃S): 1203.59 (1203.60)

### Specification for CA011

The product was obtained by incubating the resin (compound 5) with 1.5 equivalents of 2-(4,7,10-tris(2-amino-2-oxoethyl)-1,4,7,10-tetraazacyclododecan-1-yl)acetic acid, the monocarboxylate derivative of the chelator 1,4,7,10-tetraazacyclododecane-1,4,7,10-tetraacetamide (DO3AM), 0.98 × n_{chelator} HBTU and 10 equivalents of DIPEA in 500 µl of DMF. The compound was purified and the final product was analyzed by HPLC as described above (see section I.). HPLC-retention time: 2.07 min; ESI-MS (m/z): [M+H]⁺ (calculated C₄₁H₆₂N₁₁O₁₂): 900.45 (900.46) 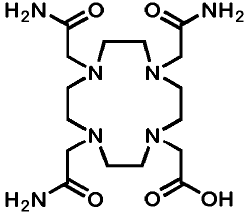 Chemical structure of the chelator 2-(4,7,10-tris(2-amino-2-oxoethyl)-1,4,7,10-tetraazacyclododecan-1 -yl)acetic acid, the monocarboxylate derivative of the chelator 1,4,7,10-tetraazacyclododecane-1,4,7,10-tetraacetamide (DO3AM), the compound used in the synthesis of CA010, CA011 and CA012.

### Specification for CA012

The product was obtained by incubating the resin (compound 6) with 1.5 equivalents of DO3AM chelator, 0.98 × n_{chelator} HBTU and 10 equivalents of DIPEA in 500 µl of DMF. The compound was purified and the final product was analyzed by HPLC as described above (see section I.). HPLC-retention time: 2.21 min; [M+H]⁺ (calculated C₄₉H₇₅N₁₂O₁₃): 1039.54 (1039.56)

### IV. Chelator Spacer Moieties Enhancing the Pharmacokinetic Properties of PSMA-617

### Specification for CA027

The product was obtained by incubating the resin (compound 4) with 1.5 equivalents ofp-NHS-Bn-DOTA chelator and 10 equivalents of DIPEA in 500 µl of DMF. The compound was purified and the final product was analyzed by HPLC as described above (see section I.). HPLC-retention time: 1.47 min; [M+H]⁺ (calculated C₃₄H₅₂N₇O₁₄): 782.33 (782.36) 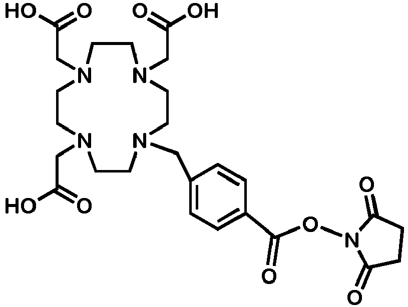 Chemical structure of *p*-NHS ester-Bn-DOTA, the chelator used for the synthesis of CA027 and CA028

### Specification for CA028

The product was obtained by incubating the resin (compound 6) with 1.5 equivalents ofp-NHS-Bn-DOTA chelator and 10 equivalents of DIPEA in 500 µl of DMF. The compound was purified and the final product was analyzed by HPLC as described above (see section I.). HPLC-retention time: 2.36 min; [M+H]⁺ (calculated C₅₅H₇₆N₉O₁₆): 1119.53 (1118.54)

### Specification for CA029

The product was obtained by incubating the resin (compound 6) with 1.5 equivalents ofp-SCN-Bn-DOTA chelator and 10 equivalents of DIPEA in 500 µl of DMF. The compound was purified and the final product was analyzed by HPLC as described above (see section I.). HPLC-retention time: 2.49 min; [M+H]⁺ (calculated C₅₇H₇₉N₁₀O₁₇S): 1207.52 (1207.53) 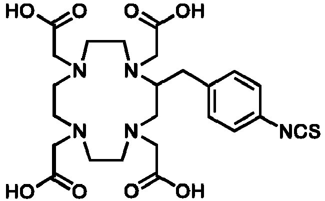 Chemical structure of the chelator *p*-SCN-Bn-DOTA, the compound used in the synthesis of CA029.

### Specification for CA030

The product was obtained by incubating the resin (compound 6) with 1.5 equivalents of p-NCS-benzyl-DOTA-GA chelator and 10 equivalents of DIPEA in 500 µl of DMF. The compound was purified and the final product was analyzed by HPLC as described above (see section I.). HPLC-retention time: 2.50 min; [M+H]⁺ (calculated C₆₀H₈₄N₁₁O₁₈S): 1278.56 (1278.57) 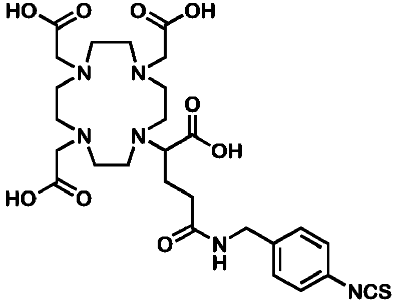 Chemical structure of the chelator p-NCS-benzyl-DOTA-GA, the compound used in the synthesis of CA030.

### V Synthesis of radiolabeled complexes:

### V.1 Radiochemical Synthesis of the ⁶⁴Cu-PSMA-Derivatives

The conjugates (1 mM in water, 5 µl, 5 nmol) were added to a mixture of 400 µl sodium acetate buffer (0.4 M in water, pH 5.0), 10 µl ascorbic acid (20% in water) and 282 µl [⁶⁴Cu]CuCl₂ in 0.1 M HCl (200 MBq). The mixture was heated at 95 °C for 5 min. The labeling was controlled by radio-HPLC (0-100% MeCN in 5 min, Monolith column), with a flow rate of 2 mL/min and retention time of 2.3 min.

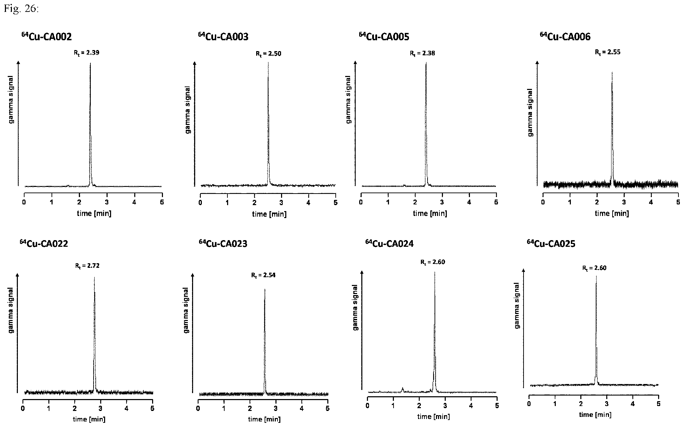

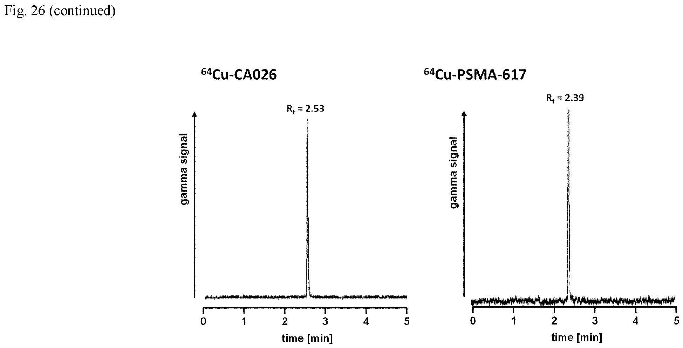

The labeling led to radiolabeling yields > 98% within 10 min (as illustrated by the radiochromatograms in Figure 26). The specific activity of e.g. ⁶⁴Cu-PSMA-CA003 was approximately 40 MBq/nmol. The same protocol was used for the labeling with ⁶⁷Cu.

### V.2 Radiochemical Synthesis of the ^{203/212}Pb-PSMA-Ligands

Eighty nmol of the conjugates (1 mM in water, 80 µl, 80 nmol) were added to 400 µl sodium acetate buffer (0.4 M in water, pH 5.0), 10 µl ascorbic acid (20% in water) and 140 µl ²⁰³Pb-chloride solution in 0.04 M HCl, with specific activity approx. 102.6 TBq/g (Lantheus Medical Imaging, USA). The mixture was then heated at 95 °C for 5 min. The labeling was controlled by radio-HPLC.

### V.3 Radiochemical Synthesis of the ⁶⁸Ga-PSMA-CA028 (CA027, CA029, CA030)

⁶⁸Ga was eluted from a ⁶⁸Ge/Ga generator (iThemba LABS, South Africa).The conjugate (1 mM in DMSO, 20 µl, 20 nmol) was added to a mixture of 320 µl sodium acetate buffer (0.4 M in water, pH 4-5), 10 µl ascorbic acid (20% in water) and 400 MBq ⁶⁸Ga in 0.6 M HCl. The mixture was heated at 95 °C for 5 min. The labeling was controlled by radio-HPLC (0-100% MeCN in 5 min, Monolith column), with a flow rate of 2 mL/min and retention time of 2.4 min.

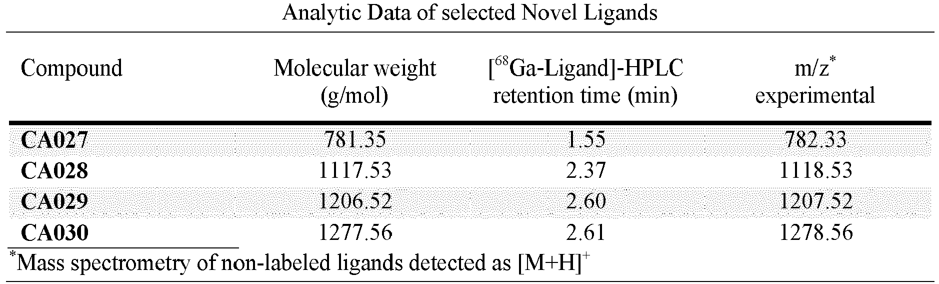

### V.4 Radiochemical Synthesis of the ¹⁷⁷Lu-PSMA-CA028 (CA027, CA029, CA030)

For ¹⁷⁷Lu labeling, approx. 20 MBq was mixed with 200 µl of 0.4 M sodium acetate buffer containing Chelex (pH = 5). 2 µl of a 1 mM solution of the compound in 10% DMSO in water, 2 µl of a saturated solution of ascorbic acid and 40 µl of the solution [¹⁷⁷Lu]LuCl₃ were mixed and heated to 95 °C for 10 min. The labelling was checked by radio-HPLC (0 - 100% ACN in water within 5 min, Monolith column).

### VI. Preclinical evaluation

*In vitro* and *in vivo* experiments were performed using the PSMA-positive C4-2 cell line, a subline of the LNCaP *(lymph node carcinoma of the prostate)* cell line (CRL-33 14; American Type Culture Collection). C4-2 cells were cultivated in RPMI 1640 (PAN Biotech) medium supplemented with 10% fetal calf serum and stable glutamine (PAN Biotech). Cells were grown at 37 °C and incubated with humidified air equilibrated with 5% CO₂.

### VI.1 In Vitro

### VI.1.1 Competitive binding assay and internalization ratio

A MultiScreen_{HTS}-DV filter plate was incubated at room temperature with 100 µl PBS containing 1% BSA per well for 30 min. After removal of the PBSBSA solution 1 × 10⁵ C4-2 cells were added Opti-MEM to each well. The inhibitory potency of the synthesized compounds was determined using 0.75 nM of ⁶⁸Ga-labeled PSMA-HBED-CC dimer (⁶⁸Ga-PSMA-10) (Schäfer M, Bauder-Wiist U, Leotta K, et al. A dimerized urea-based inhibitor of the prostate-specific membrane antigen for 68Ga-PET imaging of prostate cancer. EJNMMI research. 2012;2:23-23.*)* as a standard. All non-labeled compounds were dissolved in Opti-MEM at a volume of 300 µl with the following concentrations: 0, 0.5, 1, 2.5, 5, 10, 25, 50, 100, 500, 1000 and 5000 nM. Subsequently, 3 µl of the radiolabeled compound was added. 50 µl of this mixture was taken to obtain a 0.75 nM concentration of the radiolabeled ligand. After 45 min incubation at 37 °C, the cells were washed twice with PBS on a multiscreen vacuum manifold (Millipore, Billerica, MA) and the cell-bound radioactivity was measured with a gamma counter (Packard Cobra II, GMI, Minnesota, USA).. The inhibitory potency was determined using ⁶⁸Ga-labeled PSMA-HBED-CC dimer (i.e. PSMA-11) as reference. The Ki was calculated using a nonlinear regression algorithm (Graph Pad Prism 5.01 software). The experiments were performed in quadruplicate.

For determination of the specific internalization ratio, 24-well plates were incubated for 20 min with 0.1% poly-L-lysine in PBS at room temperature and washed once with PBS. In the next step, 1 ml RPMI medium containing 1 × 10⁵ C4-2 cells was added to each well and incubated overnight. The conditions during the experiment for each compound were: incubation at 37 °C or 4 °C with or without receptor blocking via 2-(phosphonomethyl)pentanedioic acid (2-PMPA; Axxora) at a final concentration of 500 µM. Afterwards, the cells were incubated with 250 µl of a 30 nM solution of the labeled compounds. The plates were either incubated for 45 min in a water bath at 37 °C or on ice at 4 °C. Subsequently, the cells were washed 3 times with 1 mL of ice-cold PBS and incubated with 0.5 ml glycine (50 mM in HCl pH 2.8) for 5 min. After an additional washing step with 1 mL of ice-cold PBS, the cells were lysed with 0.5 ml of 0.3 M NaOH, collected and the radioactivity was measured with a gamma-counter for 1 min. The specific cellular uptake was determined as percentage of initially added radioactivity bound to 10⁶ cells (%IA/10⁶ cells) by subtraction of the respective uptake under blocking conditions. All experiments were performed in triplicate.

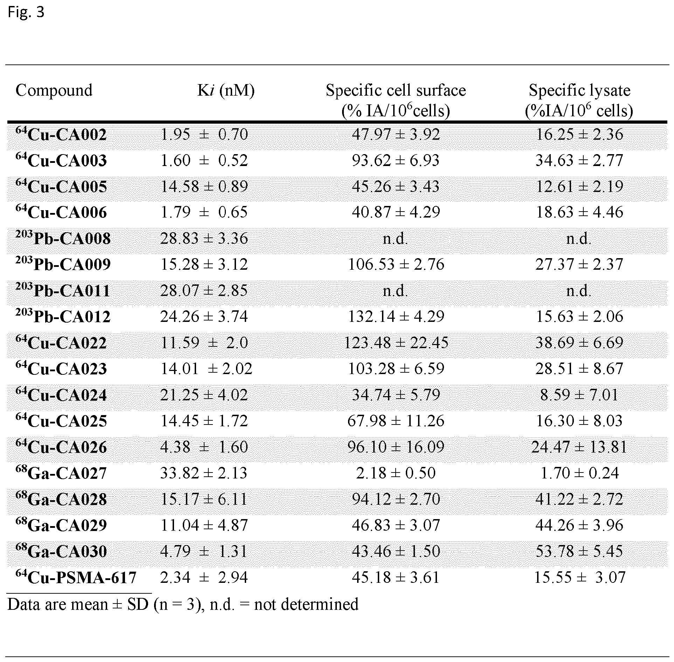

The results are given in Figure 3. The *K*ᵢ determination showed nanomolar binding affinities of the synthesized ligands to PSMA.

For the ²⁰³Pb labeled compounds, compounds CA009 and CA012 revealed the highest affinity to inhibit PSMA. Moreover, the ²⁰³Pb-labeled compounds CA009 and CA012 showed high rates of specific internalization in the PSMA positive cell line. The compounds ²⁰³Pb-CA009 and ²⁰³Pb-CA012 showed an internalization ratio up to 28.36 ± 2.23 and for 7.33 ± 1.26 injected activity/10⁶ C4-2 cells (n = 3).

As shown in the table in Figure 3, for the Cu-labeled ligands, e.g,, CA003 revealed a particularly high affinity to PSMA, followed by CA006, CA002 and CA026. Moreover, the ⁶⁴Cu-labeled compounds showed specific binding to C4-2 cells. 34.63 ± 2.77% of ⁶⁴Cu-CA003, 18.63 ± 4.46% of ⁶⁴Cu-CA005 and 38.7 ± 6.69% of ⁶⁴Cu-CA022 were internalized (n = 3). 10⁶ C4-2 cells were used for these experiments.

Further, the results of the *K*ᵢ determination for e.g. the Ga-labeled ligands revealed e.g., nanomolar binding affinities of the synthesized ligands to PSMA. As shown in Figure 3, among all new compounds CA030 showed particularly advantageous properties.

### VI.1.2 Serum Stability

### VI.1.2.1 Serum Stability of²⁰³Pb labeled compounds

The stability of the radiolabeled compounds was determined by incubation in 300 µl of human serum at 37 °C after 1 h, 2 h, 3 h, 6 h, 24 h, 48 h and 72 hours. The serum was precipitated by addition of 2 parts acetonitrile. Subsequently, the samples were vortexed and centrifuged for 5 min at 13,000 rpm (2 times) and the supernatant was analyzed by radio-HPLC (0-100% MeCN in 5 min, Monolith column).

All compounds showed a high stability in human serum for at least 48 h. CA011 and CA012 showed a particularly advantageous stability and were stable for at least 72 h.

### VI.1.2.2 Serum Stability of ⁶⁸Ga, ¹⁷⁷Lu and ⁶⁴Cu labeled compounds

After radiolabeling of the compounds the serum stability was determined by iTLC and HPLC analysis. 50 µL (20 MBq) of the labeled ligands was added to 200 µL of human serum (H4522; Sigma-Aldrich, Germany) and incubated at 37 °C for different time-points (0, 2 h, 24 h, 48 h and 72 h). 0.5 × 5 cm strips of iTLC-SG-glass microfiber chromatography paper (Folsom, California USA) were used. 0.5 µL of the radiolabeled compound in serum was applied to each strip at 1 cm from the bottom (origin) and the solvent (sodium citrate buffer (0.5 M, pH = 5.0) in case of ¹⁷⁷ Lu ligands, (1% Na-EDTA, pH = 4) in case of Cu labeled ligands) front was allowed to rise to 5 cm from the bottom. Finally, each strip was cut in 8 pieces; each piece was measured in a gamma-counter. For HPLC analysis an equal volume of ACN was added to the samples to precipitate the serum proteins. Subsequently, the samples were centrifuged for 10 min at 13,000 rpm and the pellet and supernatant were separated and the relative activity was measured. The results are expressed as percent. In addition, an aliquot of the supernatant was analyzed by radio-HPLC (0-100% ACN in 5 min, Monolith column), with a flow rate of 2 mL/min.

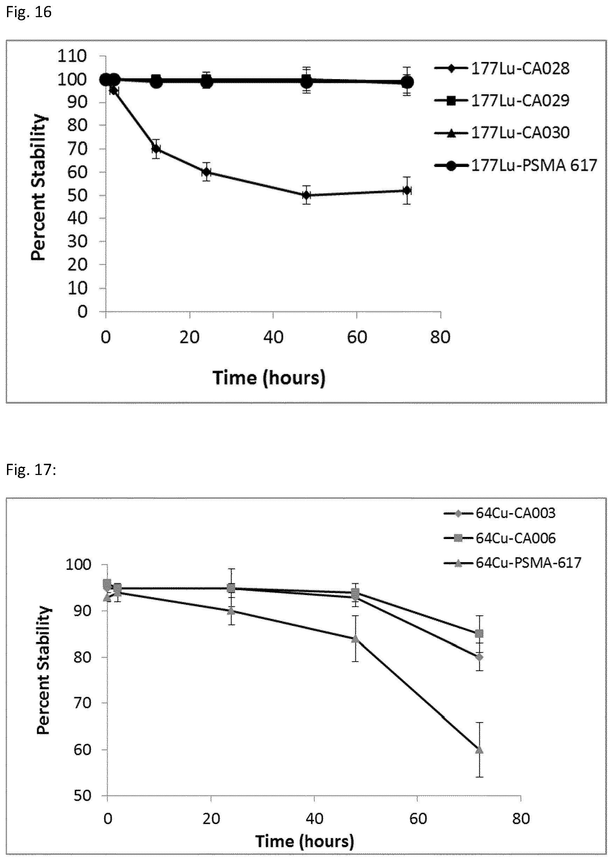

The results of the stability testings of the compounds radiolabeled with ⁶⁸Ga and ¹⁷⁷Lu is shown in Fig. 16) As indicated by HPLC and radio TLC, the ⁶⁸Ga-labeled compounds did not show degradation after incubation for 2 h in human serum. ¹⁷⁷Lu-CA028 showed 40% of free ¹⁷⁷Lu after incubation for 24 h whereas CA029, CA030 and the reference compound PSMA-617 revealed no free activity at this time point (Figure 16).

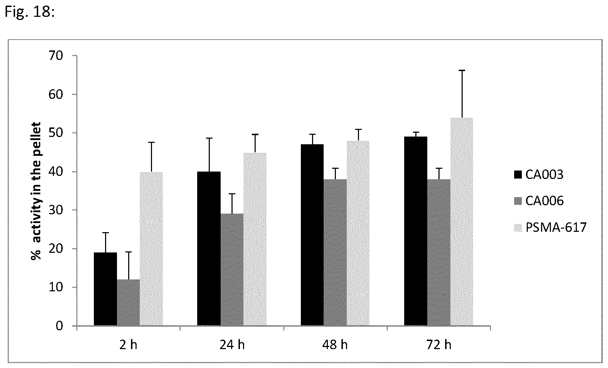

The results of the stability testings of ⁶⁴Cu-labeled compounds is shown in Figure 17 and Figure 18. Until 2 h incubation, ITLC showed that all compounds dissociate only to a degree of less than 2 ± 0.6%. After 24 h incubation only 6 ± 4% of ⁶⁴Cu-CA003 and 3 ± 1% of ⁶⁴Cu-CA006 were found to be dissociated. In contrast ⁶⁴Cu-PSMA-617 showed 13 ± 3% of free ⁶⁴Cu activity. Long term stability examination (72 h) showed that ⁶⁴Cu-CA006 (8 ± 4%) possesses stability comparable to ⁶⁴Cu-CA003 (11 ± 3%). At this point in time 18 ± 6% of ⁶⁴Cu-PSMA-617 was found to be dissociated. After incubation for 2 h, activity measurement in the pellet (Figure 18) revealed that 19.0 ± 5.2% of⁶⁴Cu-CA003, 12 ± 7.2% of ⁶⁴Cu-CA006 and 40 ± 7.5% of ⁶⁴Cu-PSMA-617 was precipitated with the protein fraction. The percentage of the activity in the pellet increased over time. The highest amount of activity was measured in the pellet of ⁶⁴Cu-PSMA-617 followed by ⁶⁴Cu-CA003, and less activity was found to be precipitated for ⁶⁴Cu-CA006

### VI.2 In vivo experiments

The *in vivo* experiments were carried out in accordance with the laws of the German Federal Republic. For PET imaging and biodistribution studies, male nude mice (Balb/c nu/nu mice) (19-23 g) were obtained from Charles River at 4-5 weeks of age and kept under specific-pathogen-free condition for 1 week prior to the study. The mice were housed with a 12-hour/12-hour light/dark cycle and had free access to water and food. The mice were anesthetized with 2% sevoflurane and inoculated subcutaneously on the right trunk with 5 × 10⁷ C4-2 cells in 50% Matrigel in Opti-MEM I (1×) medium. Organ distribution studies were performed, when the size of the tumor was approximately 1 cm³.

### VI.2.1 Lead-labeled compounds:

### VI.2.1 a Scintigraphic Imaging and Biodistribution

For small-animal imaging the mice were anesthetized with 2% sevoflurane. 0.1 nmol (1.0 MBq) of the ²⁰³Pb-ligand was injected into the tail vein. Serial planar scans were performed using a Gamma Imager SCT (Biospace Lab, Paris, France) with a parallel collimator (35 mm/1.8 mm/0.2 mm) after 10 min, 1 h, 4 h, 24 h. and 72 h. Based on the imaging results, compound CA012 was chosen for biodistribution studies. Experiments were performed in triplicate.

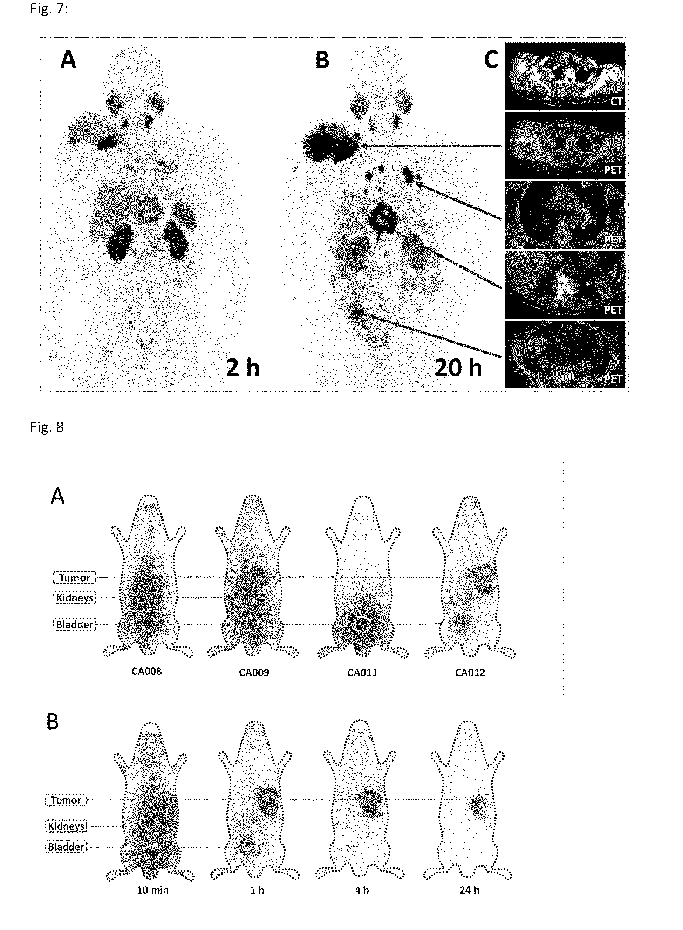

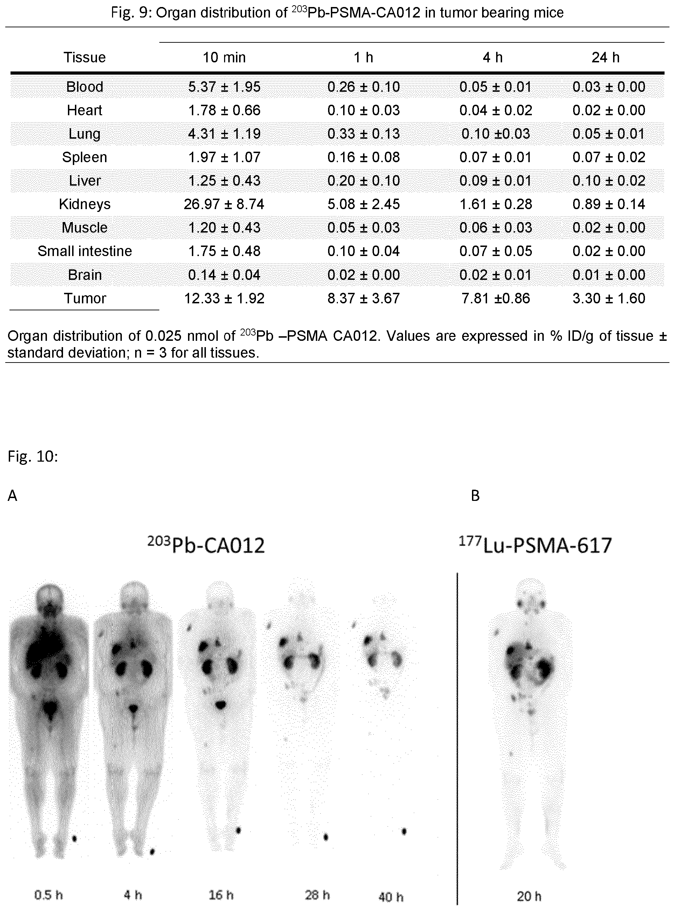

The results are given in Fig. 8 and 9.

### VI.2.2 ⁶⁴Cu-labeled compounds:

### VI.2.2 a) Stability in the blood and in vivo fate of ⁶⁴Cu-chloride and ⁶⁴Cu-CA003

The stability of ⁶⁴Cu-labeled CA003 *in vivo* was determined by ITLC and HPLC. Male BALB/c nude mice without tumor (n = 3) were injected via the tail vein with ⁶⁴Cu-CA003 (3.6 MBq; 0.26 nmol, dissolved in a total volume of approximately 100 µl of 0.9% saline ) and 800 µl blood was harvested 10 min post injection. The blood sample was centrifuged for 10 min at 13,000 rpm. Subsequently, the pellet and supernatant were separated and the relative activity was determined. ITLC was performed in order to assess the stability of the radiolabeled compound in the blood as described above. Furthermore, an aliquot of the supernatant was analyzed by radio-HPLC (0-100% ACN in 5 min, Monolith column), with a flow rate of 2 mL/min after addition an equal volume of ACN and removal of the proteins by centrifugation

The metabolization *in vivo* was studied by radio-HPLC analysis. Female Swiss mice (n = 3) without tumor were injected via the tail vein with ⁶⁴Cu-chloride (10 MBq in approximately 100 µL of 0.9% saline) or ⁶⁴Cu-CA003 (9 MBq, 0.30 nmol in approximately 100 µL of 0.9% saline). PET imaging was performed 10 min post injection and subsequently blood, the liver and the kidneys were harvested. The tissues were rinsed with precooled saline, blotted dry and treated with 2 mL of 0.1 M NH₄OAc/EtOH (35:65). The tissues were homogenized using an Ultra-Turrax T8 (IKA Labortechnik, Germany). The samples were centrifuged for 10 min at 13,000 rpm (4 °C). Subsequently, the pellet and supernatant were separated and the relative activity was measured. The results are expressed as percent. Additionally, an aliquot of supernatant was prepared for HPLC measurement by precipitation of the proteins with ACN as described above. The sample was analyzed by radio-HPLC (0-100% ACN in 5 min, Monolith column), with a flow rate of 2 mL/min. Fractions were collected every ten seconds over the whole course of the chromatography and the relative activity of the samples was measured in a gamma-counter to reconstruct a chromatogram.

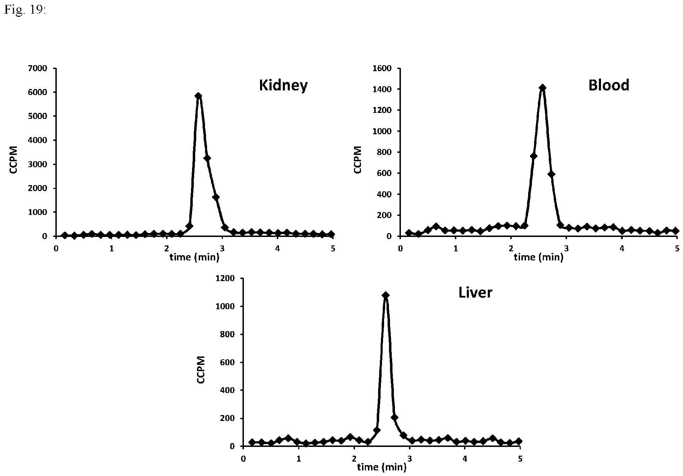

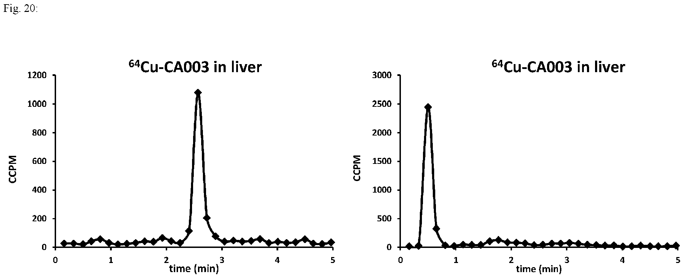

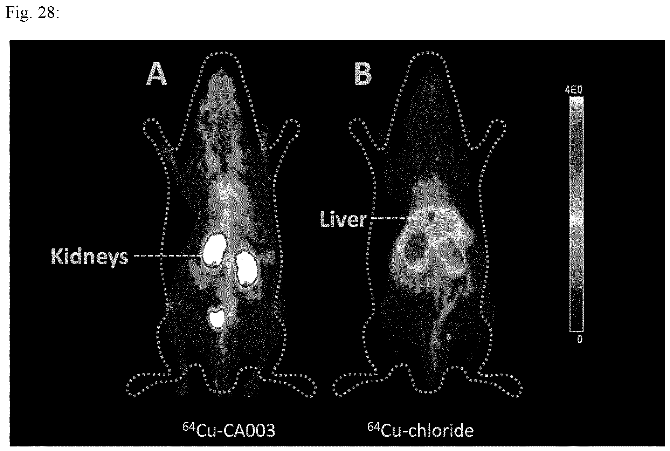

ITLC results of the blood stability showed that ⁶⁴Cu-CA003 undergo 3% of ⁶⁴Cu dissociation or 97 ± 2.3% of the intact tracer (see Fig. 19). Radio HPLC chromatograms likewise confirms the integrity of the copper complex (see Fig. 20). *In vivo* fate of ⁶⁴Cu-chloride or ⁶⁴Cu-CA003 studies was conducted and PET imaging of ⁶⁴Cu-chloride and ⁶⁴Cu-CA003 showed different pharmacokinetics (Figure S21). Maximum intensity projection PET imaging of ⁶⁴Cu-chloride indicated, lower blood circulation (1.3), high liver (2.7) and kidney uptake (3.4). Whereas ⁶⁴Cu-CA003 demonstrated longer blood circulation (2.3), higher kidney (5.7) uptake and lower liver accumulation (1.0) than ⁶⁴Cu-chloride (Figure 28). The integrity of the ⁶⁴Cu-CA003 was proven by radio HPLC chromatograms of tissue extracts of kidney, blood and liver (Figure 19). The chromatogram for ⁶⁴Cu-CA003 showed different retention time of the tracer than free copper, ⁶⁴Cu-chloride (Figure 20).

### VI.2.2 b) Organ Distribution Experiments (⁶⁴Cu Ligands) and small animal PET

Based on the results of the PET imaging, CA003, and CA023, were chosen for a biodistribution analysis using the C4-2 tumor bearing mice. Experiments were performed in triplicate. 0.025 nmol of the ⁶⁴Cu-labeled compound (1 MBq per mouse in approximately 100 µL of 0.9% saline) was administered by tail vein injection. At the time points: 10 min, 1 h, 4 h, 24 h and 72 h the organs were dissected and weighed and the activity was measured using a γ-counter (Packard Cobra Auto-gamma). The percentage of the injected dose per gram (% ID/g) was calculated. (See Fig, 6A, 6B)

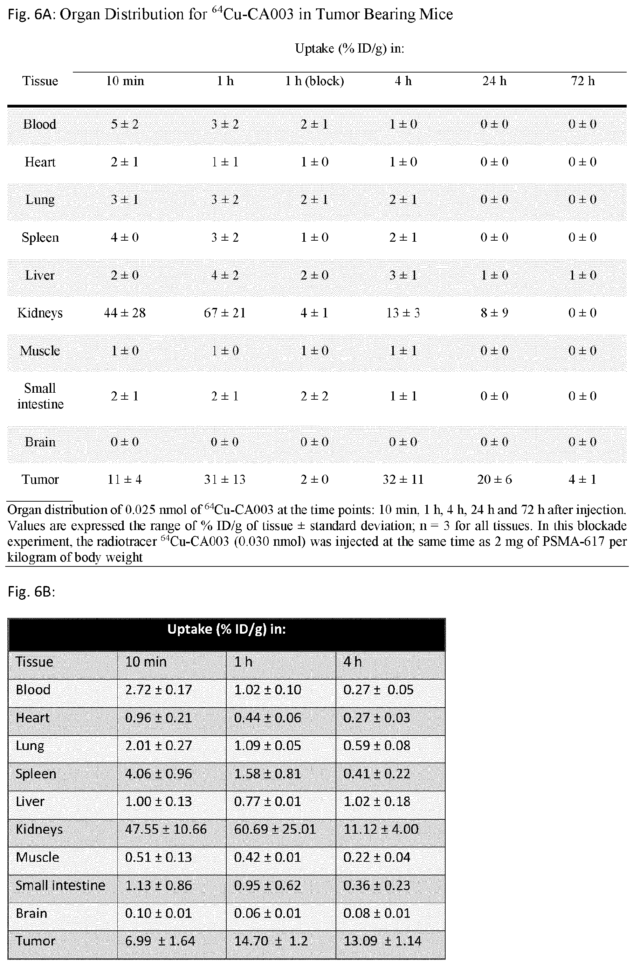

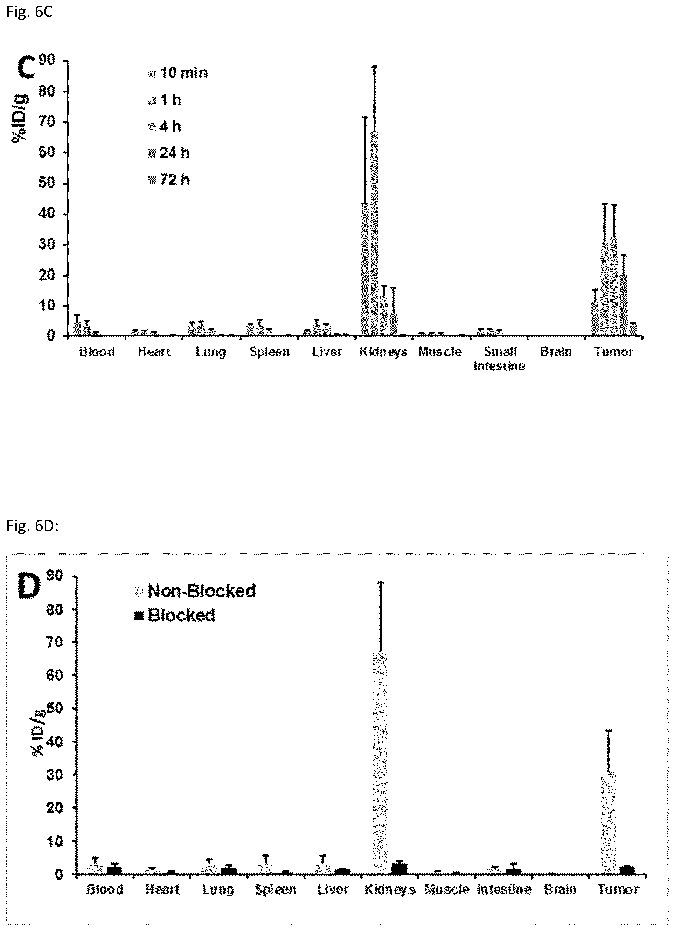

In addition to this, the experiment with simultaneous administration of PSMA-617 to block PSMA binding at 1 h (n = 3) is represented (Figure 6C and 6D).

For small-animal PET imaging with various ⁶⁴Cu-labeled PSMA ligands, 0.2 nmol, 10 MBq of approximately 100 µL in 0.9% saline the radiolabeled compound were injected into a C4-2 tumor bearing mouse. The dynamic PET was recorded in a small animal PET scanner (Siemens Inveon D-PET, Malvern, PA USA). The SUV values were obtained from conventional (non-dynamic) PET images. The formula for the SUV was: $SUV = \frac{activity in ROI \left(\frac{Bq}{ml}\right) \times animal weight \left(g\right)}{injected dose \left(Bq\right)}$

The volumes-of-interest (VOIs) were obtained by manual delineation of the appropriate whole tissue (heart, kidneys, bladder, tumor - with an approximate volume of 100-500 µl) - or parts of the tissue liver and muscle. The images were reconstructed based on the procedure: OSEM 3D/SP MAP with 16 subsets, 2 iterations and an image x-y size: 256, image z size: 161. The data were not modified with a post processing filter. The software used to analyze images and TACs was Inveon^{™} Acquisition Workplace (IAW) from Siemens IRW 4.1. Dynamic PET scans were performed 0-60 min post injection, and images were reconstructed in three time frames of 20 min (0-20 min, 20-40 min and 40-60 min) for visual display. For some compounds that showed long retention later time points (2 h, 4 h, 20 h, 45/48 h) were included as shown in Fig. 2 /Fig. 3 and Table S1+S2. After 1 h a static PET scan was generated. In order to compare the different radiotracers, the mean SUVs were plotted over time.

The results obtained for the biodistribution of the PSMA ligand ⁶⁴Cu-CA003 (n = 3) are shown in Figures 6A-6D. 10 min after injection 11.33 ± 4.11%ID/g tumor uptake is observed. After 4 h the amount of the tracer accumulation (32.34 ± 10.6% ID/g) in the tumor is much higher than in the kidneys (13.33 ± 3.36% ID/g). Time activity curves generated from the dynamic PET imaging, showed a tumor-to-muscle ratio of 10.5 and 3.0 fortumor-to-blood at 1 h post injection, see following table *VI.2.2 b_1*:

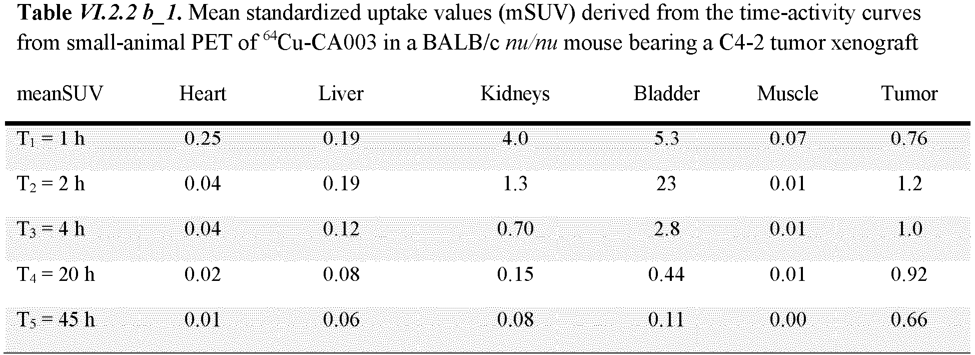

These curves demonstrated a rapid renal uptake. The organ distribution study showed that the high kidney uptake at 1 h (67.04 ± 20.89% ID/g) was largely cleared (7.48 ± 8.51% ID/g) within 24 h. In contrast the high tumor uptake value (30.83 ± 12.61% ID/g at 1 h p.i.) remained almost constant (19.99 ± 6.43% ID/g at 24 h p.i.). PET imaging confirmed the strong accumulation of the radiotracer in the tumor (Figure 4). At 1 h post injection, the amount of the radioactivity in the background organs such as kidneys decreased whereas the tumor-to-background ratio increased. 24 h after injection the PET scans demonstrated a very high tumor uptake confirming the enrichment in the tumor.

The specificity of the binding to PSMA was proven with a blockade experiment: co-injection of non labelled PSMA-617 [2 mg/kg] led to a strong decrease of the accumulation of ⁶⁴Cu-CA003 in C4-2 tumors (30.83 ± 12.61% ID/g to 2.35 ± 0.38% ID/g) and in the kidneys (67.04 ± 20.89% ID/g to 3.47 ± 0.48% ID/g) at 1 h post injection. PET imaging of ⁶⁴Cu-CA003 with excess of non-labeled PSMA (Figure 6C/6D) clearly confirmed the biodistribution results.

Time activity curves generated from the dynamic PET imaging, showed a tumor-to-muscle ratio of 10.5 and 3.0 for tumor-to-blood at 1 h post injection (Table S 1). These curves demonstrated a rapid renal uptake. The organ distribution study (Figure 6A) showed that the high kidney uptake at 1 h (67.04 ± 20.89% ID/g) was largely cleared (7.48 ± 8.51% ID/g) within 24 h. In contrast the high tumor uptake value (30.83 ± 12.61% ID/g at 1 h p.i.) remained almost constant (19.99 ± 6.43% ID/g at 24 h p.i.). PET imaging confirmed the strong accumulation of the radiotracer in the tumor (Figure 4). At 1 h post injection, the amount of the radioactivity in the background organ such as kidneys decreased whereas the tumor-to-background ratio increased. 24 h after injection the PET scans demonstrated a very high tumor uptake confirming the enrichment in the tumor. The high tumor accumulation was retained at long time points i.e. at 45 h post injection (Figure 4).

### IV.2.2 c) Comparison of ⁶⁴Cu-PSMA-CA003 with ⁶⁴Cu-PSMA-617 and ⁶⁴Cu-chloride in vivo

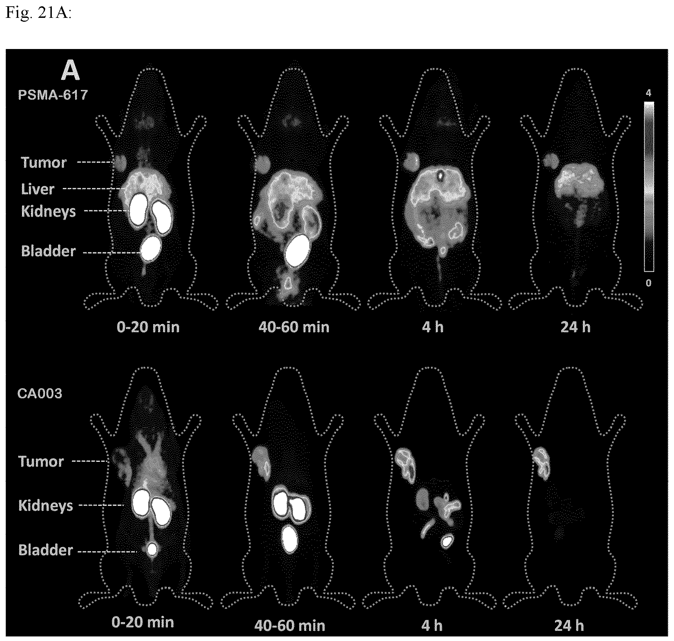

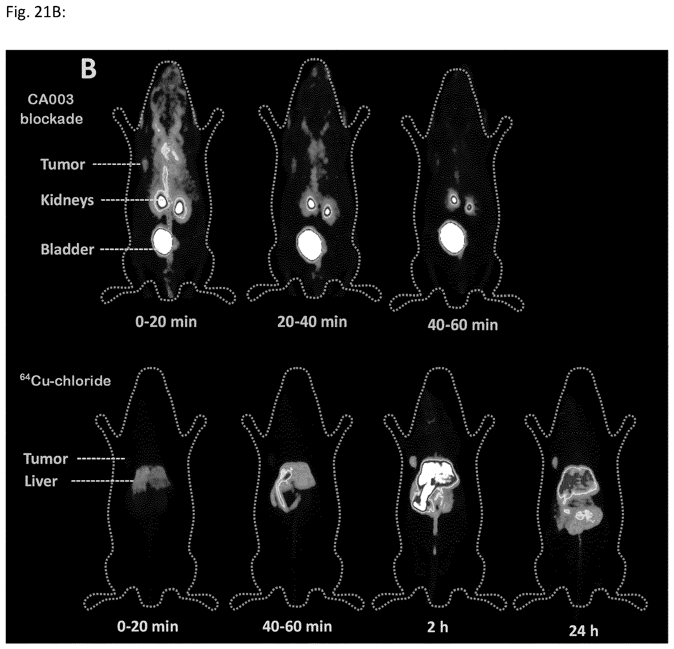

In order to prove the *in vivo* stability of the copper complexes of PSMA-CA003, ⁶⁴Cu-PSMA-CA003 was compared to ⁶⁴Cu-PSMA-617 as well as to ⁶⁴Cu-chloride (Figure 20). The compounds were studied in a small animal PET study in a C4-2 tumor xenograft. The results are shown in Figure 3. The time activity curves obtained from dynamic PET for ⁶⁴Cu-CA003 showed a high tumor-to-liver ratio (4.0) at 1 h after injection, whereas for ⁶⁴Cu-PSMA-617 the tumor-to-liver ratio was 0.37 (Figure 20/21 and Table S1/S2).

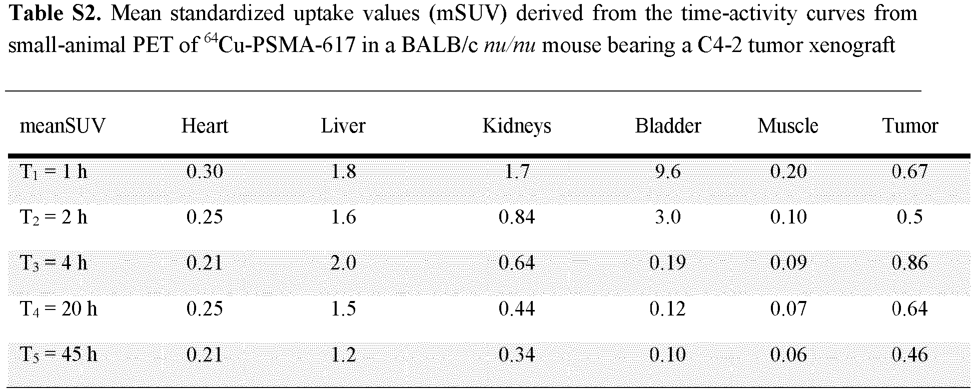

To prove that the species which is taken up into the tumor is actually ⁶⁴Cu-CA003 and not free ⁶⁴Cu, PET imaging of ⁶⁴Cu-chloride performed in C4-2 tumor bearing mice (Figure 20/21) was followed by homogenization, extraction and subsequent HPLC analysis of the respective tissue. The pharmacokinetic observed for ⁶⁴Cu-chloride is different to that of ⁶⁴Cu-CA003. PET imaging of⁶⁴Cu-chloride at maximum-intensity projections reveal an increasing tumor uptake up to 2 h after injection. In contrast to ⁶⁴Cu-CA003, ⁶⁴Cu-chloride showed a very high liver accumulation (Figure 20/21). The tumor-to-liver ratio at 2 h for ⁶⁴Cu-chloride was 0.38, whereas the tumor-to-liver ratio of ⁶⁴Cu-CA003 was 6.3.

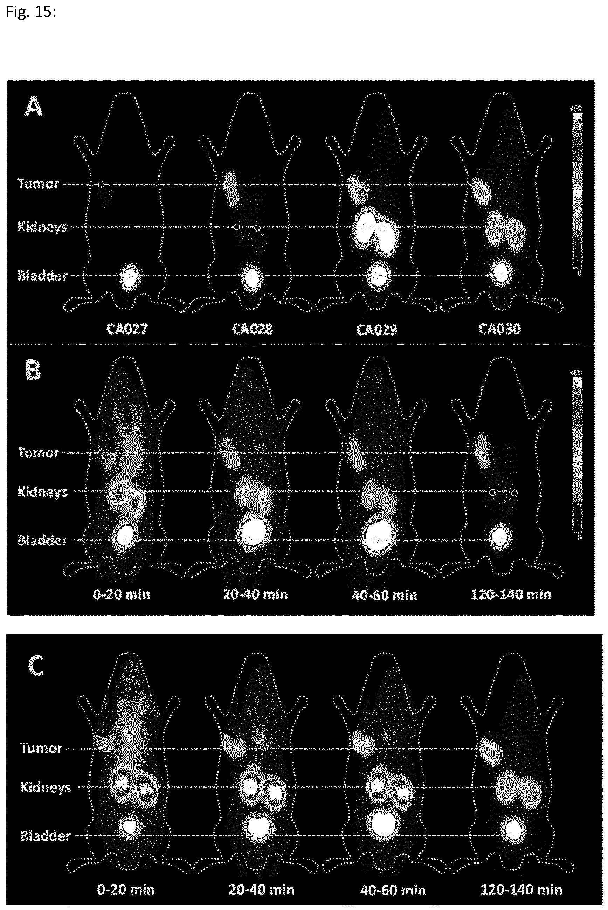

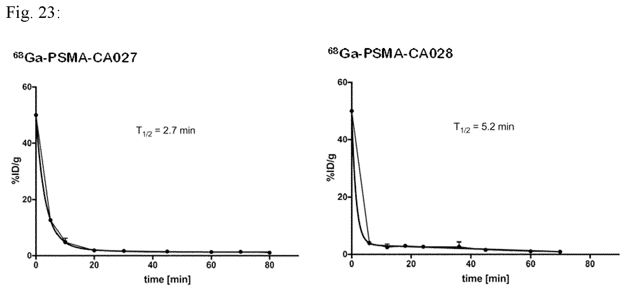

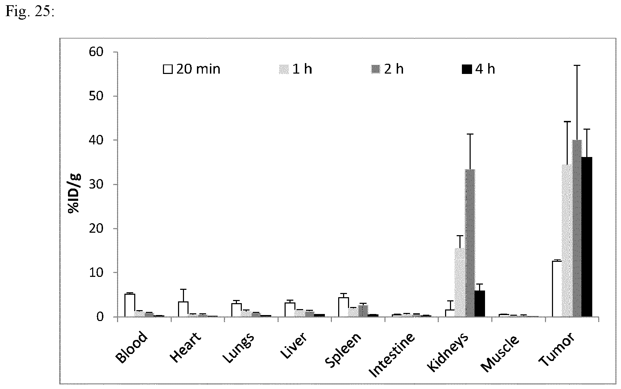

1 h post injection the time activity curves and the mean SUV body weight values generated from the dynamic PET imaging of ⁶⁸Ga-CA028 demonstrated a tumor-to-kidney ratio of 0.78. At 2 h, this ratio was increased to 3.0 (Figure 15, and Table S1.1), while the ligands ⁶⁸Ga-CA030 and ⁶⁸Ga-CA029 showed lower tumor-to-kidneys ratio of 0.52 and 0.33, respectively (Figure 3, 25 and 23).

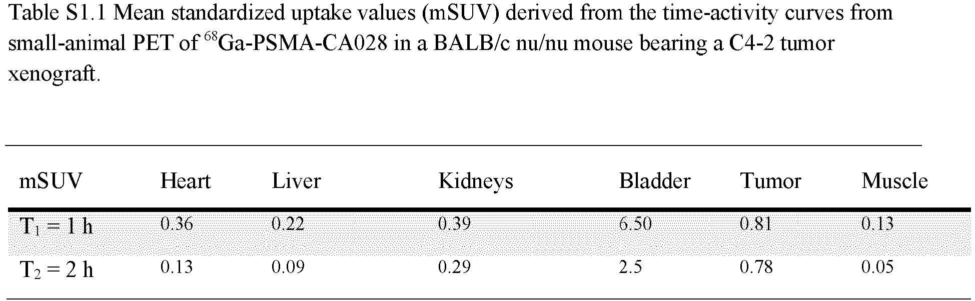

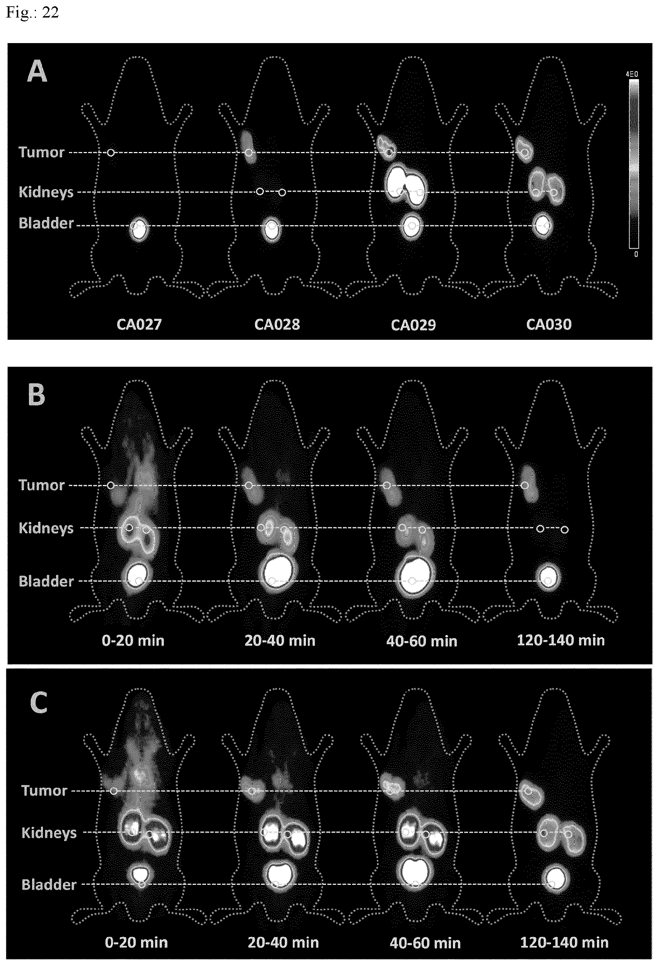

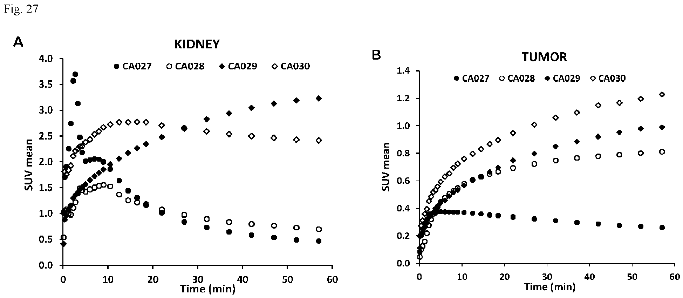

The time-activity curves revealed a fast clearance of the tracer - ⁶⁸Ga-CA028 showed a high tumor accumulation and high kidney values. In contrast, for ⁶⁸Ga-CA027 a faster clearance by the kidney at a tumor accumulation was found (Figure 22). Even though ⁶⁸Ga-CA030 demonstrated higher kidney uptake values than ⁶⁸Ga-CA028, it showed the highest tumor uptake among all compounds (Figure 15C). The small animal PET images demonstrated a very fast kidney clearance and low tumor accumulation of ⁶⁸Ga-CA027. Distinctly, the radiotracers ⁶⁸Ga-CA028, ⁶⁸Ga-CA029 and ⁶⁸Ga-CA030 showed high tumor accumulation (Figure 27B). ⁶⁸Ga-CA029 showed the highest kidney uptake, followed by ⁶⁸Ga-CA030 (Figure 27A).

### VII. In-Human Studies in a first Patient

### VII.1 PET with ⁶⁴Cu-PSMA-CA003

The PET imaging of the prostate cancer patient shown in Fig. 7 was consented by the University Hospital Heidelberg following the German laws in vigor and granted the Helsinki Declaration (permit S321/2012).

The first-in-human study was performed with 200 MBq of ⁶⁴Cu-PSMA-CA003. The first PET imaging is presented in Fig. 7 and shows imaging of a patient with high level of the prostate-specific membrane antigen. It is clearly evident, that the patient suffered from PCa in metastasized stage notably through multiple lymph node metastases, on the right shoulder the main tumor is localized.

With the new copper ligands a significant improvement of the pharmacokinetics and the tumor targeting for copper isotopes was observed. Despite the high labeling yields of PSMA-617 with ⁶⁴Cu *in vitro* (>99%), a poor *in vivo* stability with occurrence high liver uptake was observed (Cui C, Hanyu M, Hatori A, et al. Synthesis and evaluation of [(64)Cu]PSMA-617 targeted for prostate-specific membrane antigen in prostate cancer. Am J Nucl Med Mol Imaging. 2017;7:40-52).

The novel ⁶⁴Cu-labeled PSMA ligands are promising agents to target PSMA and visualize PSMA positive tumor lesions as shown in the preclinical evaluation by small-animal PET studies, organ distribution and the first-in-human application.

The imaging in a patient with the ⁶⁴Cu-PSMA-CA003 ligand demonstrated its successful translation in clinical studies.

### VII.2 Experiments with ²⁰³Pb-CA012

Two patients, with castration-resistant metastasized prostate cancer underwent planar whole-body scans (GE Hawkeye Millennium, 1" crystal, ME-collimator, 279 keV peak +/-10%, 8cm/min) at 0.4 h, 4 h, 18 h, 28 h and 42 h post-injection of 258 and 310 MBq ²⁰³Pb-CA012, respectively. Images were loaded into the QDOSE dosimetry software suite (ABX-CRO, Dresden) and coregistered. Kidneys, liver, spleen, urinary bladder, salivary glands (both left and right of parotid and submandibular glands) and several tumor lesions as well as a total-body region-of-interest (ROI) were segmented using organ-dependent percentage of maximum thresholds (15-65%) in the most suitable time-point and propagated to all other time-points performing an additional organ-based automatic rigid co-registration step. These ROIs were used to determine time-activity-curves (TAC) for each organ, tumors and total body. The first time-point (before voiding) of the uncorrected geometric mean images was used to calibrate the ROI-counts to injected activity (MBq). Red marrow TAC was calculated from venous blood (6 samples/patient) using established model assumptions [Shen, S., Meredith, R.F., Duan, J., Macey, D.J., Khazaeli, M.B., Robert, F., LoBuglio, A.F.: Improved Prediction of Myelotoxicity Using a Patient-Specific Imaging Dose Estimate for Non-Marrow-Targeting 90Y-Antibody Therapy. J Nucl Med, 43: 1245-1253, 2002. Sgouros, G. Bone Marrow Dosimetry for Radioimmunotherapy: Theoretical Considerations. J Nucl Med, 34: 689-694, 1993.].

All TACs derived from the ²⁰³Pb data were re-calculated using the replacement nuclide function of QDOSE, which automatically corrects all time-points for the physical decay of the source isotope, leaving only its biological clearance; then the physical decay of the replacement radionuclide is applied.

Bi-exponential curve fitting was applied to all organ TACs (with exception of few tumors and glands, which had to be fitted mono-exponentially). The cumulated activity was integrated assuming a linear increase from 0 to first measured time-point, numerically from first to last measured time-point using trapezoidal approximations and from last measured time-point to infinity using the fit-function. The remainder body was calculated by subtracting all source organs from the total body. The residence times of kidneys, liver, spleen, urinary bladder content, red marrow and remainder body were exported for dose calculation in OLINDA 1.1 [Stabin, M. G., Sparks, R.B., Crowe, E. OLINDA/EXM: the second-generation personal computer software for internal dose assessment in nuclear medicine. J Nucl Med, 46(6):1023-1027, 2005] using the organ masses of the male-adult-phantom.

The potential therapeutic nuclide ²¹²Pb decays further to ²¹²Bi, ²¹²Po and ²⁰⁸Tl. Assuming that the daughter nuclides remain at the site of decay of the parent nuclide and transient equilibrium between ²¹²Pb and its daughters, the same residence time as for 212Pb was applied to the daughter nuclides. OLINDA calculations were performed for all nuclides individually. Respective decay steps were summed up, using weighting factors of 64.07% for ²¹²Po and 35.93% for ²⁰⁸Tl according to their branching ratios. According to the suggestions from the Committee on Medical Internal Radiation Dose and the US Department of Energy [ Sgouros G, Roeske JC, McDevitt MR, et al. MIRD pamphlet no. 22 (abridged): radiobiology and dosimetry of alpha-particle emitters for targeted radionuclide therapy. J Nucl Med. 2010;51:311-28. Feinendegen LE, McClure JJ. Meeting report: alpha-emitters for medical therapy-workshop of the United States Department of Energy, Denver, Colorado, may 30-31, 1996. Radiat Res. 1997;148:195-201.], physical absorbed doses were translated into equivalent doses using weighting factors of 5 for alpha and 1 for beta and photon radiation; Thus, reflecting the relative biological efficacy in regard to deterministic radiation effects - considered the leading factor in therapeutic settings. Tumor and salivary gland volumes were measured individually based on CT-segmentation and their absorbed doses were approximated by using a power function interpolating the spherical model estimates [Stabin, M. G., Konijnenberg, M. Re-evaluation of Absorbed Fractions for Photons and Electrons in Small Spheres. J Nucl Med, 41: 149-160, 2000.].

### ²⁰³Pb Imaging Data of Patients

With an injected activity of 258-310 MBq, the 279 keV gamma rays emitted from ²⁰³Pb with an 80% abundance probability were found to be sufficient to obtain clear planar scans (Fig. 10). These scans show the tracer labeled with the lead isotope specifically enriches in the target tissue. Consequently the method provides a successful approach to use isotopes of lead for the intended applications.

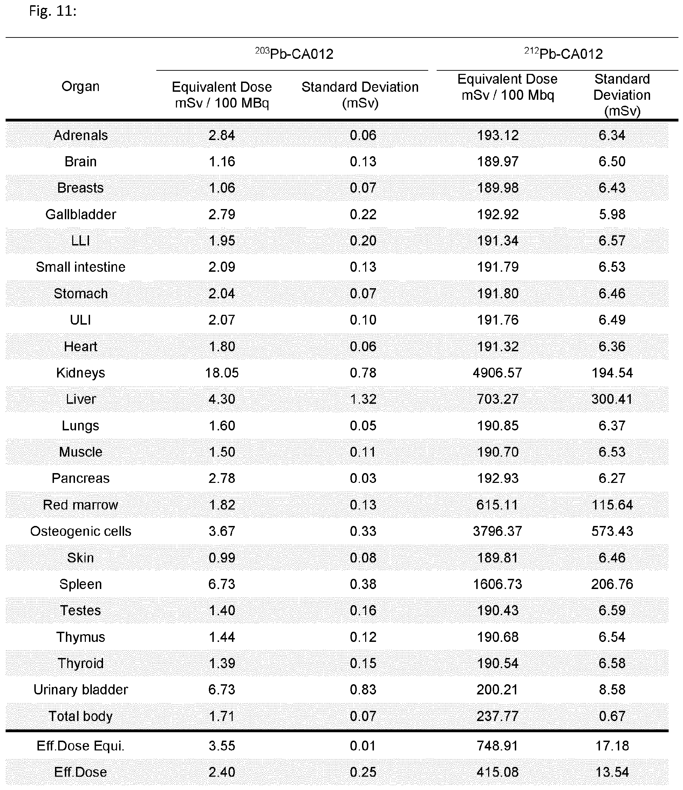

(The Organ distribution of ²⁰³Pb-PSMA-CA012 in tumor bearing mice is shown in the Table in Figure 11)

### VII.3 Dosimetry estimates ²⁰³Pb-CA012

### Dosimetry Estimates

The dosimetry estimate for diagnostic ²⁰³Pb-CA012 is presented in the left column of the table in Figure 11. All organ absorbed doses are dominated by photons (primary emission at 279 keV), low probability emissions in sum contribute <10% in all organs, respectively. A typical clinical examination with 250-300 MBq, e.g. for individual dosimetry prediction, translates into a radiation burden of 6.0-7.5 mSv.

During decay from ²¹²Pb to stable ²⁰⁸Pb, regardless whether by the Polonium or Thallium branch, two beta- and one alpha-particle are emitted per atom. The safety dosimetry estimate for therapeutic ²¹²Pb-CA012, taking into account the complete succeeding decay chain, is presented on the right column of Figure 11. Assuming an RBE=5 for alpha and RBE=1 for beta and gamma radiation the equivalent doses for therapeutic ²¹²Pb-CA012 consist of 96.4% alpha, 2.2% beta and 1.4% gamma contribution. Remarkably: the initial beta decay of ²¹²Pb, which is directly traced by ²⁰³Pb, contributes less than 1% to the total equivalent dose; 99% of the equivalent dose arises from the succeeding daughter nuclides.

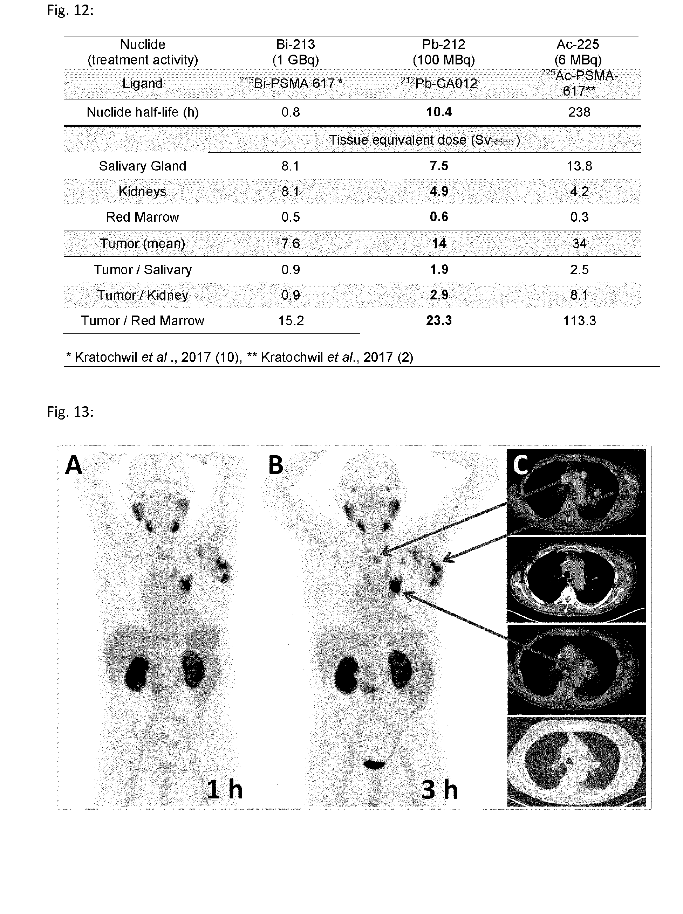

Amongst the OLINDA organs, kidneys and red-marrow might be dose limiting, together with the salivary glands which were assessed using the spherical model. The therapeutic range of a radiopharmaceutical is defined by the ratio between tumor dose and dose-limiting organs. The most relevant dosimetry information are summarized and compared to other PSMA-targeting alpha therapies in Figure 12.

### VII.4 Human PET-scan- ⁶⁸Ga-CA028 and ⁶⁸Ga-CA030

Method for CA028 radiation dosimetry was performed as previously described in Afshar-Oromieh *et al.,* 2015 (Afshar-Oromieh A, Hetzheim H, Kratochwil C, et al. The Theranostic PSMA Ligand PSMA-617 in the Diagnosis of Prostate Cancer by PET/CT: Biodistribution in Humans, Radiation Dosimetry, and First Evaluation of Tumor Lesions. J Nucl Med. 2015;56:1697-1705.)

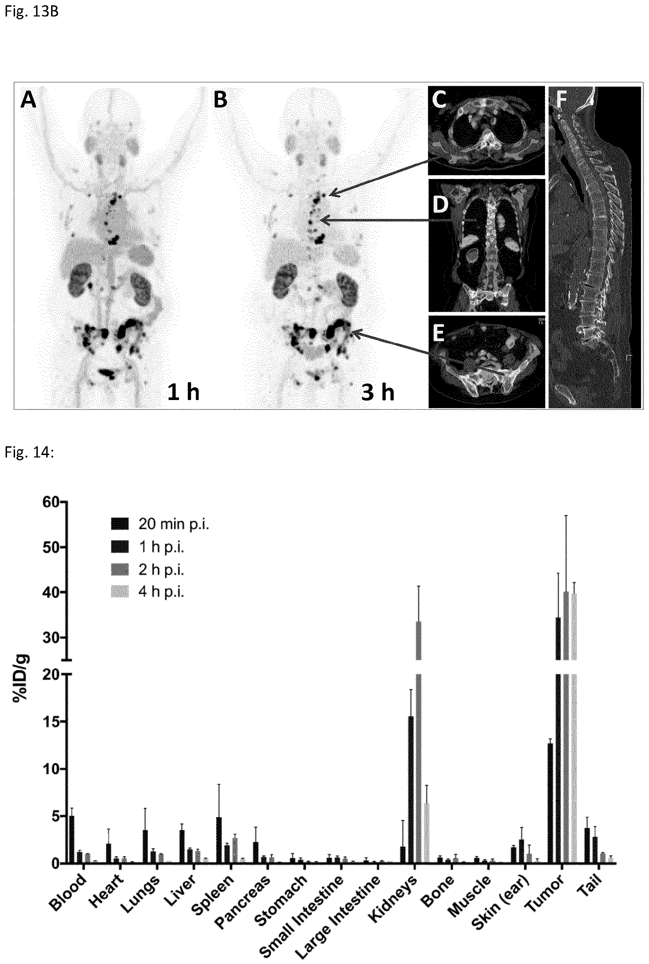

Images were obtained with ⁶⁸Ga-CA028 and ⁶⁸Ga-CA030, respectively, which were applied via intravenous (either 339 MBq / 20 nmol ⁶⁸Ga-CA028 or 295 MBq / 20 nmol ⁶⁸Ga-CA030 per patient). The diagnostic examination of ⁶⁸Ga-CA028 was conducted at the point of time after 1 h and 3 h injection. (See Figure 14)

Diagnostic PSMA-PET/CT examinations were performed 1 h and 3 h after antecubital injection of 339 MBq / 20 nmol ⁶⁸Ga-CA028 or 295 MBq / 20 nmol ⁶⁸Ga-CA030 per patient, respectively. The method for assessing the biodistribution was performed as previously described by Afshar-Oromieh *et al.* (Afshar-Oromieh A, Hetzheim H, Kratochwil C, et al. The Theranostic PSMA Ligand PSMA-617 in the Diagnosis of Prostate Cancer by PET/CT: Biodistribution in Humans, Radiation Dosimetry, and First Evaluation of Tumor Lesions. J Nucl Med. 2015;56: 1697-1705). The activity distributions of the source organs were determined with the clinical standard software Syngo (Siemens), which was used to define the VOIs in the PET images. This reference, Afshar-Oromieh *et al,* was also used as a standard of reference for ⁶⁸Ga-PSMA-617

In order to prove the clinical applicability of ⁶⁸Ga-CA028 and ⁶⁸Ga-CA030, PET/CT imaging in first patients was performed. The resulting images are illustrated in Figure 13A and Figure 13B. The imaging with ⁶⁸Ga-CA028 and ⁶⁸Ga-CA030 confirmed the results obtained *in vitro* and in the animal models. The PET scans and the SUVs were acquired with standard scanner settings, calibrated for pure positron emitters (Wadas TJ, Pandya DN, Solingapuram Sai KK, Mintz A. Molecular targeted alpha-particle therapy for oncologic applications. AJR Am J Roentgenol. 2014;203:253-260). The SUV-values obtained at 1 h versus 3 h post injection are presented in the following table. As reflected by these values a high and stable accumulation in the tumor is achieved.

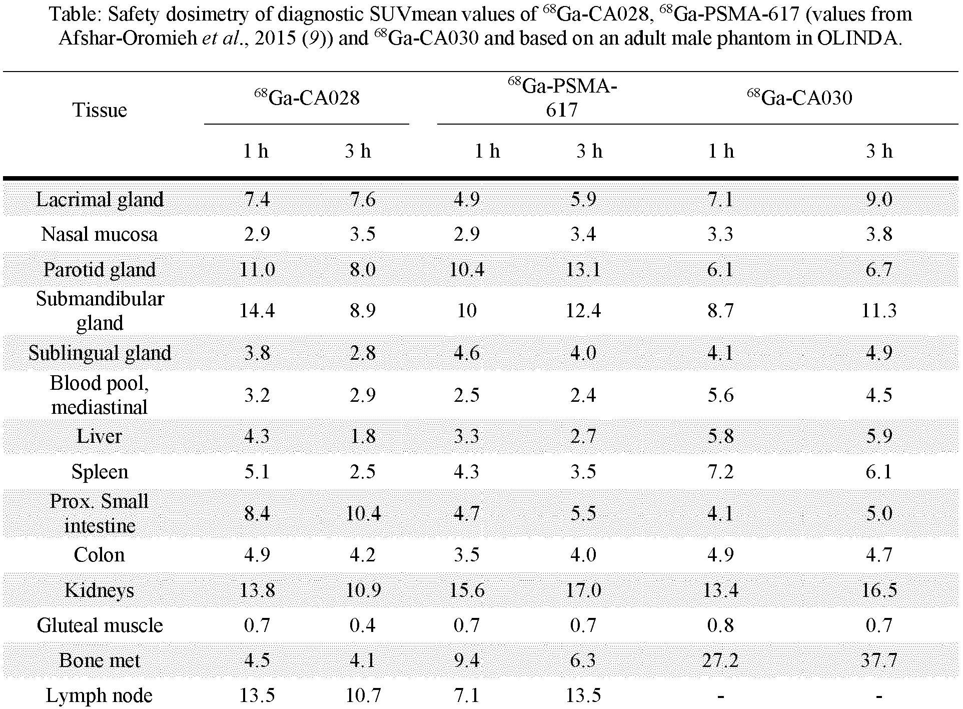

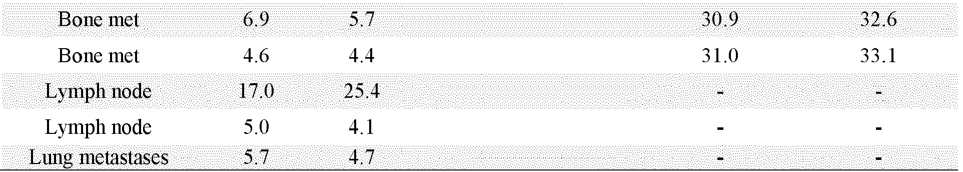

## Claims

1. A compound of formula (1) 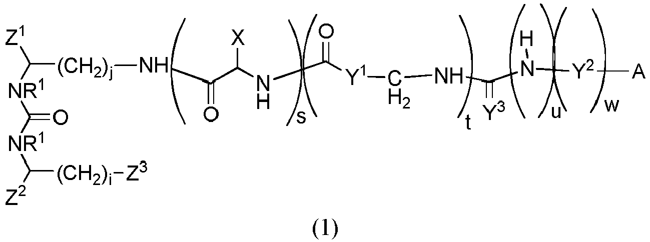
or a pharmaceutically acceptable salt or solvate thereof,
wherein Y³ is O or S,
wherein s, t, u and w are, independently of each other, 0 or 1,
wherein i is an integer of from 1 to 3,
wherein j is an integer of from 3 to 5,
and wherein Z¹, Z² and Z³ are independently of each other, selected from the group consisting of - CO₂H, -SO₂H, -SOsH, -OSOsH, and -OPO₃H_{2,}
R¹ is -CH₃ or H, preferably H
wherein X is 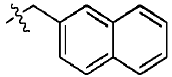
Y¹ and Y² are independently, of each other, selected from the group consisting of , optionally substituted, aryl, alkylaryl (-alkyl-aryl-), cycloalkyl, heterocycloalkyl, heteroaryl and alkylheteroaryl (-alkyl-heteroaryl),
and wherein A is a chelator residue having the structure 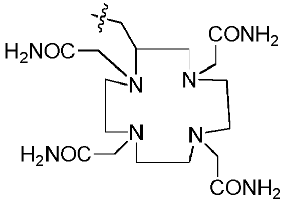
or

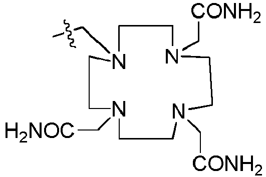

2. The compound of claim 1, wherein Y¹ is

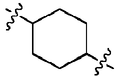

3. The compound of claim 1 or 2, wherein i is 2 and j is 4, and wherein the compound has preferably the structure (1a)

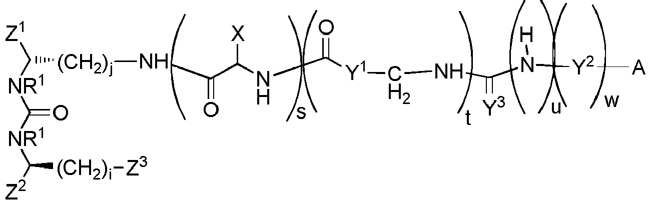

4. The compound of any one of claims 1 to 3, wherein u and w are 1, and wherein
Y³ is S, and wherein Y² is 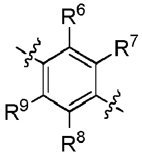
and wherein R⁶, R⁷, R⁸ and R⁹ are, independently of each other H or alkyl, preferably H.

5. The compound of any of of claims 1 to 4, wherein A is

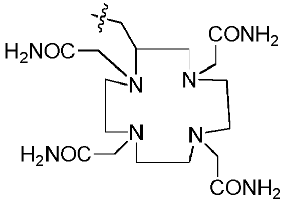

6. The compound of any one of claims 1 to 5, wherein all amino acid residues present in the compound have L-configuration.

7. The compound of any one of claim 1 to 3, wherein u and w are 0.

8. The compound of any one of claims 5 to 7 having a structure selected from the group consisting of 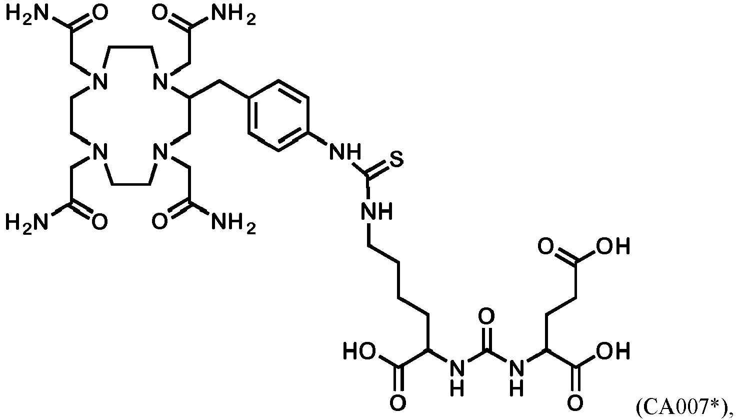 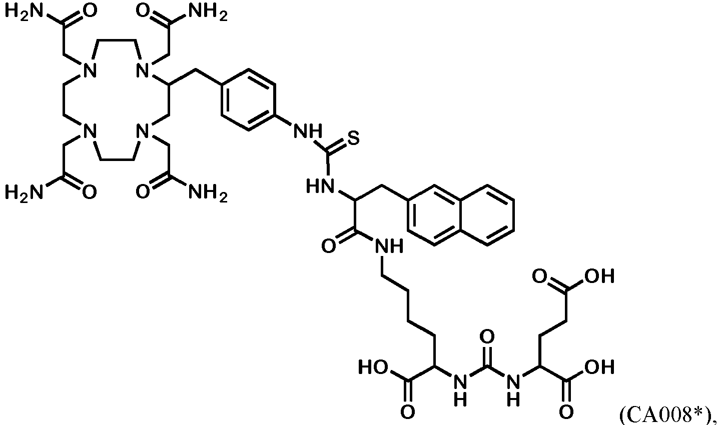 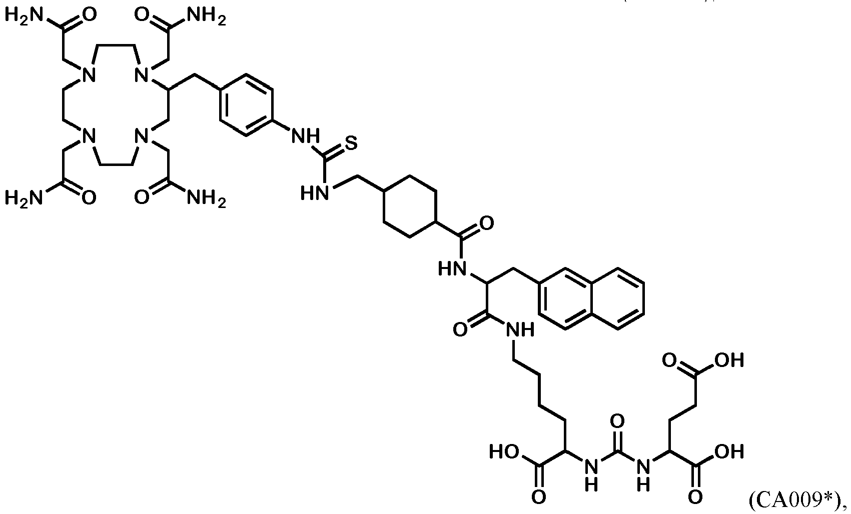 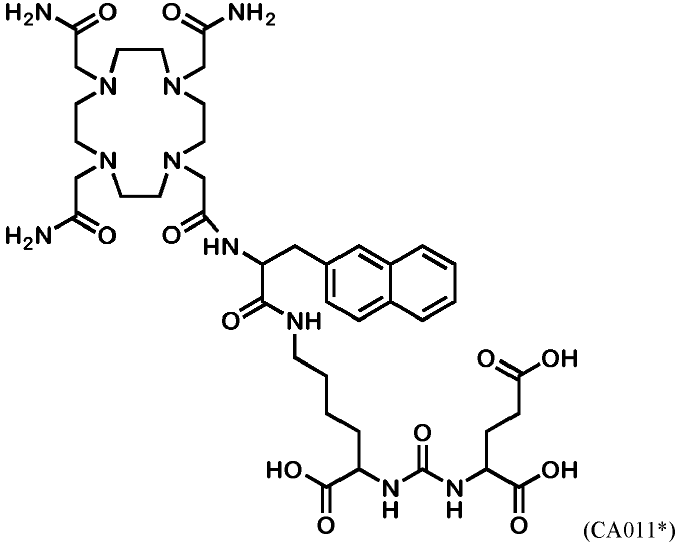 and 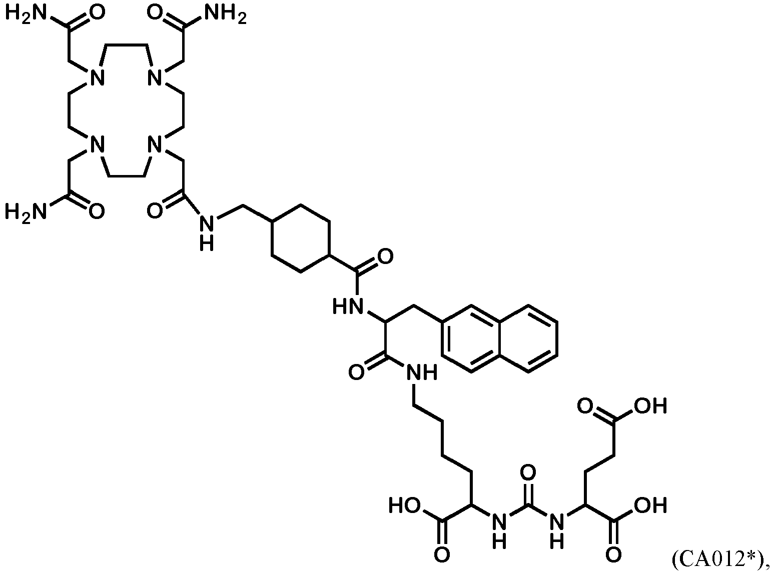 preferably a structure selected from the group consisting of 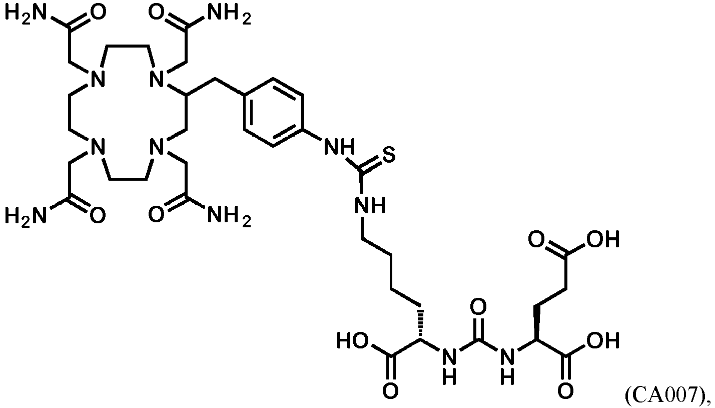 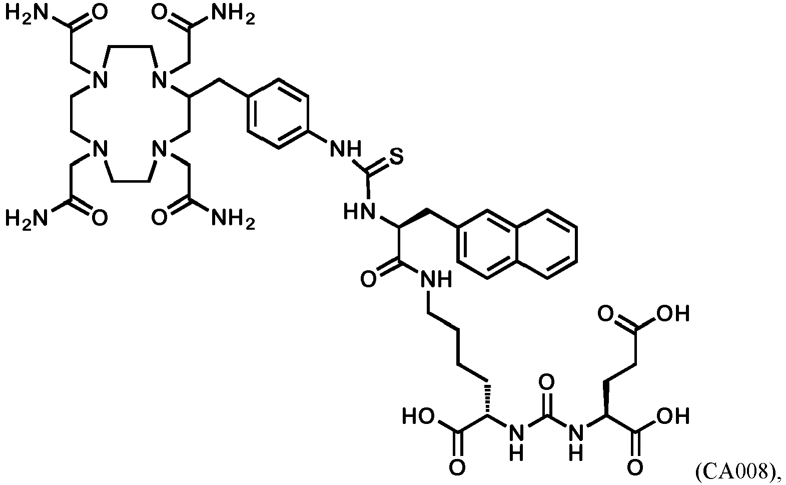 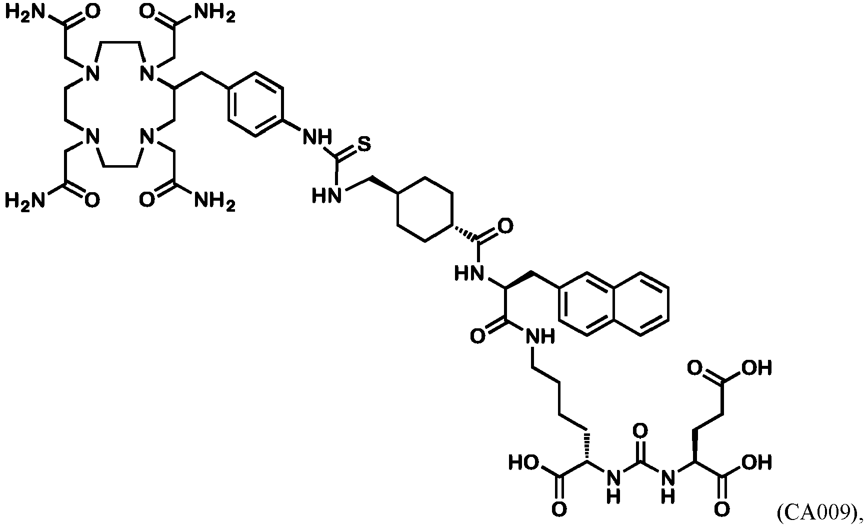 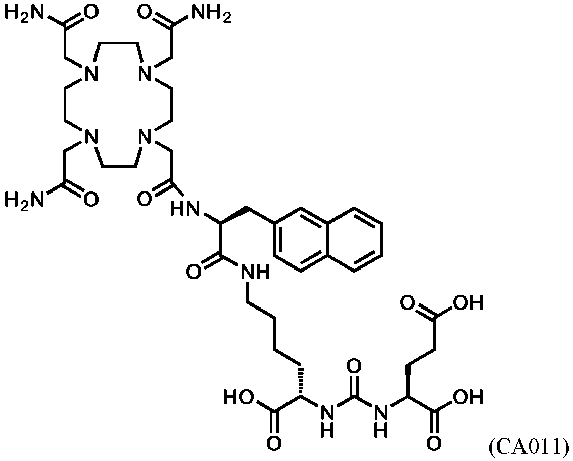 and

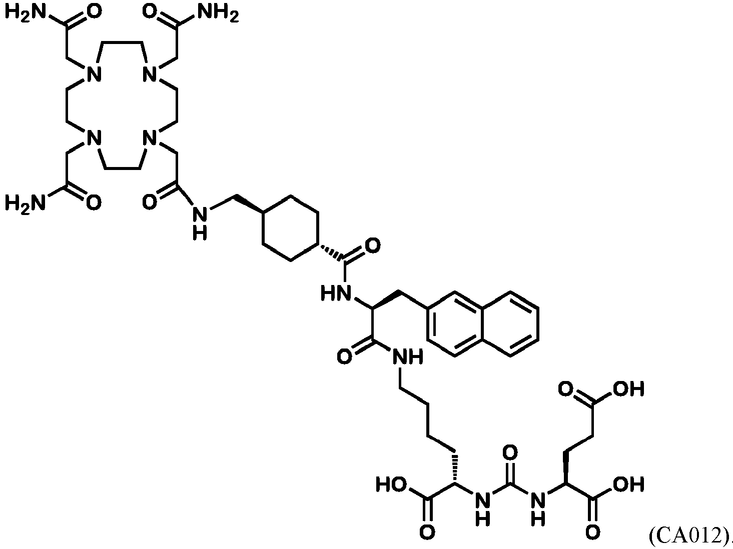

9. A compound having the following formula: 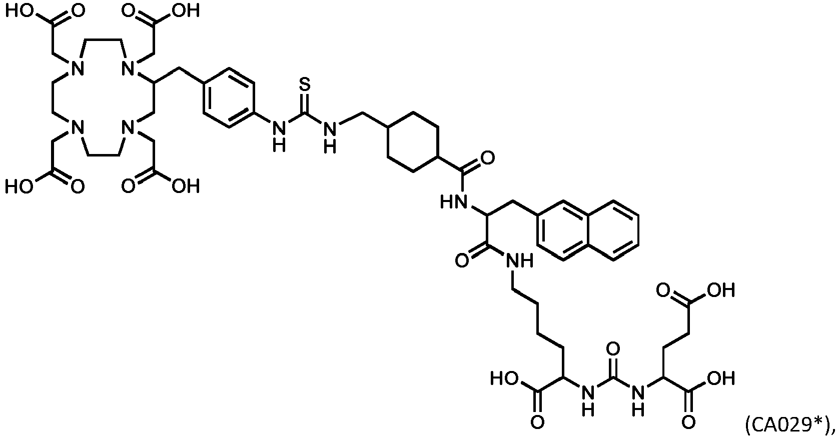 preferably the following structure: 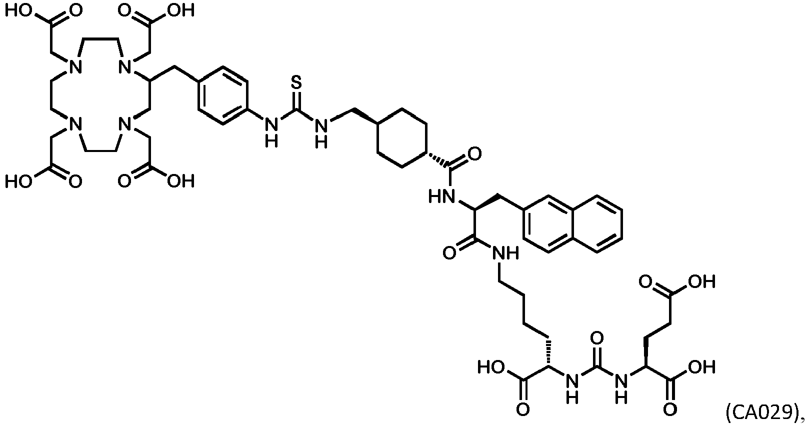 or a pharmaceutically acceptable salt or solvate thereof.

10. Complex comprising
- a radionuclide of Pb, and
- the compound of any one claims 1 to 8 or a salt thereof.

11. The complex of claim 10, wherein, the radionuclide is selected from the group consisting of ²⁰³Pb, ²¹²Pb, ²¹¹ Pb, ²¹³ Pb, ²¹⁴Pb, ²⁰⁹Pb, ¹⁹⁸Pb, ¹⁹⁷Pb, preferably consisting of ²⁰³Pb and ²¹²Pb.

12. A complex comprising
- a radionuclide selected from ⁶⁸Ga and ¹⁷⁷Lu, and
- the compound of claim 9 or a salt thereof.

13. A pharmaceutical composition comprising a compound of any one of claim 1 to 9 or a complex of any one of claims 10 to 12.

14. A compound of any one of claims 1 to 9 or a complex of any one of claims 10 to 12 or a pharmaceutical composition of claim 13 for use in treating, ameliorating or preventing PSMA-expressing cancer and/or metastases thereof, in particular prostate cancer and/or metastases thereof.

15. A compound of any one of claims 1 to 9 or a complex of any one of claims 10 to 12 or a pharmaceutical composition of claim 13 for use in the diagnosis of cancer, preferably for use in tumor imaging using positron emission tomography (PET) or single photon emission computed tomography (SPECT), wherein the radionuclide is preferably ²⁰³Pb.
